# EUROPEAN PATENT APPLICATION

(11) **EP 4 847 503 A1**
(43) Date of publication of application: **02.12.2026**
(21) Application number: 25761105.3
(22) Date of filing: 28.02.2025
(51) Int. Cl.: C07D 403/14, A61K 31/496, A61K 31/497, A61P 29/00

(54) **TETRAZOLE COMPOUND, PHARMACEUTICAL COMPOSITION THEREOF AND USE THEREOF**

(30) Priority: 29.02.2024 CN 202410228284; 26.04.2024 CN 202410508458; 29.07.2024 CN 202411019150; 12.10.2024 CN 202411423681; 20.12.2024 CN 202411887462
(71) Applicant: Shanghai Huilun Pharmaceutical Co., Ltd., Shanghai 200241 (CN)
(72) Inventor: WANG, Xinfeng, Shanghai 200241 (CN); DU, Shuqi, Shanghai 200241 (CN); WU, Xunian, Shanghai 200241 (CN); CHEN, Lei, Shanghai 200241 (CN); QIN, Jihong, Shanghai 200241 (CN)
(74) Representative: Mewburn Ellis LLP
(86) International application number: PCT/CN2025/079748
(87) International publication number: WO 2025/180473

(57) **Abstract**

The present invention provides a tetrazole compound, a pharmaceutical composition thereof, and a use thereof. The compound of the present invention has a brand-new structure, a low clearance rate, high plasma exposure, good oral bioavailability, and good pharmacokinetic properties, and is conducive to drug development. The compound of the present invention or a pharmaceutically acceptable salt thereof can be used for preventing and/or treating pain, including neuropathic pain, etc.

## Description

This application claims priority to Chinese Patent Application No. CN2024102282843, filed on February 29, 2024; Chinese Patent Application No. CN2024105084581, filed on April 26, 2024; Chinese Patent Application No. CN2024110191507, filed on July 29, 2024; Chinese Patent Application No. CN2024114236812, filed on October 12, 2024; and Chinese Patent Application No. CN202411887462X, filed on December 20, 2024. The entire contents of the aforementioned Chinese patent applications are incorporated herein by reference.

### TECHNICAL FIELD

The present invention relates to the field of pharmaceuticals, and more specifically, to a tetrazole-containing compound, a pharmaceutical composition thereof, and uses thereof.

### BACKGROUND

The renin-angiotensin system (RAS) is a critical humoral regulatory system in the human body, with angiotensin II (Ang II) being its major component. Ang II not only plays a vital role in maintaining circulatory stability and water-sodium retention, but is also involved in other physiological functions, including the growth and development of the nervous system. An increasing body of evidence indicates that the RAS plays an important role in neuroprotection and nerve regeneration, and studies have shown that the Ang II metabolic pathway is associated with pain sensitivity in human populations. The receptors for Ang II primarily include the angiotensin II type 1 receptor (AT1R) and the angiotensin II type 2 receptor (AT2R). Both are G protein-coupled receptors and possess similar binding affinities for Ang II. Among them, AT2R is associated with pain mechanisms within the nervous system and is primarily expressed in dorsal root ganglia and trigeminal ganglia. Compared to normal nerves, injured nerves and painful neuromas exhibit higher expression levels of AT2R. Upon activation, AT2R can sensitize ion channels in neurons via the second messenger pathways activated by the G protein-coupled receptor. This sensitization leads to the activation of the ion channels, thereby triggering neuronal excitation. AT2R antagonists have been demonstrated to be effective in alleviating pain through both animal studies (Pain Medicine. 2013, 14, 1557-1568; Pain Medicine. 2013, 14, 692-705) and clinical trials (Lancet. 2014, 383, 1637-1647). Related review articles can be found in Expert Opin. Investig. Drugs. 2014, 23, 1-12; Expert Opin. Ther. Targets. 2015, 19, 25-35, and the like.

### SUMMARY

The present invention provides a compound represented by formula (I) or a pharmaceutically acceptable salt thereof: 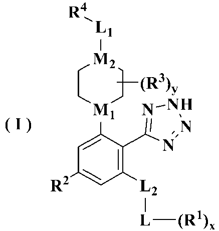
wherein: M₁ and M₂ are each independently selected from C or N;
L₁ is a bond or -CH₂-;
L₂ is a bond, -CH₂-, -C(O)-, -NH-, -C(O)NH-, -NHC(O)-, -O-, or -S-, wherein the - CH₂- and -NH- are optionally substituted with one or two R^{L2}, and R^{L2} are each independently selected from halogen, cyano, C₁₋₃ alkyl, C₁₋₃ alkoxy, C₁₋₃ haloalkyl, C₁₋₃ haloalkoxy, C₃₋₆ cycloalkyl, or 3- to 6-membered heterocyclyl;
L is selected from L₃ or Ring A;
L₃ is selected from C₁₋₆ alkyl, C₁₋₆ alkoxy, C₁₋₆ haloalkyl, C₁₋₆ haloalkoxy, or C₁₋₆ hydroxyalkyl;
Ring A is monocyclic or bicyclic, specifically selected from C₃₋₁₀ cycloalkyl, 3- to 10-membered heterocyclyl, C₆₋₁₄ aryl, 5- to 10-membered heteroaryl, 5- to 6-membered heterocyclyl fused to 5- to 6-membered heteroaryl, or 5- to 6-membered heterocyclyl fused to aryl;
R¹ are each independently selected from halogen, hydroxy, amino, amido, C₁₋₃ alkylamino, di(C₁₋₃ alkyl)amino, cyano, C₁₋₆ alkyl, C₁₋₆ alkoxy, C₁₋₆ haloalkyl, C₁₋₆ haloalkoxy, C₁₋₆ hydroxyalkyl, C₃₋₆ cycloalkyl, or 3- to 6-membered heterocyclyl;
or, any two R¹ are joined to form a 3- to 8-membered heterocyclyl or C₃₋₈ cycloalkyl;
R² is selected from amino, C₁₋₆ alkyl, C₁₋₆ haloalkyl, C₁₋₆ haloalkoxy, C₃₋₆ cycloalkyl, 3- to 6-membered heterocyclyl, C₆₋₁₀ aryl, 5- to 6-membered heteroaryl, -OR^{2.1} or -NHR^{2.1}; the C₁₋₆ alkyl, C₁₋₆ haloalkyl, C₁₋₆ haloalkoxy, C₃₋₆ cycloalkyl, 3- to 6-membered heterocyclyl, C₆₋₁₀ aryl, and 5- to 6-membered heteroaryl are optionally further substituted with one or more R^{2.1};
R^{2.1} is selected from C₁₋₆ alkyl, C₁₋₆ haloalkyl, C₁₋₆ haloalkoxy, C₃₋₆ cycloalkyl, or 3-to 6-membered heterocyclyl, wherein the C₁₋₆ alkyl, C₁₋₆ haloalkyl, C₁₋₆ haloalkoxy, C₃₋₆ cycloalkyl, and 3- to 6-membered heterocyclyl are optionally substituted with one or more R^{2.1.1};
R^{2.1.1} is selected from halogen, amino, cyano, hydroxy, C₁₋₆ alkoxy, C₃₋₆ cycloalkyl, or 3- to 6-membered heterocyclyl;
R³ are each independently selected from hydrogen, C₁₋₆ alkyl, C₁₋₆ alkoxy, C₁₋₆ haloalkyl, or C₁₋₆ haloalkoxy;
R⁴ is selected from 5- to 6-membered heteroaryl, 5- to 6-membered heterocyclyl fused to 5- to 6-membered heteroaryl, 5- to 6-membered heteroaryl fused to 5- to 6-membered heteroaryl, 5- to 6-membered heteroaryl fused to phenyl, 5- to 6-membered heterocyclyl fused to phenyl, or 5- to 6-membered heterocyclyl, wherein the 5- to 6-membered heteroaryl, 5- to 6-membered heterocyclyl fused to 5- to 6-membered heteroaryl, 5- to 6-membered heteroaryl fused to 5- to 6-membered heteroaryl, 5- to 6-membered heteroaryl fused to phenyl, 5- to 6-membered heterocyclyl fused to phenyl, and 5- to 6-membered heterocyclyl are optionally substituted with one or more R^{4.1};
R^{4.1} is selected from halogen, hydroxy, cyano, amino, C₁₋₆ alkyl, C₁₋₆ alkoxy, C₁₋₆ haloalkyl, C₁₋₆ hydroxyalkyl, C₁₋₆ haloalkoxy, C₃₋₆ cycloalkyl, or 3- to 6-membered heterocyclyl;
x and y are each independently selected from 0, 1, 2, 3, or 4.

In a preferred embodiment of the present invention, the compound of formula (I) is further represented by formula (I-A) or formula (I-B):

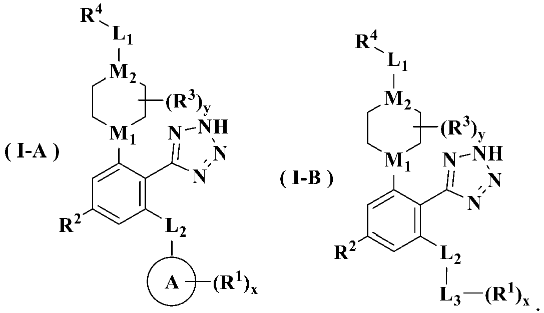

In a preferred embodiment of the present invention, the L₁ is selected from -CH₂-; or, the R⁴ is selected from 5- to 6-membered heteroaryl, 5- to 6-membered heteroaryl fused to 5-to 6-membered heteroaryl, 5- to 6-membered heteroaryl fused to phenyl, 5- to 6-membered heterocyclyl fused to phenyl, 5- to 6-membered heterocyclyl fused to 5- to 6-membered heteroaryl, or 6-membered heterocyclyl; preferably, the R⁴ is selected from thiazolyl, pyrazolyl, pyridazinyl, imidazolyl, pyridinyl, isoxazolyl, triazolyl, tetrazolyl, oxazolyl, pyrazinyl, pyrimidinyl, oxadiazolyl, pyridinonyl, pyridazinonyl, pyrimidinonyl, pyrazinonyl, imidazopyridinyl, benzimidazolyl, benzothiazolyl, benzoxazolyl, pyrimidinonophenyl, pyrimidinonopyridinyl, pyrazolophenyl, pyridinonophenyl, pyrazinonophenyl, pyrimidinonothiazolyl, pyrimidinonopyrazolyl, or oxazolopyridinyl; more preferably, the R⁴ is selected from thiazolyl, pyridazinyl, benzothiazolyl, or pyrazinyl.

In a certain embodiment of the present invention, the L³ is amino.

In a preferred embodiment of the present invention, the R⁴ is selected from pyridazinyl.

In a preferred embodiment of the present invention, the compound of formula (I-A) is further represented by formula (II):

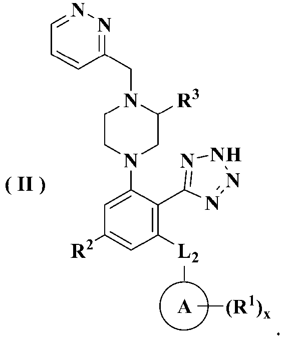

In a certain aspect of the present invention, the L₂ of the present invention may further be selected from a bond, -CH₂-, or -(CH₂)₂-.

In a certain aspect of the present invention, the L₃ of the present invention may further be selected from C₂₋₆ alkenyl, C₂₋₆ alkynyl, -P(=O)R^{1.1}R^{1.2}, -S(=O)₂R^{1.1}, -S(=O)(=NR^{1.1})R^{1.2}, C₃₋₆ cycloalkyl, 3- to 6-membered heterocyclyl, C₃₋₆ cycloalkyl C₁₋₃ alkyl, 3- to 6-membered heterocyclyl C₁₋₃ alkyl, or -OR^{1.1};

R^{1.1} and R^{1.2} are each independently selected from halogen, hydroxy, cyano, amino, C₁₋₆ alkyl, C₁₋₆ hydroxyalkyl, C₁₋₆ alkoxy, C₁₋₆ haloalkyl, C₁₋₆ haloalkoxy, C₃₋₆ cycloalkyl, or 3- to 6-membered heterocyclyl.

In a preferred embodiment of the present invention, the L₃ is selected from C₁₋₄ alkyl, C₁₋₄ alkoxy, C₁₋₄ haloalkyl, C₁₋₄ haloalkoxy, or C₁₋₄ hydroxyalkyl.

In a preferred aspect of the present invention, the L₃ of the present invention is selected from C₂₋₄ alkenyl, C₂₋₄ alkynyl, -P(=O)R^{1.1}R^{1.2}, -S(=O)₂R^{1.1}, -S(=O)(=NR^{1.1})R^{1.2}, C₃₋₄ cycloalkyl, 3- to 4-membered heterocyclyl, C₃₋₄ cycloalkyl C₁₋₃ alkyl, 3- to 4-membered heterocyclyl C₁₋₃ alkyl, or -OR^{1.1}.

In a preferred aspect of the present invention, the R^{1.1} and R^{1.2} of the present invention are each independently selected from halogen, hydroxy, cyano, amino, C₁₋₃ alkyl, C₁₋₃ hydroxyalkyl, C₁₋₃ alkoxy, C₁₋₃ haloalkyl, C₁₋₃ haloalkoxy, C₃₋₄ cycloalkyl, or 3- to 4-membered heterocyclyl.

In a preferred embodiment of the present invention, the x is 0, 1, 2, or 3; preferably 0, 1, or 2.

In a preferred embodiment of the present invention, the Ring A is selected from 5- to 6-membered heteroaryl, 5-membered heteroaryl fused to phenyl, 5-membered heterocyclyl fused to phenyl, 5-membered heterocyclyl fused to 6-membered heteroaryl, 6-membered heterocyclyl fused to 5-membered heteroaryl, C₆₋₁₀ aryl, C₃₋₈ cycloalkyl, or 3- to 8-membered heterocyclyl; preferably, the Ring A is selected from pyridazinyl, pyrazolyl, pyridinyl, phenyl, pyrrolyl, imidazolyl, triazolyl, tetrazolyl, furyl, thienyl, cyclopropyl, cyclobutyl, cyclopentyl, cyclohexyl, tetrahydropyranyl, pyrrolidinyl, pyridinonyl, 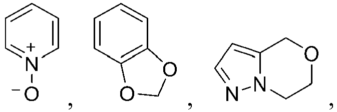 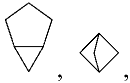 morpholinyl, piperidinyl, oxiranyl, aziridinyl, oxetanyl, azetidinyl, piperazinyl, benzopyrrolyl, benzopyrrolidinyl, diazaspiro[3.3]heptanyl, or oxaazaspiro[3.3]heptanyl.

In a certain aspect of the present invention, the Ring A of the present invention is selected from tetrahydrofuranyl, 2-azaspiro[3.3]heptane, pyrrolidinonyl, azaspiro[2.5]octanyl, or cyclohexenyl,

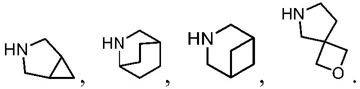

In a preferred embodiment of the present invention, the R¹ are each independently selected from halogen, hydroxy, amino, amido, cyano, C₁₋₄ alkyl, C₁₋₄ alkoxy, C₁₋₄ haloalkyl, C₁₋₄ haloalkoxy, C₁₋₄ hydroxyalkyl, C₃₋₄ cycloalkyl, or 3- to 4-membered heterocyclyl; preferably, the R¹ are each independently selected from fluoro, chloro, hydroxy, amino, amido, cyano, methyl, ethyl, propyl, methoxy, ethoxy, propoxy, trifluoromethyl, difluoromethyl, trifluoroethyl, hydroxymethyl, hydroxyethyl, cyclopropyl, cyclobutyl, oxiranyl, aziridinyl, oxetanyl, or azetidinyl.

In a preferred embodiment of the present invention, the compound of formula (I-B) is further represented by formula (III):

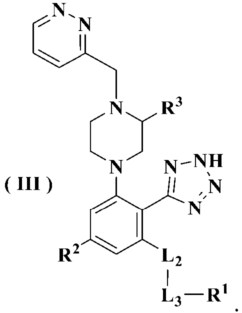

In a preferred embodiment of the present invention, the L₃ is selected from a bond, methylene, ethylene, propylene, halogen-substituted methylene, halogen-substituted ethylene, halogen-substituted propylene, hydroxy-substituted methylene, hydroxy-substituted ethylene, or hydroxy-substituted propylene.

In a preferred embodiment of the present invention, the L₃ is selected from a bond, - CH₂-, -CH₂CH₂-, -CH(CH₃)CH₂-, or -CH₂-CH(F)-CH₂-.

In a preferred embodiment of the present invention, the L₂ is selected from a bond, - O-, -S-, or -NH-.

In a certain aspect of the present invention, the L₂ of the present invention is selected from -NH-.

In a preferred embodiment of the present invention, the R¹ is selected from C₁₋₃ alkyl, C₁₋₃ alkoxy, C₁₋₃ hydroxyalkyl, C₁₋₃ haloalkyl, C₁₋₃ alkyl-substituted amino, or -SF₅.

In a preferred embodiment of the present invention, the R¹ is selected from methyl, ethyl, methoxy, ethoxy, hydroxymethyl, hydroxyethyl, -CHF₂, -CF₃, -NHCH₃, or -SF₅.

In a preferred embodiment of the present invention, the R¹ are each independently selected from chloro, fluoro, methoxy, methyl, -CH₂F, cyclopropyl, -CF₃, 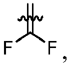 or hydroxy.

In a preferred embodiment of the present invention, the L₂ is a bond, -CH₂-, -CF₂-, - C(O)-, -NH-, -O-, or -S-, wherein the -CH₂- and -NH- are optionally substituted with one or two RL₂, and RL₂ are each independently selected from halogen, cyano, C₁₋₃ alkyl, C₁₋₃ alkoxy, C₁₋₃ haloalkyl, C₁₋₃ haloalkoxy, C₃₋₄ cycloalkyl, or 3- to 4-membered heterocyclyl. In a preferred embodiment of the present invention, the R^{L2} are each independently selected from fluoro, chloro, methyl, ethyl, propyl, methoxy, ethoxy, trifluoromethyl, difluoromethyl, trifluoroethyl, cyclopropyl, cyclobutyl, aziridinyl, oxiranyl, azetidinyl, or oxetanyl.

In a preferred embodiment of the present invention, the L₂ is selected from -NH-, - CH₂-, -C(O)NH-, -NHC(O)-, -CF₂-, -O-, or -S-.

In a preferred embodiment of the present invention, the L₂ is a bond, -O-, -S-, -NH-, or -CH₂-.

In a preferred embodiment of the present invention, the R² is selected from amino, C₁₋₄ alkyl, C₁₋₄ haloalkyl, C₁₋₄ haloalkoxy, C₃₋₆ cycloalkyl, 3- to 6-membered heterocyclyl, -OR^{2.1} or -NHR^{2.1}; preferably, the R² is selected from amino, methyl, ethyl, propyl, n-butyl, isobutyl, cyclopropyl, piperidinyl, morpholinyl, piperazinyl, -OR^{2.1} or -NHR^{2.1};
R^{2.1} is selected from C₁₋₄ alkyl, C₁₋₄ haloalkyl, C₁₋₄ haloalkoxy, C₃₋₄ cycloalkyl, or 3-to 4-membered heterocyclyl, wherein the C₁₋₄ alkyl, C₁₋₄ haloalkyl, C₁₋₄ haloalkoxy, C₃₋₆ cycloalkyl, and 3- to 6-membered heterocyclyl are optionally substituted with one or more R^{2.1.1}; preferably, R^{2.1} is selected from methyl, ethyl, propyl, n-butyl, isobutyl, methoxy, ethoxy, propoxy, cyclopropyl, cyclobutyl, cyclopentyl, oxiranyl, aziridinyl, oxetanyl, azetidinyl, piperidinyl, piperazinyl, morpholinyl, trifluoromethyl, difluoromethyl, or difluoroethyl;
The R^{2.1.1} is selected from halogen, amino, cyano, hydroxy, C₁₋₄ alkoxy, C₃₋₆ cycloalkyl, or 3- to 6-membered heterocyclyl; preferably, the R^{2.1.1} is selected from fluoro, chloro, methyl, ethyl, propyl, n-butyl, isobutyl, methoxy, ethoxy, propoxy, cyclopropyl, cyclobutyl, cyclopentyl, oxiranyl, aziridinyl, oxetanyl, azetidinyl, piperidinyl, piperazinyl, morpholinyl, trifluoromethyl, difluoromethyl, or difluoroethyl;
or, R³ is selected from hydrogen, C₁₋₄ alkyl, C₁₋₄ alkoxy, C₁₋₄ haloalkyl, or C₁₋₄ haloalkoxy; preferably, R³ is selected from hydrogen, methyl, ethyl, propyl, methoxy, ethoxy, propoxy, trifluoromethyl, or difluoromethyl.

In a preferred embodiment of the present invention, the R³ is methyl.

In a preferred embodiment of the present invention, the R² of the present invention is selected from

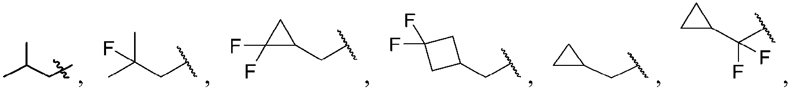

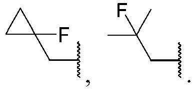

In a preferred embodiment of the present invention, the R² is

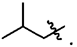

In a preferred embodiment of the present invention, the R^{4.1} is selected from fluoro, chloro, hydroxy, cyano, amino, C₁₋₄ alkyl, C₁₋₄ alkoxy, C₁₋₄ haloalkyl, C₁₋₄ hydroxyalkyl, C₁₋₄ haloalkoxy, C₃₋₄ cycloalkyl, or 3- to 4-membered heterocyclyl; preferably, the R^{4.1} is selected from fluoro, chloro, hydroxy, cyano, amino, methyl, ethyl, propyl, methoxy, ethoxy, propoxy, trifluoromethyl, difluoromethyl, hydroxymethyl, cyclopropyl, or cyclobutyl.

In a preferred embodiment of the present invention, the aforementioned 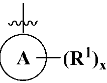 is selected from

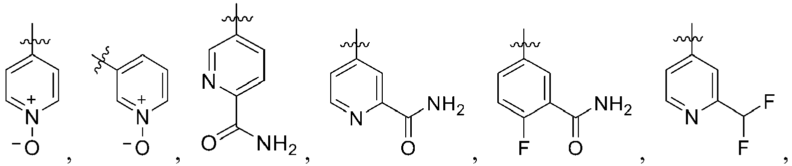

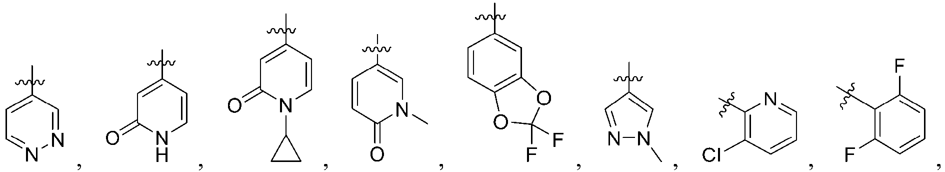

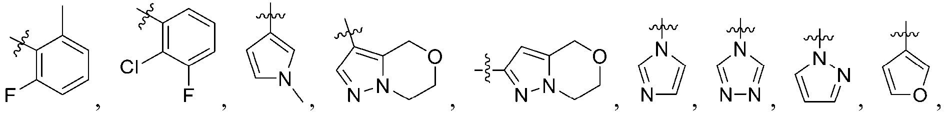

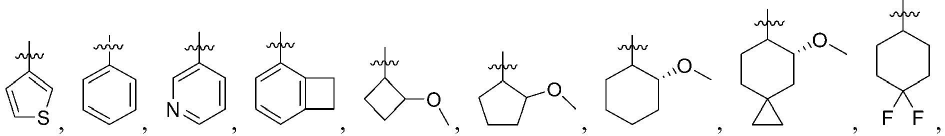

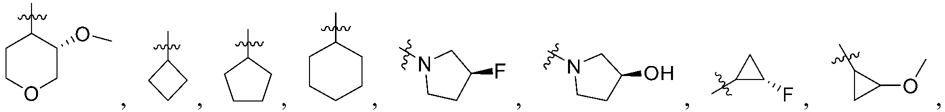

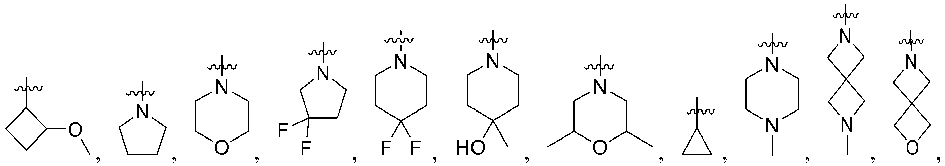

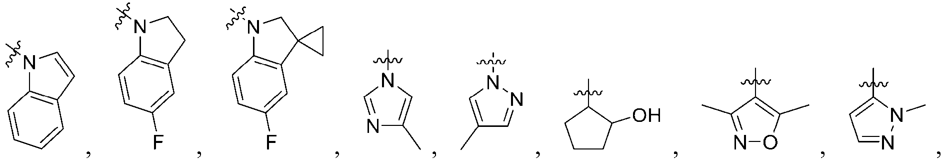

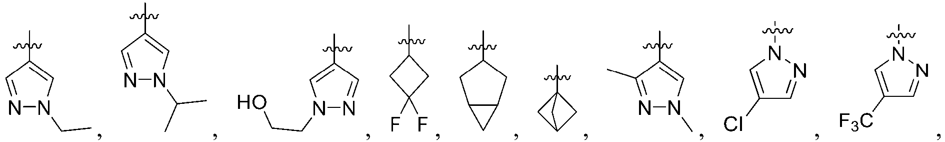

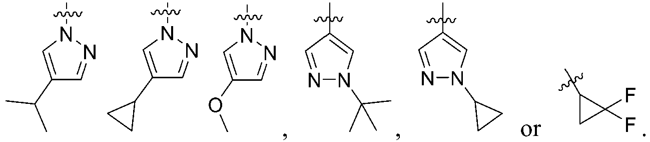

In a preferred embodiment of the present invention, the 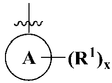 is selected from

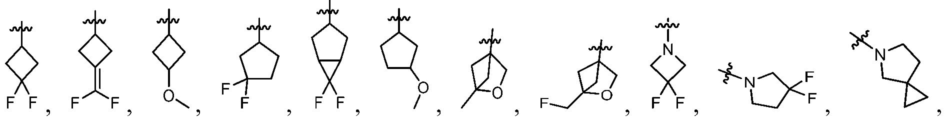

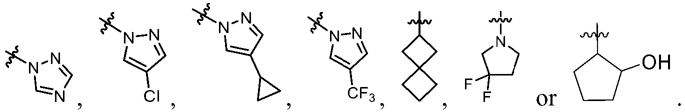

In a certain aspect of the present invention, the 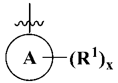 is selected from

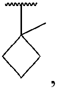

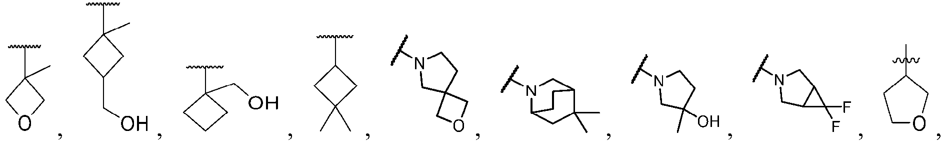

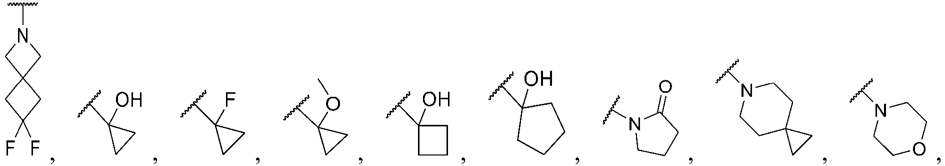

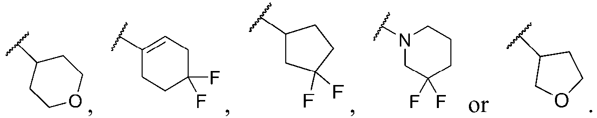

In a preferred embodiment of the present invention, the 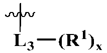 is selected from methyl, 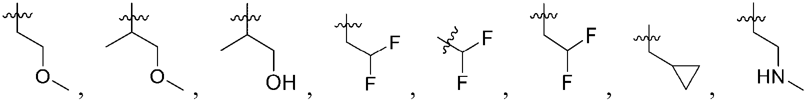 or

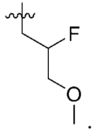

In a preferred embodiment of the present invention, the 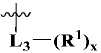 and -L₃-R¹ are each independently selected from methyl, methoxy, ethynyl, vinyl, -CF₃, -OCF₃,

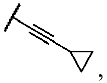

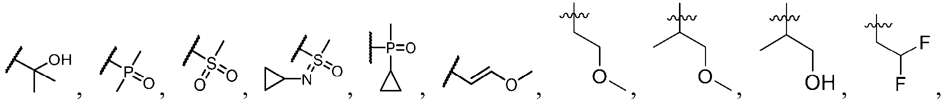

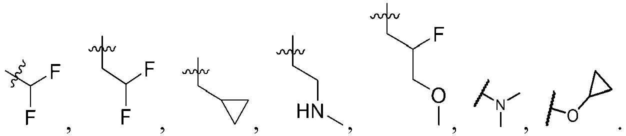

In a preferred embodiment of the present invention, the compound is selected from the compounds of Table 1 below:

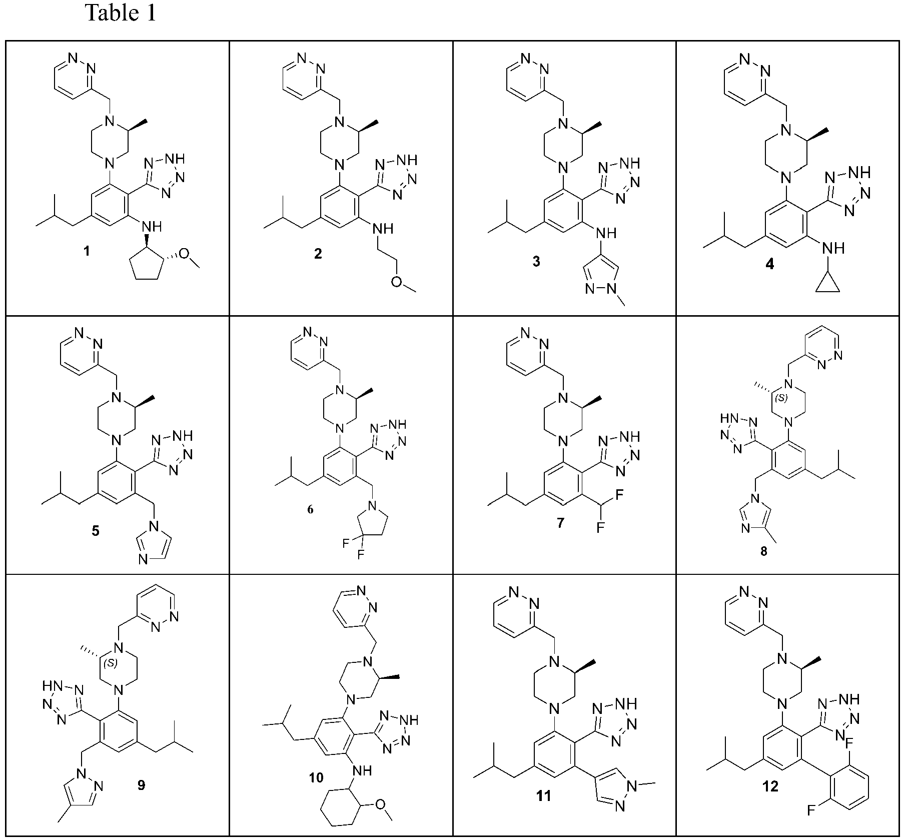

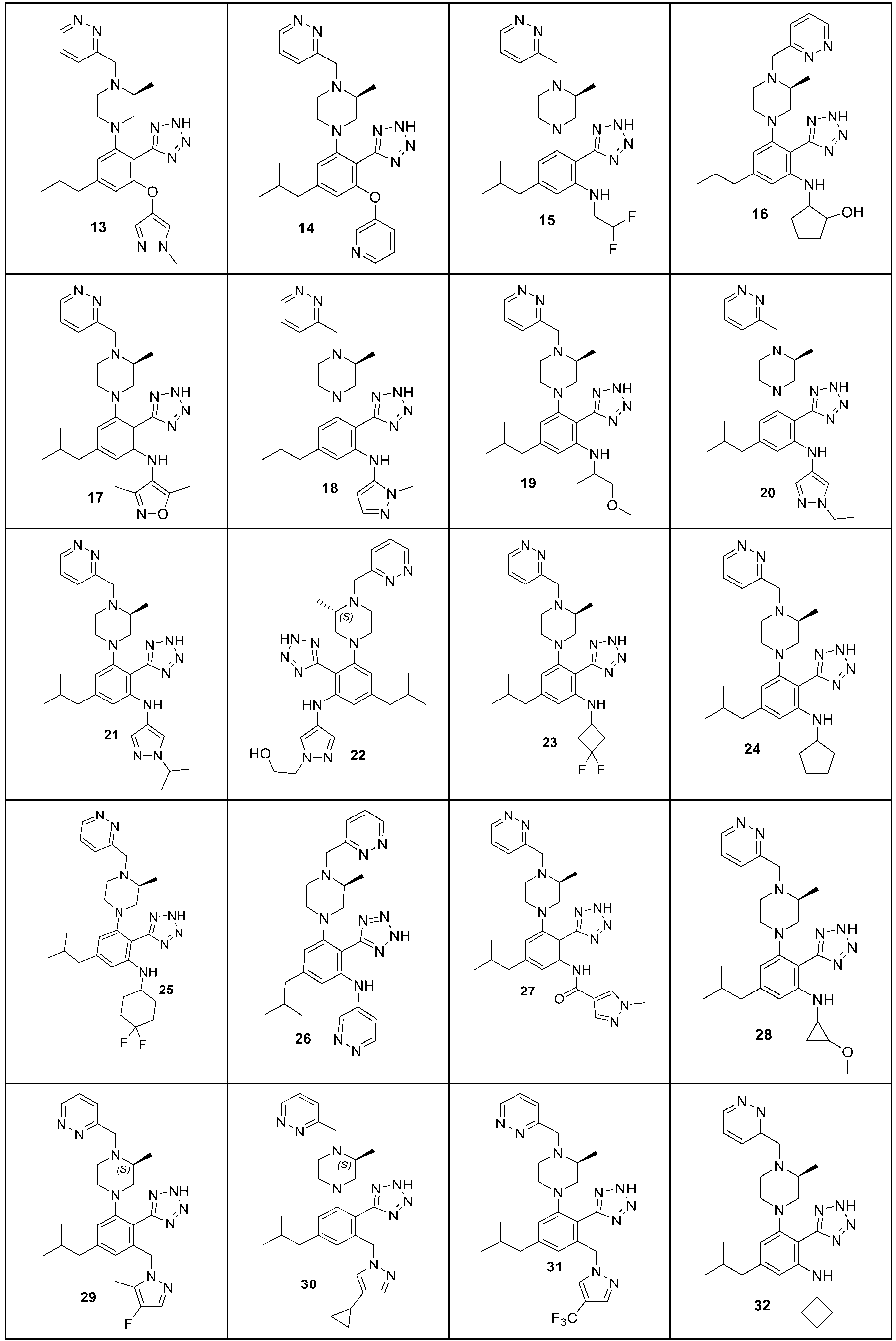

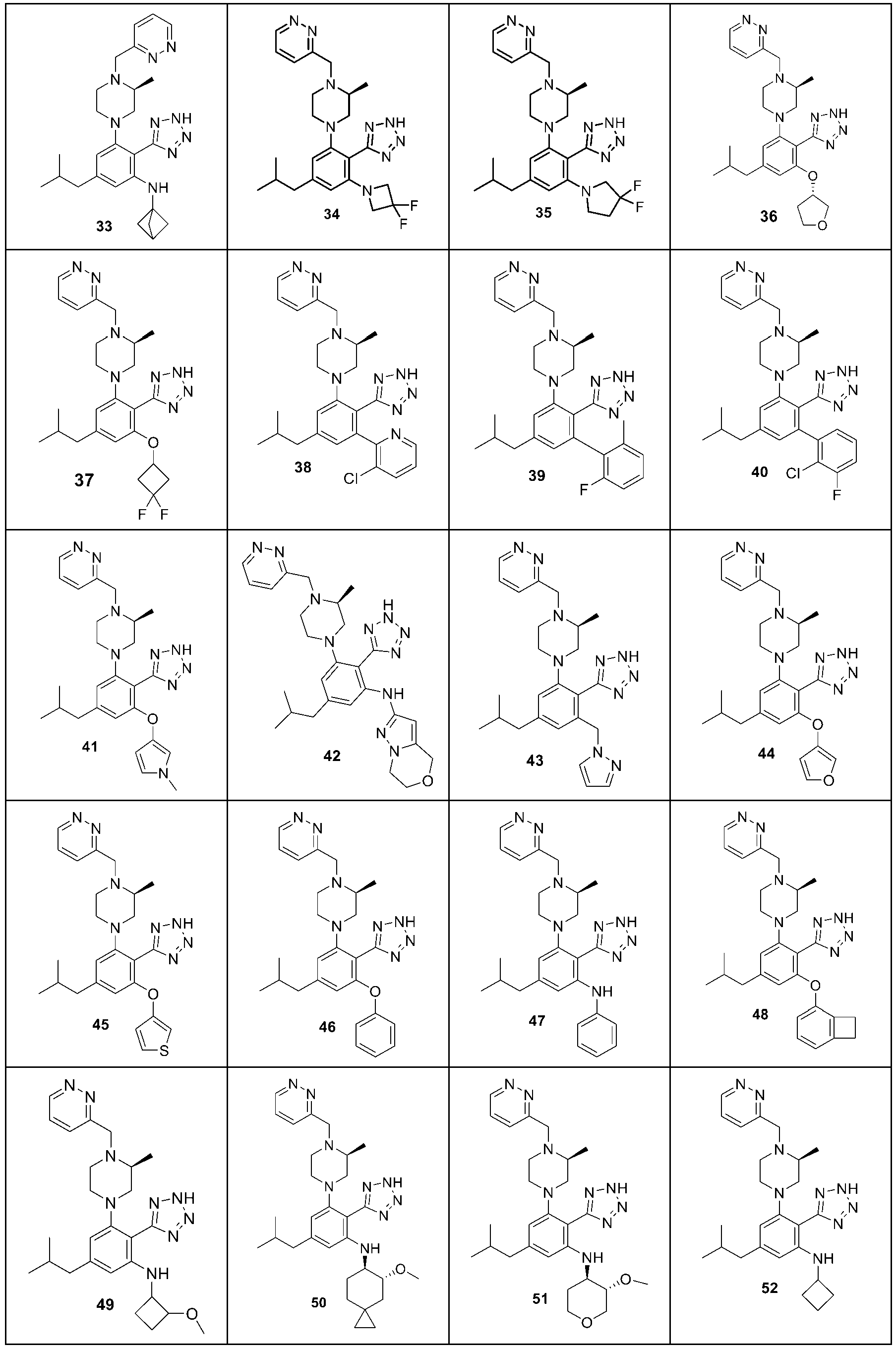

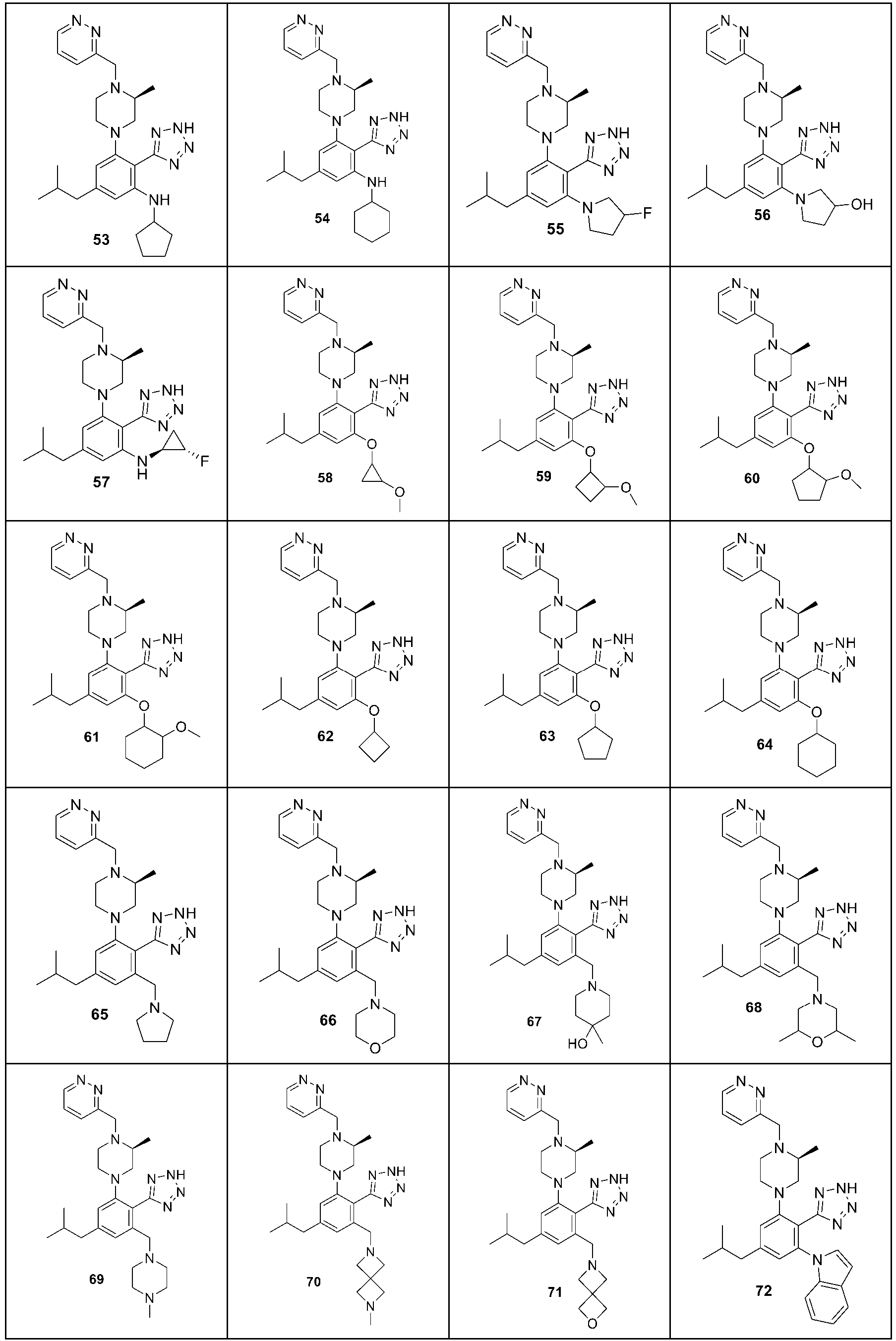

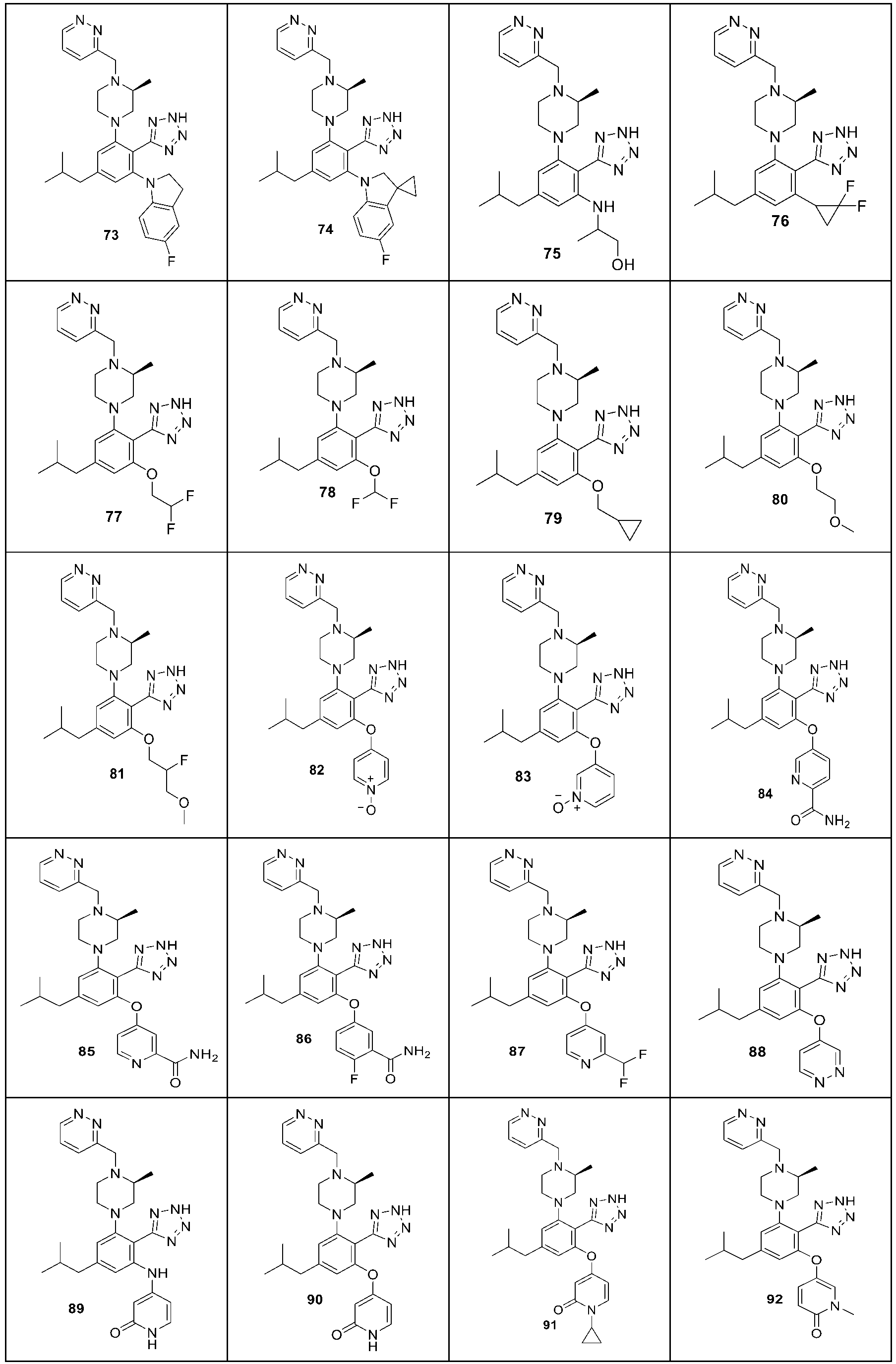

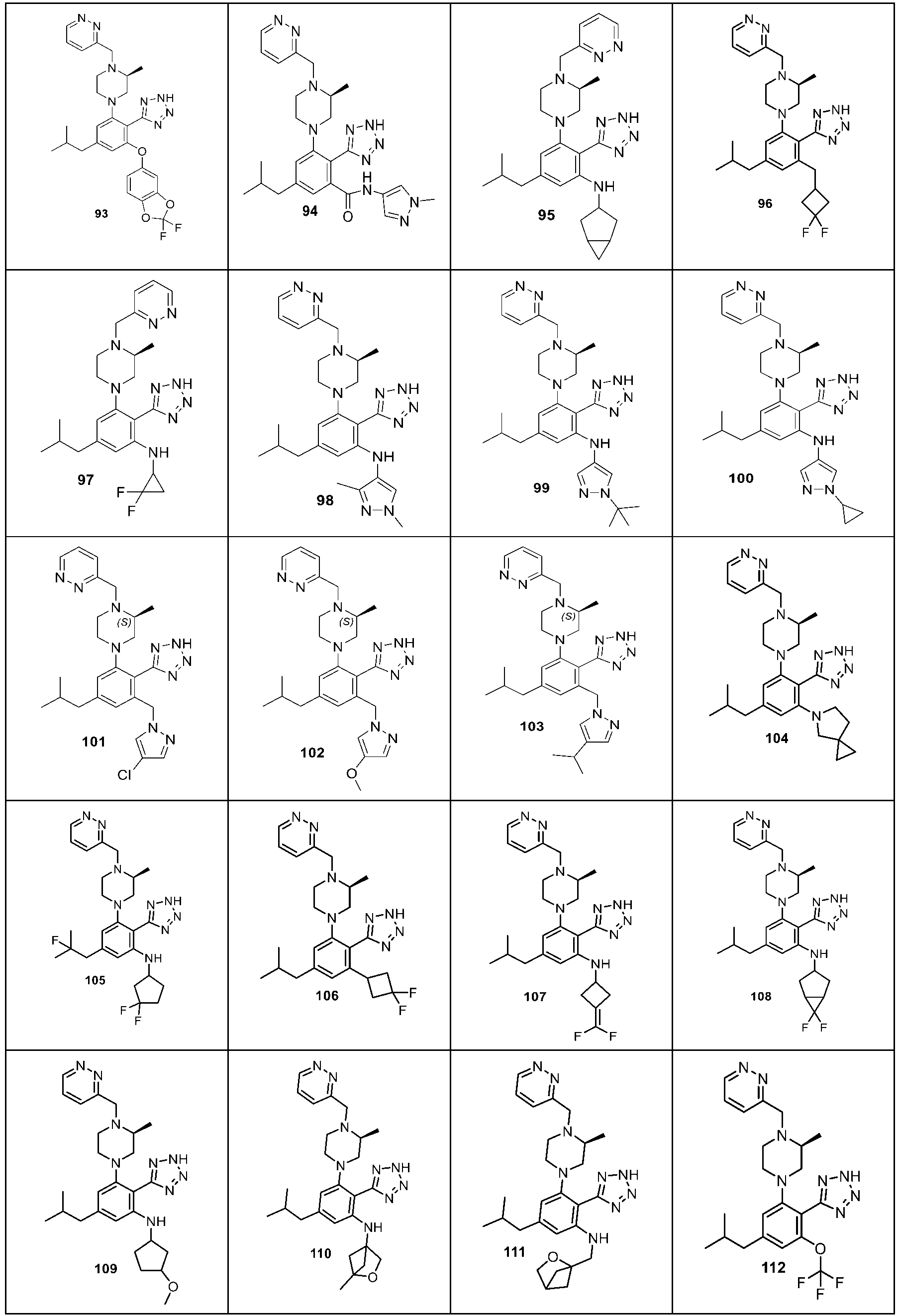

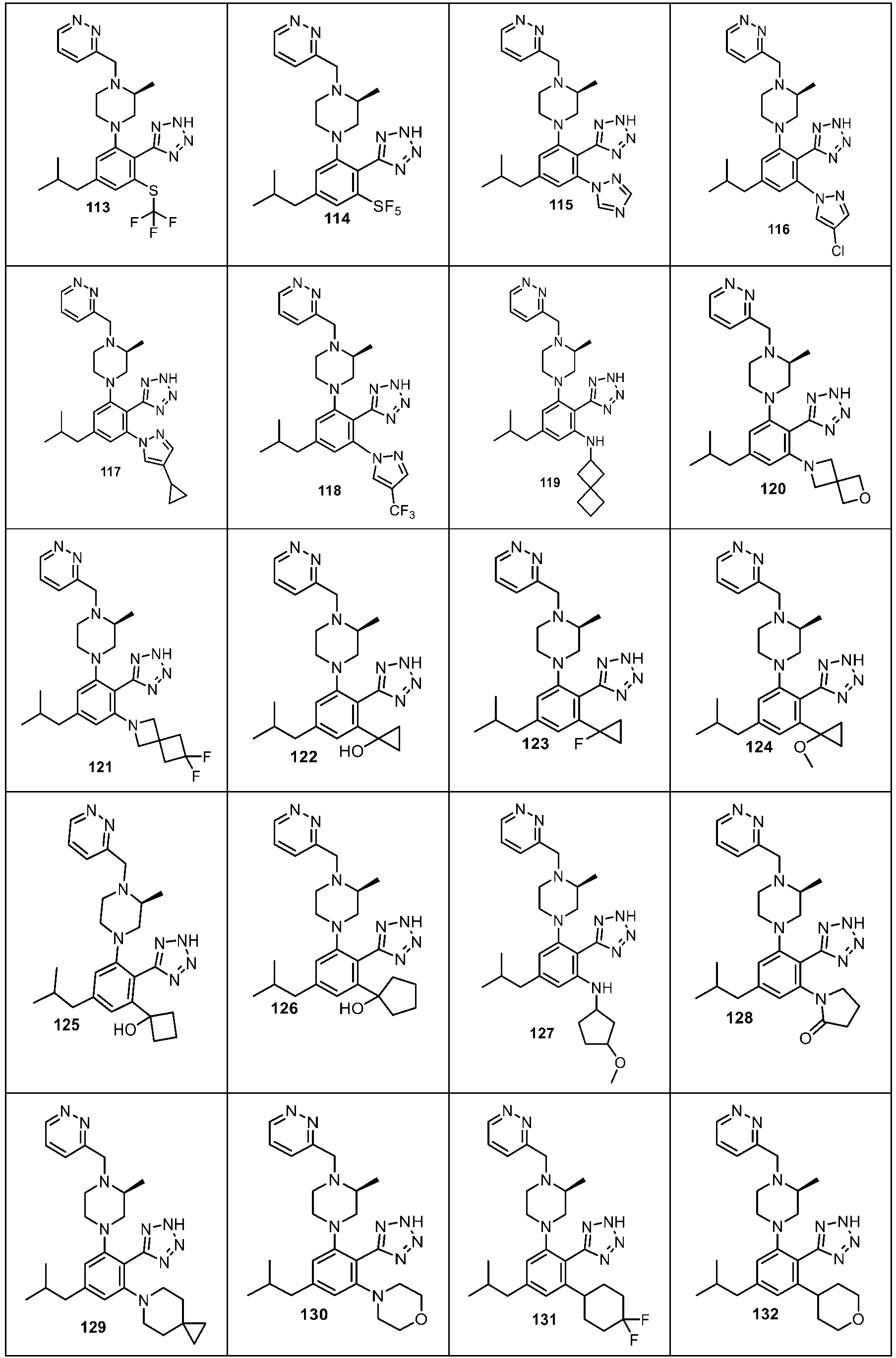

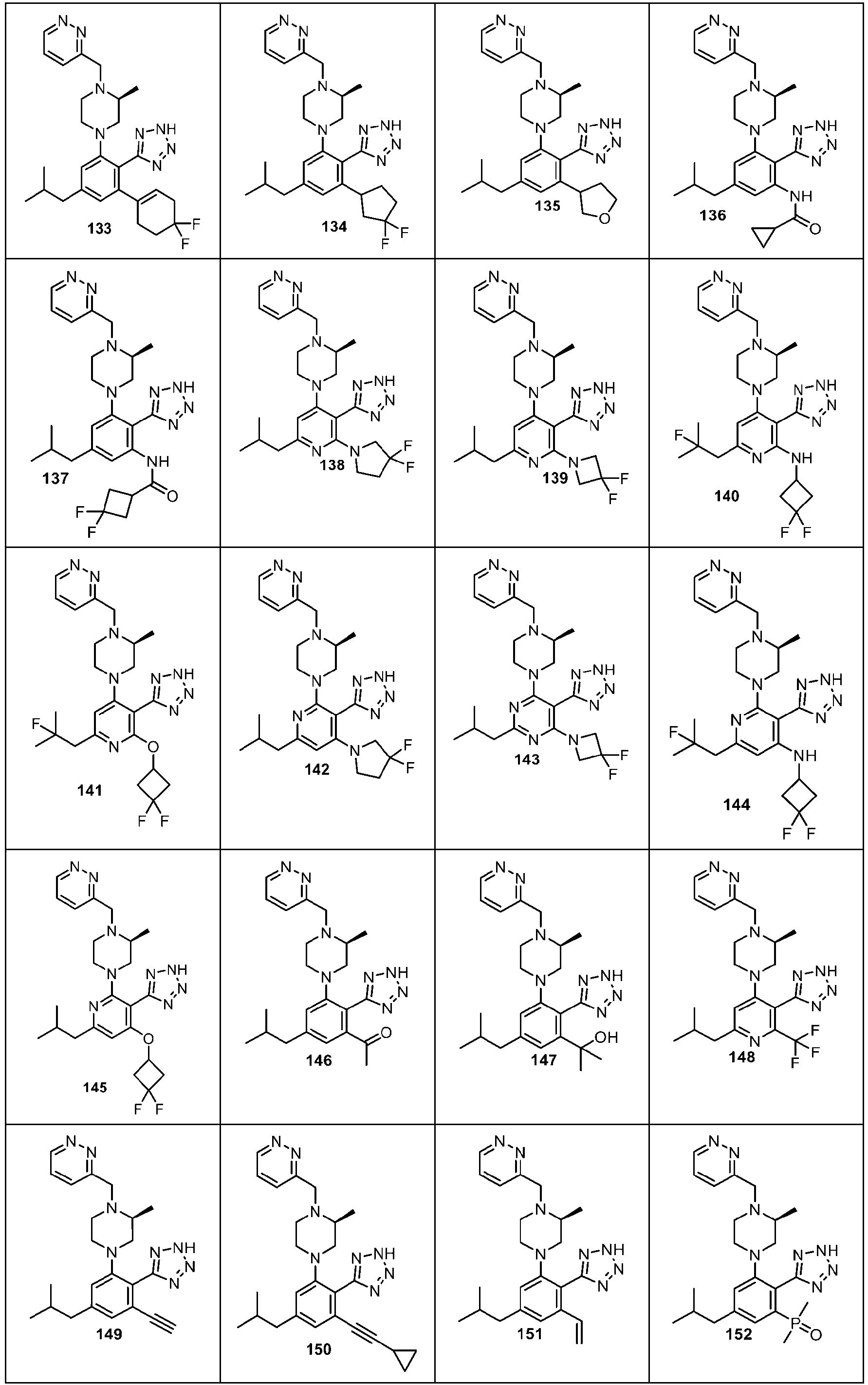

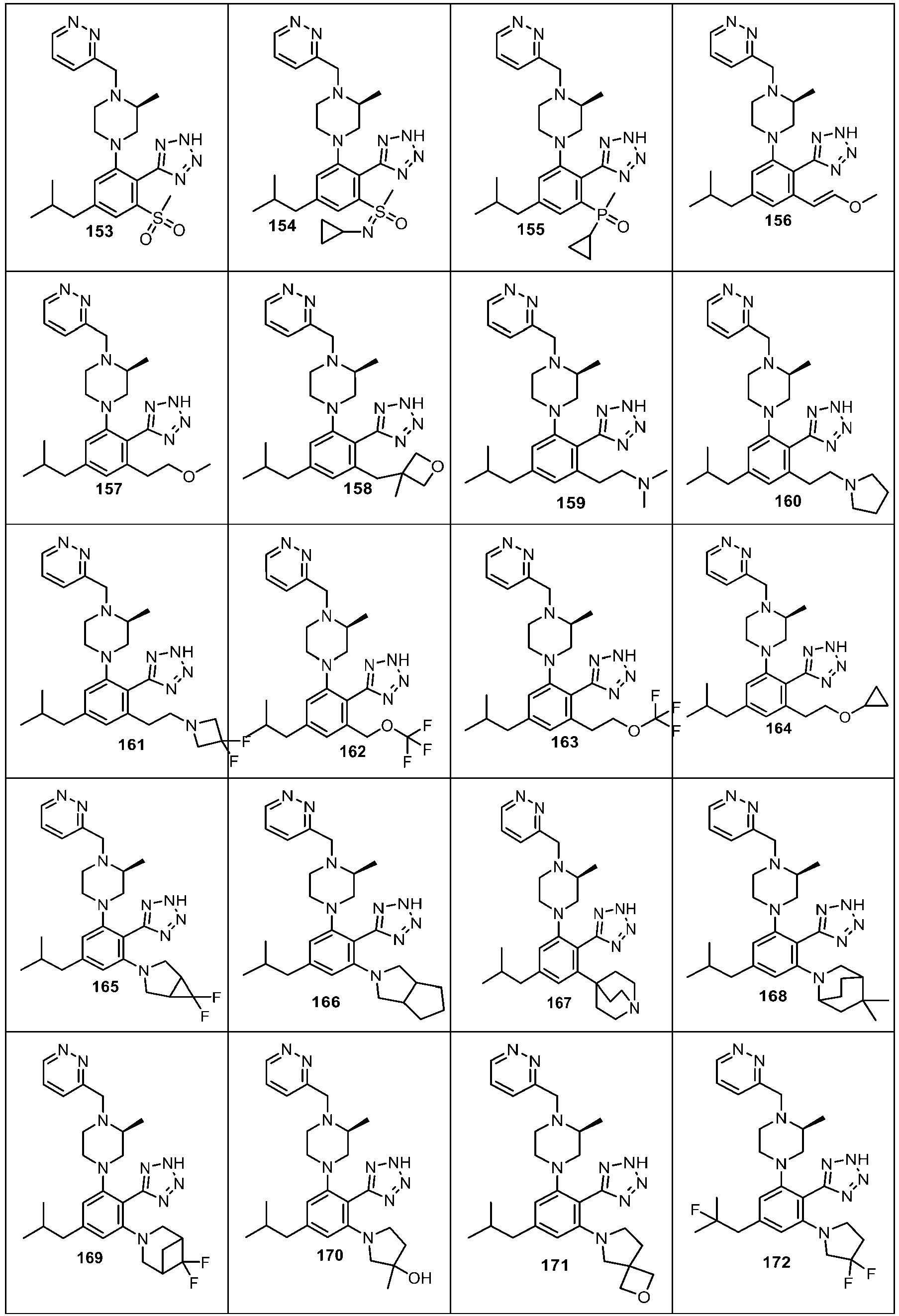

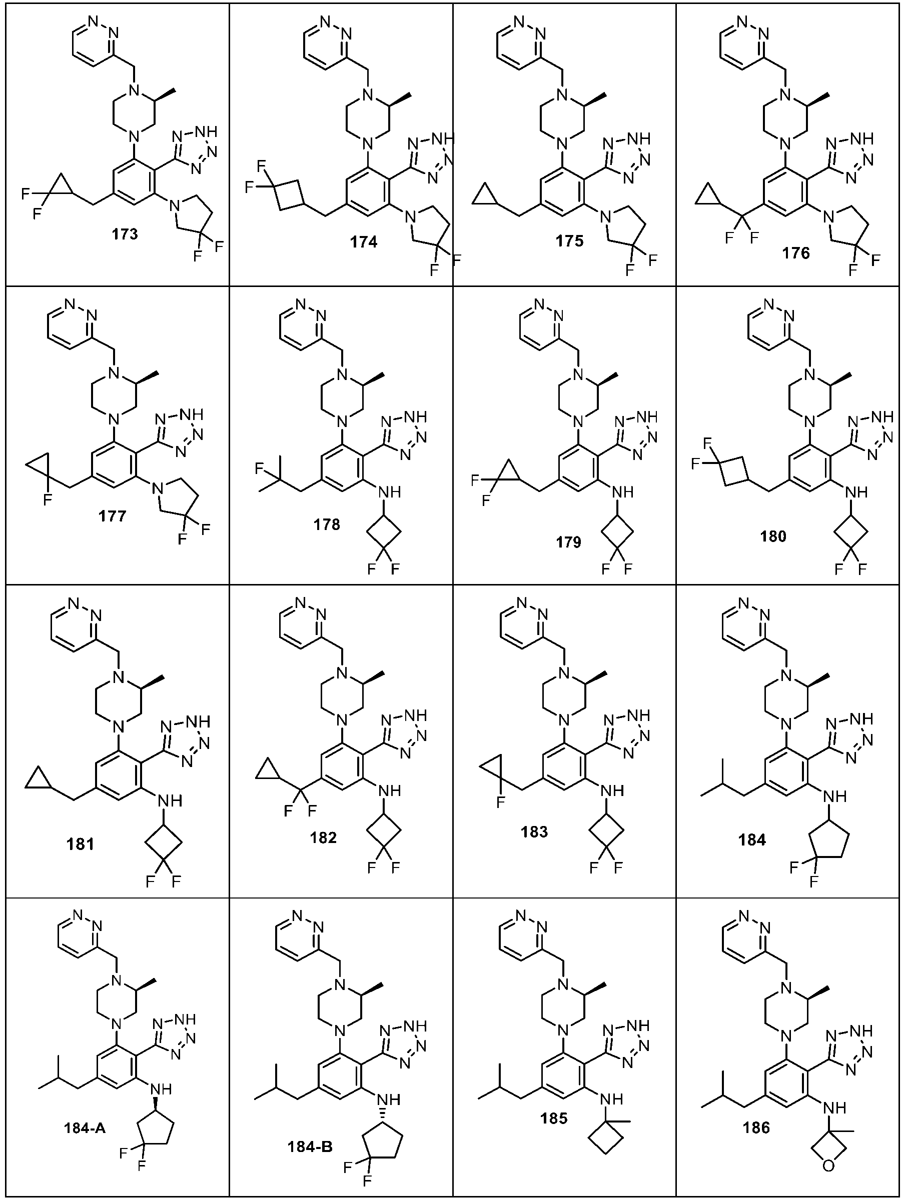

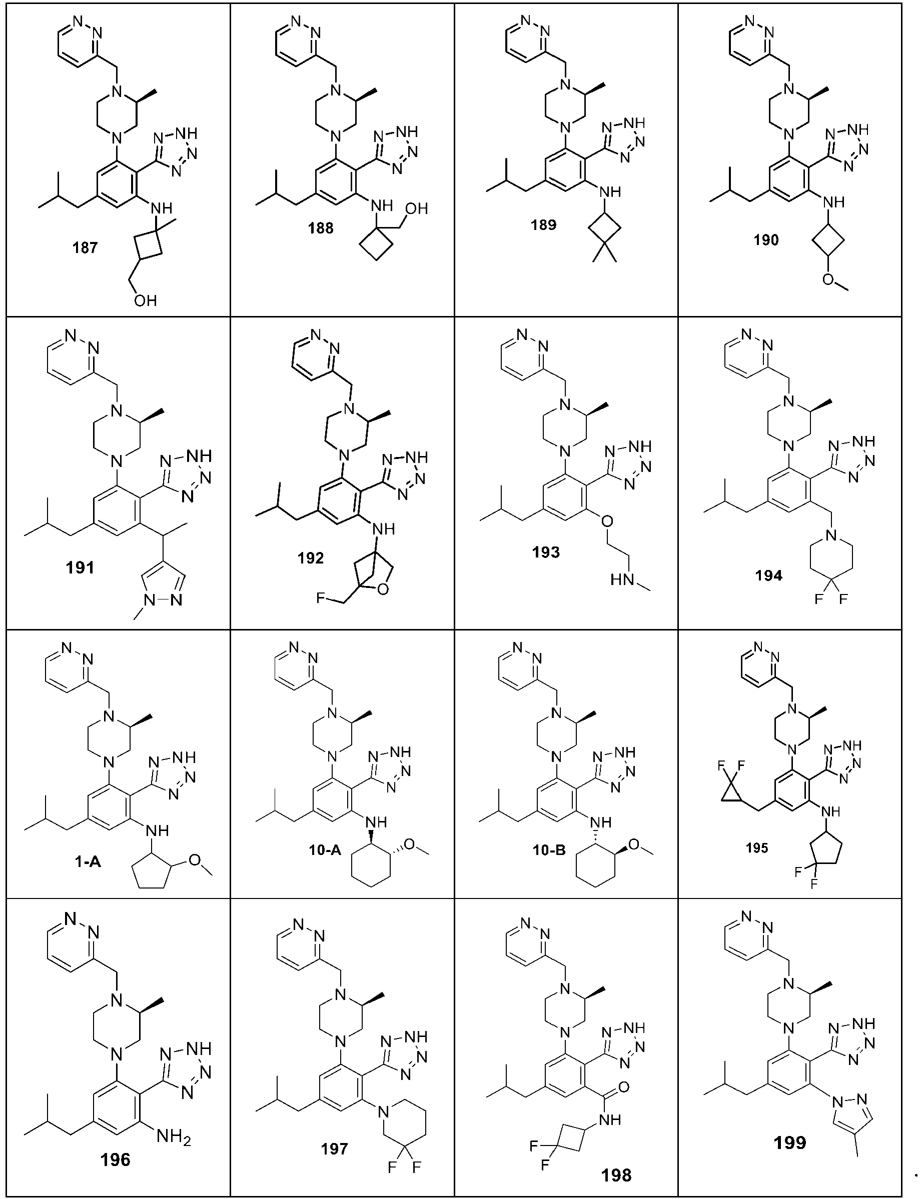

The present invention provides a pharmaceutical composition, the pharmaceutical composition comprising:
(1) the compound represented by formula (I) or a pharmaceutically acceptable salt thereof as described above, and
(2) a pharmaceutically acceptable excipient.

The present invention provides use of the compound represented by formula (I) or a pharmaceutically acceptable salt thereof as described above, or the aforementioned pharmaceutical composition in the preparation of a medicament, wherein the medicament is useful for preventing and/or treating pain; preferably, the pain is neuropathic pain.

The present invention provides use of the compound represented by formula (I) or a pharmaceutically acceptable salt thereof as described above, or the aforementioned pharmaceutical composition in the preparation of a medicament for an AT2R-mediated disease.

Without departing from the common general knowledge in the art, the above preferred conditions may be arbitrarily combined to obtain various preferred embodiments of the present invention.

### DEFINITIONS

Unless otherwise stated, the terms used in the specification and claims have the following meanings.

The term "oxo" refers to =O, wherein an oxygen atom replaces two hydrogen atoms on the same carbon atom, that is, replacing a methylene group with a carbonyl group.

The term "thio" refers to =S, wherein a sulfur atom replaces two hydrogen atoms on the same carbon atom.

The term "alkyl" refers to a saturated aliphatic hydrocarbon group, which is a straight or branched chain group containing 1 to 20 carbon atoms, preferably an alkyl containing 1 to 6 carbon atoms, and more preferably an alkyl containing 1 to 4 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, and the like, as well as various branched isomers thereof, and the like. Preferred in the present invention are methyl, ethyl, isopropyl, isobutyl, tert-butyl, haloalkyl, deuterated alkyl, alkoxy-substituted alkyl, and hydroxy-substituted alkyl.

The term "cycloalkyl" refers to a saturated or partially unsaturated cyclic hydrocarbon substituent, including monocyclic cycloalkyl, spirocyclic cycloalkyl, and bridged cycloalkyl. The cycloalkyl ring contains 3 to 10 carbon atoms, preferably contains 3 to 8 carbon atoms, and more preferably contains 3 to 4 carbon atoms. Non-limiting examples of cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The cycloalkyl can be optionally substituted or unsubstituted; when substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxy, or carboxylate.

The term "heterocyclyl" refers to a saturated or partially unsaturated cyclic hydrocarbon substituent, which contains 3 to 20 ring atoms, wherein one or more ring atoms are heteroatoms selected from nitrogen, oxygen, C(O), and S, but excluding a ring portion of - O-O-, -O-S-, or -S-S-, and the remaining ring atoms are carbon, including monocyclic heterocyclyl, spiroheterocyclyl, and bridged heterocyclyl. It preferably contains 3 to 10 ring atoms, of which 1 to 4 are heteroatoms; more preferably contains 3 to 8 ring atoms; and most preferably contains 3 to 6 ring atoms. In the present invention, "membered" refers to the number of ring atoms; for example, a 3- to 6-membered heterocyclyl refers to one containing 3 to 6 ring atoms. Non-limiting examples of heterocyclyl include oxetanyl, thietanyl, azetidinyl, tetrahydropyranyl, azepanyl, pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, and the like; preferably oxetane, thietane, azetidine, tetrahydrofuranyl, tetrahydropyranyl, 1-imino-1-oxothiane, azepanyl, piperidinyl, piperazinyl, pyridinonyl, 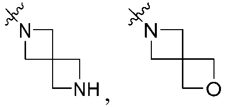 and the like.

The heterocyclyl can be optionally substituted or unsubstituted; when substituted, the substituent is preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, thio, carboxy, or carboxylate.

The term "aryl" refers to a 6- to 14-membered all-carbon monocyclic or fused polycyclic (that is, rings sharing adjacent pairs of carbon atoms) group having a conjugated π-electron system, preferably 6- to 10-membered, such as phenyl and naphthyl. More preferably phenyl.

The aryl can be substituted or unsubstituted; when substituted, the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, -P(=O)R⁵⁻¹R⁵⁻², -NHC(O)R⁵⁻¹, -NHS(O)₂R⁵⁻¹, carboxy, or carboxylate.

The term "heteroaryl" refers to a heteroaromatic system containing 1 to 4 heteroatoms and 5 to 14 ring atoms, wherein the heteroatoms are selected from oxygen, sulfur, and nitrogen. The heteroaryl is preferably a 5- to 8-membered monoheteroaryl or an 8- to 10-membered biheteroaryl, more preferably a 5-membered monoheteroaryl, a 6-membered monoheteroaryl, an 8-membered biheteroaryl, a 9-membered bicyclic heteroaryl, or a 10-membered bicyclic heteroaryl, such as imidazolyl, furyl, thienyl, thiazolyl, pyrazolyl, pyrrolyl, triazolyl, tetrazolyl, pyridinyl, pyrimidinyl, pyrazinyl, pyridazinyl, piperazinyl, thienopyrrolyl, thiazolopyrrolyl, oxazolopyrrolyl, furopyrrolyl, thienofuranyl, oxazoloimidazolyl, thiazolofuranyl, thiadiazoloimidazolyl, oxazoloimidazolyl, benzopyrrolyl, thienothiazolyl, 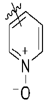 and the like.

The heteroaryl can be optionally substituted or unsubstituted; when substituted, the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxy, or carboxylate.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropoxy, cyclobutoxy, cyclopentyloxy, cyclohexyloxy. The alkoxy can be optionally substituted or unsubstituted; when substituted, the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxy, or carboxylate.

"Haloalkyl" refers to an alkyl substituted with one or more halogens, wherein the alkyl is as defined above. Non-limiting examples include: trifluoromethyl, difluoromethyl.

"Haloalkoxy" refers to an alkoxy substituted with one or more halogens, wherein the alkoxy is as defined above.

"Hydroxyalkyl" refers to an alkyl substituted with hydroxy, wherein the alkyl is as defined above. Non-limiting examples include: -CH₂OH, -C(CH₃)₂(OH).

"Amido" refers to 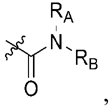 wherein R_{A} and R_{B} are each independently selected from hydrogen or C₁₋₆ alkyl; preferably, R_{A} and R_{B} are each independently selected from hydrogen or C₁₋₃ alkyl; more preferably, R_{A} and R_{B} are each independently selected from hydrogen, methyl, or ethyl.

Different terms such as "X is selected from A, B, or C", "X is selected from A, B, and C", "X is A, B, or C", and "X is A, B, and C" all express the same meaning, that is, they indicate that X can be any one or more of A, B, and C.

In the present invention, "------" represents a bond or is absent;

In the present invention, " 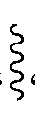 "on a substituent represents the point of attachment of the substituent to the substituted site.

In the present invention, a "-" at the end of a group indicates that the group is attached to the rest of the molecule through this site. For example, -CH₂- represents methylene.

Those skilled in the art will understand that, according to the conventions used in the art, " 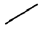 " used in the structural formulas of the groups described in the present application refers to a linking bond, and the corresponding group is connected to other fragments or groups in the compound through this " 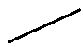 ". When a group is O, it represents a double bond (e.g., =O) or a single bond (e.g., O-).

All hydrogens described in the present invention may be replaced by their isotope deuterium, and any hydrogen in the example compounds involved in the present invention may also be replaced by a deuterium atom.

"Optional" or "optionally" means that the subsequently described event or circumstance may or may not occur, and that the description includes instances where the event or circumstance occurs and instances where it does not. For example, "a heterocyclic group optionally substituted with alkyl" means that the alkyl may or may not be present, and the description includes instances where the heterocyclic group is substituted with alkyl and instances where the heterocyclic group is not substituted with alkyl.

"One or more" refers to 1, 2, 3, 4, 5, 6, 7, 8, 9, or more, preferably 1, 2, 3, or 4.

"Substituted" means that one or more hydrogen atoms in a group, preferably up to 5, and more preferably 1 to 3 hydrogen atoms, are independently replaced by a corresponding number of substituents. It is understood that substituents only exist at their possible chemical positions, and those skilled in the art can determine (experimentally or theoretically) possible or impossible substitutions without undue effort. For example, an amino or hydroxy group with a free hydrogen may be unstable when attached to a carbon atom having an unsaturated (e.g., olefinic) bond.

"Pharmaceutical composition" refers to a mixture containing one or more of the compounds described herein, or physiologically/pharmaceutically acceptable salts or prodrugs thereof, with other chemical components, as well as other components such as physiologically/pharmaceutically acceptable carriers and excipients. The purpose of a pharmaceutical composition is to facilitate administration to an organism, favoring the absorption of the active ingredient to thereby exert biological activity.

"Pharmaceutically acceptable" refers to being relatively non-toxic, safe, and suitable for use in patients.

"Pharmaceutically acceptable salt" refers to a salt obtained by reacting a compound with a pharmaceutically acceptable acid or base. When the compound contains a relatively acidic functional group, a base addition salt can be obtained by contacting the compound with a sufficient amount of a pharmaceutically acceptable base in a suitable inert solvent. When the compound contains a relatively basic functional group, an acid addition salt can be obtained by contacting the compound with a sufficient amount of a pharmaceutically acceptable acid in a suitable inert solvent. Specific reference can be made to the Handbook of Pharmaceutical Salts: Properties, Selection, and Use (P. Heinrich Stahl, Camille G. Wermuth, 2011, 2nd Revised Edition).

In the present invention, "NA" means "not tested".

Unless otherwise specified, all technical and scientific terms used herein have their standard meanings in the art to which the claimed subject matter belongs. If there are multiple definitions for a term, the definition herein shall prevail.

Based on the common general knowledge in the art, the above preferred conditions may be arbitrarily combined to obtain various preferred embodiments of the present invention.

The reagents and raw materials used in the present invention are commercially available or prepared with reference to methods disclosed in the prior art.

For chiral carbons in the compounds of the present invention, unless otherwise specified, they are in the R configuration or S configuration.

General preparation methods for the compounds of the present invention are as follows:

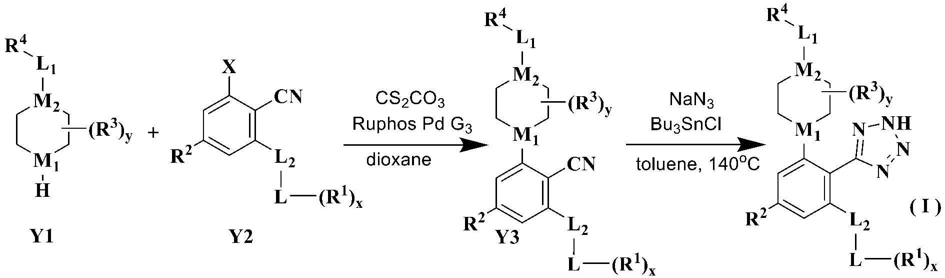

Wherein: X is halogen, and the definitions of M₁, M₂, L, L₁, L₂, R¹, R², R³, R⁴, x, and y are the same as defined above in the present invention.

### BRIEF DESCRIPTION OF THE DRAWINGS

FIG. 1 is the timeline of part 1.7 of Test Example 5.
FIG. 2 is the timeline of part 2.2.7 of Test Example 6.

### DETAILED DESCRIPTION

The present invention will be further described below by way of examples, but the present invention is not thereby limited to the scope of the examples. For experimental methods where specific conditions are not indicated in the following examples, they are carried out according to conventional methods and conditions, or selected according to product manuals. Unless otherwise specified, the reagents used in the present invention are commercially available or prepared with reference to methods disclosed in the prior art.

### Preparation of Intermediate A

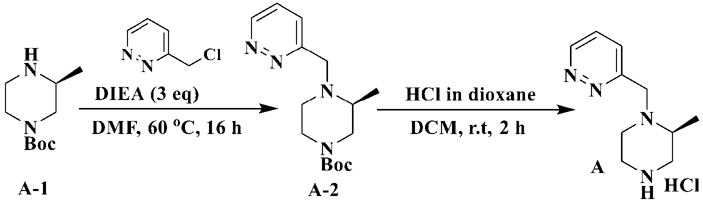

### Step 1: Preparation of Compound A-2

Under a nitrogen atmosphere at room temperature, N,N-diisopropylethylamine (5.81 g, 44.94 mmol) and 3-(chloromethyl)pyridazine (2.31 g, 17.97 mmol) were added to a solution of Compound **A-1** (3.0 g, 14.98 mmol) in N,N-dimethylformamide (30 mL). The reaction mixture was heated to 60 °C and stirred for 16 hours. After the reaction was completed, the reaction mixture was quenched by adding ice water (50 mL) and extracted with ethyl acetate (50 mL x 3). The combined organic layers were washed with saturated brine (50 mL), dried over anhydrous sodium sulfate and concentrated. The resulting residue was purified by silica gel column chromatography with methanol/dichloromethane (0 - 5%) to give Compound **A-2** (2.5 g, yield 57.08%). LCMS (ESI, m/z): 293.0 [M+H]⁺.

### Step 2: Preparation of Intermediate A

At room temperature, a solution of hydrogen chloride in 1,4-dioxane (8 mL, 4 M) was added to a solution of Compound **A-2** (2.5 g, 8.55 mmol) in dichloromethane (30 mL). The reaction mixture was stirred at room temperature for 2 hours. After the reaction was completed, the reaction mixture was concentrated. The resulting residue afforded the product (S)-3-((2-methylpiperazin-1-yl)methyl)pyridazine hydrochloride as a yellow solid without purification (2.4 g of Intermediate **A,** crude). LCMS (ESI, m/z): 193.0 [M+H]⁺.

### Example 1 Preparation of Compound 1

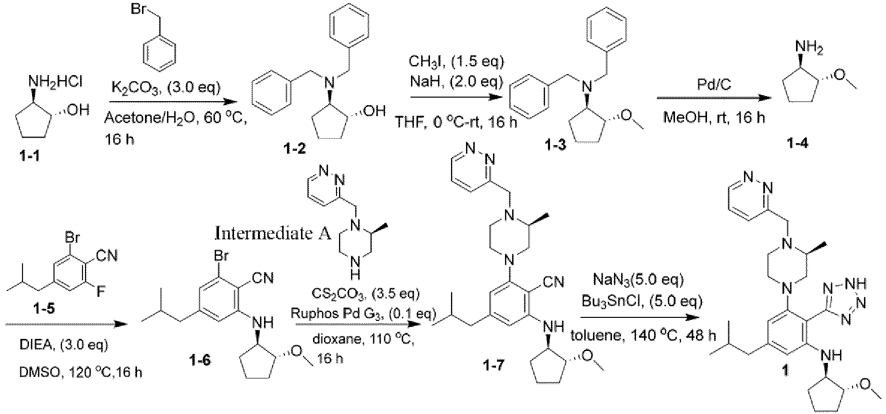

### Step 1: Preparation of Compound 1-2

Under a nitrogen atmosphere, potassium carbonate (6 g, 29 mmol) and benzyl bromide (5 g, 29 mmol) were respectively added to a solution of (1R,2R)-2-aminocyclopentanol hydrochloride (2 g, 14.5 mmol) in acetone/water (v:v = 5:1) (55 mL) at room temperature. The reaction was carried out at 60 °C for 16 hours, and the desired product was detected by LCMS. The reaction mixture was dried over anhydrous sodium sulfate, filtered, and concentrated to dryness. The resulting residue was directly purified by column chromatography (ethyl acetate/petroleum ether = 10%) to give Compound **1-2** as a yellow oil (3.8 g, 92%). LCMS (ESI, m/z): 282.1 [M+H]⁺.

### Step 2: Preparation of Compound 1-3

Under a nitrogen atmosphere, sodium hydride (60% dispersion in mineral oil) (1.1 g, 27 mmol) was added to a solution of Compound **1-2** (3.8 g, 13.5 mmol) in tetrahydrofuran (50 mL) at 0 °C, and the reaction was carried out at room temperature for 1 hour. Methyl iodide (2.9 g, 20.25 mmol) was added under an ice bath, and the reaction was carried out at room temperature for 16 hours. The desired product was detected by LCMS, and the reaction mixture was poured into ice water (50 mL). The system was extracted with ethyl acetate (2 x 50 mL). The combined organic phases were washed with saturated brine (50 mL), dried over anhydrous sodium sulfate, and concentrated to dryness. The resulting residue was directly purified by column chromatography (ethyl acetate/petroleum ether = 10%) to give Compound **1-3** as a colorless oil (3.8 g, 95%). LCMS (ESI, m/z): 296.1 [M+H]⁺.

### Step 3: Preparation of Compound 1-4

Under a hydrogen atmosphere, Pd/C (310 mg) was added to a solution of Compound **1-3** (3.1 g, 10.5 mmol) in methanol (40 mL) at room temperature, and the reaction was carried out at room temperature for 16 hours. The desired product was detected by LCMS, and the system was filtered and concentrated to dryness. The resulting residue was directly purified by column chromatography (methanol/dichloromethane = 5%) to give Compound **1-4** as a colorless oil (700 mg, 58%). LCMS (ESI, m/z): 116.2 [M+H]⁺.

### Step 4: Preparation of Compound 1-6

Under a nitrogen atmosphere, Compound **1-5** (890 mg, 3.48 mmol) and N,N-diisopropylethylamine (1.3 g, 10.44 mmol) were respectively added to a solution of Compound **1-4** (400 mg, 3.48 mmol) in dimethyl sulfoxide (5 mL). The temperature was raised to 120 °C and the reaction was carried out for 16 hours. The desired product was detected by LCMS, and the reaction mixture was poured into water (20 mL). The system was extracted with ethyl acetate (2 x 30 mL). The combined organic phases were washed with saturated brine (30 mL), dried over anhydrous sodium sulfate, and concentrated to dryness. The resulting residue was directly purified by column chromatography (methanol/dichloromethane = 5%) to give Compound **1-6** as a yellow solid (700 mg, 58%). LCMS (ESI, m/z): 351.0 [M+H]⁺.

### Step 5: Preparation of Compound 1-7

Under a nitrogen atmosphere, Intermediate A hydrochloride (187.4 mg, 0.82 mmol), cesium carbonate (932 mg, 2.87 mmol), and (2-dicyclohexylphosphino-2',6'-diisopropoxy-1,1'-biphenyl)(2-amino-1,1'-biphenyl-2-yl)palladium(II) methanesulfonate (70 mg, 0.082 mmol) were respectively added to a solution of Compound **1-6** (285 mg, 0.82 mmol) in anhydrous 1,4-dioxane (5 mL). The temperature was raised to 110 °C and the reaction was carried out for 16 hours. The desired product was detected by LCMS, and the reaction mixture was poured into water (20 mL). The system was extracted with ethyl acetate (2 x 30 mL). The combined organic phases were washed with saturated brine (30 mL), dried over anhydrous sodium sulfate, and concentrated to dryness. The resulting residue was directly purified by column chromatography (methanol/dichloromethane = 5%) to give Compound **1-7** as a yellow oil (200 mg, 53%). LCMS (ESI, m/z): 463.4 [M+H]⁺.

### Step 6: Preparation of Compound 1

Compound **1-7** (100 mg, 0.21 mmol) and toluene (2 mL) were added to a 10 mL sealed tube, and tri-n-butyltin chloride (352 mg, 1.08 mmol) and sodium azide (71 mg, 1.08 mmol) were respectively added. The reaction was then carried out at 140 °C for 48 hours and monitored by LCMS. After the reaction was completed, the resulting residue was concentrated under reduced pressure. The resulting residue was directly purified by column chromatography (methanol/dichloromethane = 5%) to give a crude yellow solid (120 mg). The obtained crude product was purified by preparative high-performance liquid chromatography. Purification conditions: (Column specifications: Sunfire C18 5 µm, 19 mm * 250 mm; Mobile phase A: water (0.1% formic acid), Mobile phase B: acetonitrile; flow rate: 20 mL/min; elution gradient: 30% B to 49% B over 17 minutes; Detection wavelength: UV 254 nm/214 nm; Retention time (minutes): 7.6-9.4) to give Compound 1 (10.45 mg, 9.8%). LCMS (ESI, m/z): 506.4 [M+H]⁺.

¹H NMR (400 MHz, DMSO-d₆) δ 9.14 - 9.09 (m, 1H), 7.72 - 7.62 (m, 2H), 6.42 (s, 1H), 6.39 - 6.25 (m, 2H), 4.15 (d, J = 14.4 Hz, 1H), 3.68 - 3.60 (m, 2H), 3.50 - 3.47 (m, 1H), 3.23 (s, 3H), 2.76 - 2.64 (m, 3H), 2.60 - 2.53 (m, 2H), 2.47 - 2.33 (m, 4H), 2.11 - 2.02 (m, 1H), 1.92 - 1.82 (m, 1H), 1.77 - 1.68 (m, 1H), 1.66 - 1.56 (m, 3H), 1.38 - 1.28 (m, 1H), 0.95 (d, J = 6.0 Hz, 3H), 0.93 - 0.88 (m, 6H).

### Example 2 Preparation of Compound 2

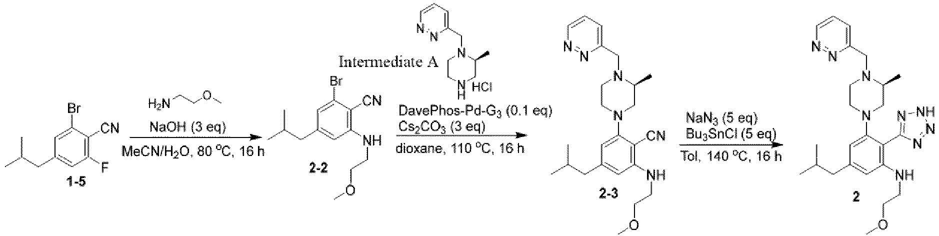

### Step 1: Preparation of Compound 2-2

Under a nitrogen atmosphere, sodium hydroxide (281.0 mg, 7.03 mmol) and 2-methoxyethan-1-amine (294.0 mg, 3.51 mmol) were added to a solution of Compound **1-5** (600.0 mg, 2.34 mmol) in acetonitrile and water (10 mL / 2 mL) at room temperature. The reaction mixture was heated to 80 °C and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with ethyl acetate/petroleum ether (0 - 20%) to give the product Compound **2-2** as a yellow oil (200.0 mg, yield 27.4%). LCMS (ESI, m/z): 311.0 [M+H]⁺.

### Step 2: Preparation of Compound 2-3

At room temperature, Intermediate A hydrochloride (147.0 mg, 0.64 mmol), methanesulfonato2-(dicyclohexylphosphino)-2'-(N,N-dimethylamino)-1,1'-biphenylpalladium(II) (49 mg, 0.064 mmol), and cesium carbonate (628.0 mg, 1.93 mmol) were added to a solution of Compound **2-2** (200.0 mg, 0.64 mmol) in 1,4-dioxane (5 mL) and water (1 mL). The reaction mixture was heated to 110 °C under a nitrogen atmosphere and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give the product Compound **2-3** as a yellow solid (50.0 mg, yield 18.41%). LCMS (ESI, m/z): 423.1 [M+H]⁺.

### Step 3: Preparation of Compound 2

Under a nitrogen atmosphere, sodium azide (38.46 mg, 0.591 mmol) and tri-n-butyltin azide (192.57 mg, 0.591 mmol) were added to a solution of Compound **2-3** (50.0 mg, 0.118 mmol) in toluene (2 mL) in a microwave tube. The reaction mixture was heated to 140 °C and stirred for 16 hours. After the reaction was completed, the reaction mixture was filtered through solid potassium fluoride, the solid was washed with ethyl acetate, and the filtrate was concentrated. The resulting residue was directly purified by column chromatography with methanol/dichloromethane (0 - 10%) to give a crude yellow solid. The obtained crude product was purified by preparative high-performance liquid chromatography. Purification conditions: (Column specifications: Sunfire C18 10 µm, 19 mm * 250 mm; Mobile phase A: water (0.1% formic acid), Mobile phase B: acetonitrile; flow rate: 20 mL/min; elution gradient: 27 - 37% B, over 18 minutes; Detection wavelength: UV 254 nm/214 nm; Retention time (minutes): 6.8 - 8.1) to give Compound **2** (8.58 mg, yield 15.57%). LCMS (ESI, m/z): 466.2 [M+H]⁺.

¹H NMR (400 MHz, CD₃OD) δ 9.09 (d, J = 4.4 Hz, 1H), 7.86 (d, J = 8.4 Hz, 1H), 7.76 - 7.66 (m, 1H), 6.43 (s, 2H), 4.30 (d, J = 14.4 Hz, 1H), 3.75 (d, J = 14.4 Hz, 1H), 3.63 - 3.52 (m, 2H), 3.34 (s, 5H), 2.97 - 2.84 (m, 3H), 2.79 - 2.60 (m, 3H), 2.58 - 2.48 (m, 1H), 2.46 (d, J = 7.2 Hz, 2H), 1.99 - 1.79 (m, 1H), 1.08 (d, J = 5.6 Hz, 3H), 0.94 (d, J = 6.4 Hz, 6H).

### Example 3 Preparation of Compound 3

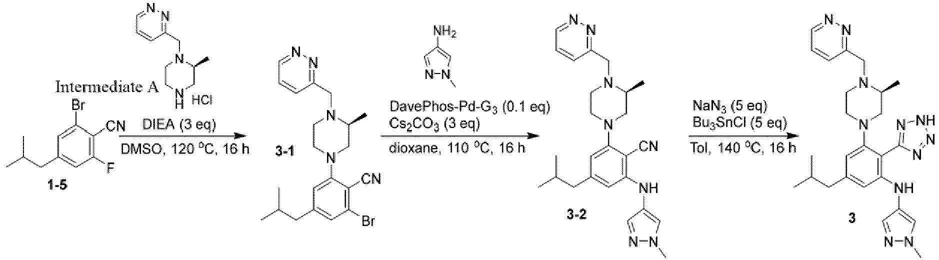

### Step 1: Preparation of Compound 3-1

Under a nitrogen atmosphere, N,N-diisopropylethylamine (3.03 g, 23.43 mmol) and Intermediate A (1.5 g, 7.81 mmol) were added to a solution of Compound **1-5** (2.0 g, 7.81 mmol) in dimethyl sulfoxide (20 mL) at room temperature. The reaction mixture was heated to 120 °C and stirred for 16 hours. After the reaction was completed, the reaction mixture was quenched by adding ice water (50 mL) and extracted with ethyl acetate (50 mL x 3). The combined organic layers were washed with saturated brine (50 mL), dried over anhydrous sodium sulfate and concentrated. The resulting residue was purified by silica gel column chromatography with ethyl acetate/petroleum ether (0 - 20%) to give the product Compound **3-1** as a yellow oil (2.3 g, yield 68.76%). LCMS (ESI, m/z): 428.1 [M+H]⁺.

### Step 2: Preparation of Compound 3-2

At room temperature, 1-methyl-1H-pyrazol-4-amine (102.0 mg, 1.05 mmol), methanesulfonato2-(dicyclohexylphosphino)-2'-(N,N-dimethylamino)-1,1'-biphenylpalladium(II) (54 mg, 0.07 mmol), and cesium carbonate (685 mg, 2.1 mmol) were added to a solution of Compound **3-1** (300.0 mg, 0.7 mmol) in 1,4-dioxane (5 mL). The reaction mixture was heated to 110 °C under a nitrogen atmosphere and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give the product Compound **3-2** as a yellow solid (83.0 mg, yield 26.66%). LCMS (ESI, m/z): 445.1 [M+H]⁺.

### Step 3: Preparation of Compound 3

Under a nitrogen atmosphere, sodium azide (61 mg, 0.933 mmol) and tri-n-butyltin azide (303.8 mg, 0.933 mmol) were added to a solution of Compound **3-2** (83.0 mg, 0.187 mmol) in toluene (2 mL) in a microwave tube. The reaction mixture was heated to 140 °C and stirred for 16 hours. After the reaction was completed, the reaction mixture was filtered through solid potassium fluoride, the solid was washed with ethyl acetate, and the filtrate was concentrated. The resulting residue was directly purified by column chromatography with methanol/dichloromethane (0 - 10%) to give a crude yellow solid. The obtained crude product was purified by preparative high-performance liquid chromatography. Purification conditions: (Column specifications: Sunfire C18 10 µm, 19 mm * 250 mm; Mobile phase A: water (0.1% formic acid), Mobile phase B: acetonitrile; flow rate: 20 mL/min; elution gradient: 20 - 30% B over 17 minutes; Detection wavelength: UV 254 nm/214 nm; Retention time (minutes): 7.5 - 9.6) to give Compound **3** (14.89 mg, yield 16.36%). LCMS (ESI, m/z): 488.1 [M+H]⁺.

¹H NMR (400 MHz, CD₃OD) δ 9.17 - 9.01 (m, 1H), 7.92 - 7.80 (m, 1H), 7.78 - 7.66 (m, 1H), 7.55 (s, 1H), 7.37 (s, 1H), 6.52 (d, J = 11.2 Hz, 2H), 4.34 (d, J = 14.4 Hz, 1H), 3.87 (s, 3H), 3.80 (d, J = 14.4 Hz, 1H), 3.03 - 2.89 (m, 3H), 2.81 - 2.67 (m, 3H), 2.62 - 2.52 (m, 1H), 2.38 (d, J = 7.2 Hz, 2H), 1.93 - 1.74 (m, 1H), 1.11 (d, J = 6.0 Hz, 3H), 0.89 (d, J = 6.4 Hz, 6H).

### Example 4 Preparation of Compound 4

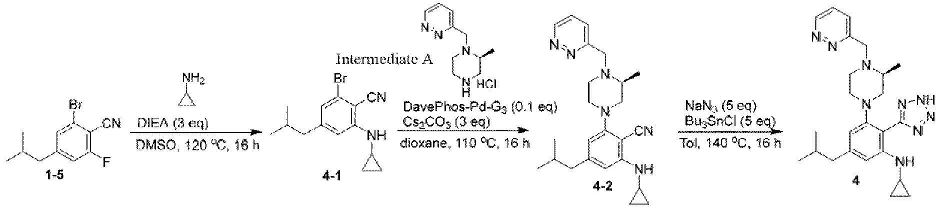

### Step 1: Preparation of Compound 4-1

Under a nitrogen atmosphere, N,N-diisopropylethylamine (756.96 mg, 5.86 mmol) and cyclopropanamine (222.9 mg, 3.90 mmol) were added to a solution of Compound 1-5 (500.0 mg, 1.95 mmol) in dimethyl sulfoxide (5 mL) at room temperature. The reaction mixture was heated to 120 °C and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with ethyl acetate/petroleum ether (0 - 20%) to give the product Compound **4-1** as a yellow oil (160.0 mg, yield 27.95%). LCMS (ESI, m/z): 333.9 [M+CH₃CN]⁺.

### Step 2: Preparation of Compound 4-2

At room temperature, Intermediate **A** hydrochloride (124.8 mg, 0.54 mmol), methanesulfonato2-(dicyclohexylphosphino)-2'-(N,N-dimethylamino)-1,1'-biphenylpalladium(II) (42 mg, 0.054 mmol), and cesium carbonate (533.4 mg, 1.64 mmol) were added to a solution of Compound **4-1** (160.0 mg, 0.54 mmol) in 1,4-dioxane (5 mL) and water (1 mL). The reaction mixture was heated to 110 °C under a nitrogen atmosphere and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give the product Compound **4-2** as a yellow solid (50.0 mg, yield 22.65%). LCMS (ESI, m/z): 405.2 [M+H]⁺.

### Step 3: Preparation of Compound 4

Under a nitrogen atmosphere, sodium azide (40.17 mg, 0.618 mmol) and tri-n-butyltin azide (201.15 mg, 0.618 mmol) were added to a solution of Compound **4-2** (50.0 mg, 0.123 mmol) in toluene (2 mL) in a microwave tube. The reaction mixture was heated to 140 °C and stirred for 16 hours. After the reaction was completed, the reaction mixture was filtered through solid potassium fluoride, the solid was washed with ethyl acetate, and the filtrate was concentrated. The resulting residue was directly purified by column chromatography with methanol/dichloromethane (0 - 10%) to give a crude yellow solid. The obtained crude product was purified by preparative high-performance liquid chromatography. Purification conditions: (Column specifications: Sunfire C18 10 µm, 19 mm * 250 mm; Mobile phase A: water (0.1% formic acid), Mobile phase B: acetonitrile; flow rate: 20 mL/min; elution gradient: 28 - 38% B over 17 minutes; Detection wavelength: UV 254 nm/214 nm; Retention time (minutes): 8.5 - 10) to give Compound 4 (2.83 mg, yield 5.12%). LCMS (ESI, m/z): 448.1 [M+H]⁺.

¹H NMR (400 MHz, CD₃OD) δ 9.09 (dd, J = 4.9, 1.6 Hz, 1H), 7.87 (dd, J = 8.5, 1.6 Hz, 1H), 7.72 (dd, J = 8.5, 4.9 Hz, 1H), 6.83 (s, 1H), 6.47 (s, 1H), 4.31 (d, J = 14.5 Hz, 1H), 3.76 (d, J = 14.4 Hz, 1H), 2.93 (d, J = 9.4 Hz, 3H), 2.83 - 2.64 (m, 3H), 2.56 (dd, J = 12.2, 6.2 Hz, 1H), 2.51 - 2.40 (m, 3H), 2.05 - 1.81 (m, 1H), 1.09 (d, J = 5.8 Hz, 3H), 0.95 (d, J = 6.6 Hz, 6H), 0.83 - 0.72 (m, 2H), 0.53 - 0.42 (m, 2H).

### Example 5 Preparation of Compound 5

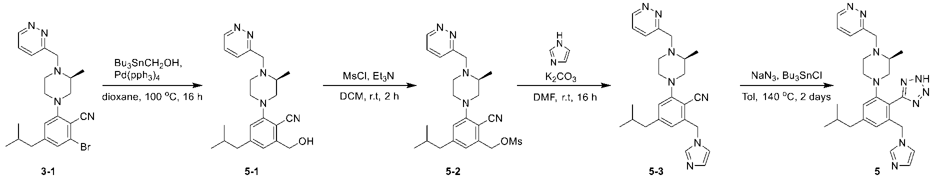

### Step 1: Preparation of Compound 5-1

Under a nitrogen atmosphere, (hydroxymethyl)tributylstannane (282.0 mg, 0.87 mmol, 1.5 equiv) and tetrakis(triphenylphosphine)palladium (67.0 mg, 0.058 mmol, 0.1 equiv) were added to a solution of (S)-2-bromo-4-isobutyl-6-(3-methyl-4-(pyridazin-3-ylmethyl)piperazin-1-yl)benzonitrile (250.0 mg, 0.58 mmol, 1.0 equiv) in dioxane (5 mL) in a microwave tube. The reaction mixture was heated to 100 °C and stirred for 16 hours. After the reaction was completed, it was quenched with water and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give yellow Compound 5-1 (130 mg, yield 59.1%). LCMS: (ESI, m/z): 380.2 [M+H]⁺.

### Step 2: Preparation of Compound 5-2

At room temperature, triethylamine (103.0 mg, 1.02 mmol, 3.0 equiv) and methanesulfonyl chloride (78.2 mg, 0.68 mmol, 2.0 equiv) were added to a solution of (S)-2-(hydroxymethyl)-4-isobutyl-6-(3-methyl-4-(pyridazin-3-ylmethyl)piperazin-1-yl)benzonitrile (130.0 mg, 0.34 mmol, 1.0 equiv) in dichloromethane (5 mL). The reaction mixture was stirred at room temperature for 2 hours. After the reaction was completed, the reaction mixture was quenched by adding ice water (10 mL) and extracted with dichloromethane (10 mL x 3). The combined organic layers were washed with saturated brine (20 mL), dried over anhydrous sodium sulfate, and concentrated to give yellow Compound 5-2 (160 mg, crude). LCMS: (ESI, m/z): 458.2 [M+H]⁺.

### Step 3: Preparation of Compound 5-3

At room temperature, imidazole (119.0 mg, 1.75 mmol, 5.0 equiv) and potassium carbonate (150.0 mg, 1.05 mmol, 3.0 equiv) were added to a solution of (S)-2-cyano-5-isobutyl-3-(3-methyl-4-(pyridazin-3-ylmethyl)piperazin-1-yl)benzyl methanesulfonate (160.0 mg, 0.35 mmol, 1.0 equiv) in N,N-dimethylformamide (5 mL), and the reaction was carried out at room temperature for 16 hours. The desired product was detected by LCMS. After the reaction was completed, it was quenched with water and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound 5-3 as a yellow oil (120 mg, yield 80.0%). LCMS: (ESI, m/z): 430.0 [M+H]⁺.

### Step 4: Preparation of Compound 5

Under a nitrogen atmosphere, sodium azide (91.0 mg, 1.4 mmol, 5 equiv) and tri-n-butyltin azide (456.0 mg, 1.4 mmol, 5 equiv) were added to a solution of (S)-2-((1H-imidazol-1-yl)methyl)-4-isobutyl-6-(3-methyl-4-(pyridazin-3-ylmethyl)piperazin-1-yl)benzonitrile (120.0 mg, 0.28 mmol, 1.0 equiv) in toluene (4 mL) in a microwave tube. The reaction mixture was heated to 140 °C and stirred for 2 days. After the reaction was completed, the reaction mixture was filtered through solid potassium fluoride, the solid was washed with ethyl acetate, and the filtrate was concentrated. The resulting residue was directly purified by column chromatography with methanol/dichloromethane (0 - 10%) to give a crude yellow solid. The obtained crude product was purified by preparative high-performance liquid chromatography. Purification conditions: (Column specifications: Sunfire C18 10 µm, 19 mm * 250 mm; Mobile phase A: water (0.1% formic acid), Mobile phase B: acetonitrile; flow rate: 20 mL/min; elution gradient: 5 - 15% B over 16 minutes; Detection wavelength: UV 254 nm/214 nm; Retention time (minutes): 9.2 - 10.5) to give Compound 5 (30.17 mg, yield 22.8%). LCMS: (ESI, m/z): 473.2 [M+H]⁺.

¹H NMR (400 MHz, DMSO-d₆) δ 9.11 - 9.10 (m, 1H), 8.13 (s, 0.62HCOOH), 7.65 - 7.64 (m, 2H), 7.40 (s, 1H), 6.99 (s, 1H), 6.91 (s, 1H), 6.86 (s, 1H), 6.79 (s, 1H), 5.00 (s, 2H), 4.06 (d, J = 14.0 Hz, 1H), 3.59 (d, J = 14.0 Hz, 1H), 2.70 - 2.65 (m, 3H), 2.48 - 2.44 (m, 4H), 2.36 - 2.29 (m, 1H), 2.12 - 2.05 (m, 1H), 1.86 - 1.79 (m, 1H), 0.85 (d, J = 6.4 Hz, 9H).

### Example 6 Preparation of Compound 6

Replacing Compound 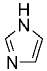 with Compound 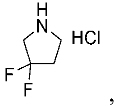 referring to the synthesis method of Compound 5, Compound 6 (11.48 mg, yield 13.14%) was obtained. LCMS: (ESI, m/z): 512.4 [M+H]⁺.

¹H NMR (400 MHz, CD₃OD) δ 9.09 (d, *J* = 3.6 Hz, 1H), 7.82 (d, *J* = 8.4 Hz, 1H), 7.73 - 7.69 (m, 1H), 7.05 (s, 1H), 6.99 (s, 1H), 4.26 (d, *J* = 14.4 Hz, 1H), 3.77 (d, *J* = 14.4 Hz, 1H), 3.52 (s, 2H), 2.86 - 2.83 (m, 3H), 2.71 - 2.61 (m, 4H), 2.57 - 2.50 (m, 5H), 2.37 - 2.31 (m, 1H), 2.12 - 2.02 (m, 2H), 1.95 - 1.88 (m, 1H), 0.98 (d, *J* = 6.0 Hz, 3H), 0.93 (d, *J* = 6.4 Hz, 6H).

### Example 7 Preparation of Compound 7

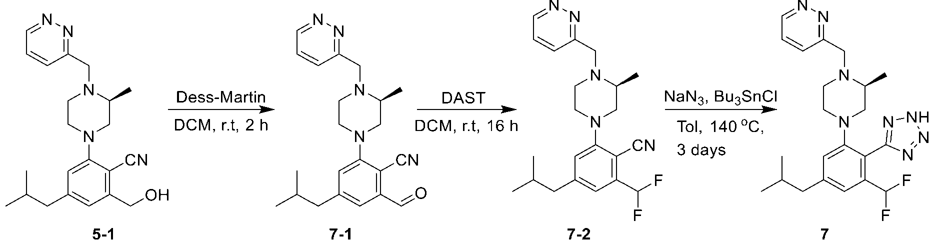

### Step 1: Preparation of Compound 7-1

At room temperature, Dess-Martin periodinane (336.0 mg, 0.792 mmol, 1.5 equiv) was added to a solution of (S)-2-(hydroxymethyl)-4-isobutyl-6-(3-methyl-4-(pyridazin-3-ylmethyl)piperazin-1-yl)benzonitrile (200.0 mg, 0.528 mmol, 1.0 equiv) in dichloromethane, and the reaction mixture was stirred at room temperature for 2 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give the product (S)-2-formyl-4-isobutyl-6-(3-methyl-4-(pyridazin-3-ylmethyl)piperazin-1-yl)benzonitrile Compound 7-1 as a yellow solid (120.0 mg, yield 60.28%). LCMS: (ESI, m/z): 378.4 [M+H]⁺.

### Step 2: Preparation of Compound 7-2

At 0 °C, diethylaminosulfur trifluoride (128.0 mg, 0.796 mmol, 2.5 equiv) was added to a solution of (S)-2-formyl-4-isobutyl-6-(3-methyl-4-(pyridazin-3-ylmethyl)piperazin-1-yl)benzonitrile (120.0 mg, 0.318 mmol, 1.0 equiv) in dichloromethane, and the reaction mixture was stirred at room temperature for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give the product (S)-2-(difluoromethyl)-4-isobutyl-6-(3-methyl-4-(pyridazin-3-ylmethyl)piperazin-1-yl)benzonitrile Compound 7-2 as a yellow solid (68.0 mg, yield 53.46%). LCMS: (ESI, m/z): 400.2 [M+H]⁺.

### Step 3: Preparation of Compound 7

Under a nitrogen atmosphere, sodium azide (62.0 mg, 0.952 mmol, 5 equiv) and tri-n-butyltin azide (309.0 mg, 0.952 mmol, 5 equiv) were added to a solution of (S)-2-(difluoromethyl)-4-isobutyl-6-(3-methyl-4-(pyridazin-3-ylmethyl)piperazin-1-yl)benzonitrile (76.0 mg, 0.190 mmol, 1.0 equiv) in toluene (4 mL) in a microwave tube. The reaction mixture was heated to 140 °C and stirred for 3 days. After the reaction was completed, the reaction mixture was filtered through solid potassium fluoride, the solid was washed with ethyl acetate, and the filtrate was concentrated. The resulting residue was directly purified by column chromatography with methanol/dichloromethane (0 - 10%) to give a crude yellow solid. The obtained crude product was purified by preparative high-performance liquid chromatography. Purification conditions: (Column specifications: Sunfire C18 10 µm, 19 mm * 250 mm; Mobile phase A: water (0.1% formic acid), Mobile phase B: acetonitrile; flow rate: 20 mL/min; elution gradient: 23 - 33% B over 16 minutes; Detection wavelength: UV 254 nm/214 nm; Retention time (minutes): 8.9 - 10.6) to give Compound 7 (42.63 mg, yield 50.76%). LCMS: (ESI, m/z): 443.5 [M+H]⁺.

¹H NMR (400 MHz, CD₃OD) δ 9.13 - 9.11 (m, 1H), 7.83 - 7.80 (m, 1H), 7.74 - 7.71 (m, 1H), 7.28 (s, 1H), 7.18 (s, 1H), 6.47 (t, *J* = 55.2 Hz, 1H), 4.39 (d, *J* = 14.4 Hz, 1H), 3.94 (d, *J* = 14.4 Hz, 1H), 2.97 - 2.92 (m, 3H), 2.86 - 2.81 (m, 1H), 2.73 - 2.68 (m, 2H), 2.61 (d, *J* = 7.2 Hz, 2H), 2.55 - 2.50 (m, 1H), 1.98 - 1.89 (m, 1H), 1.03 (d, *J= 5.6* Hz, 3H), 0.95 (d, *J* = 6.4 Hz, 6H).

### Example 8 Preparation of Compound 8

Replacing Compound 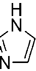 with Compound 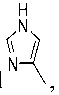 referring to the synthesis method of Compound 5, Compound 8 (19.44 mg, yield 30.5%) was obtained. LCMS: (ESI, m/z): 487.4 [M+H]⁺.

¹H NMR (400 MHz, CD₃OD) δ 9.08 (d, *J* = 4.8 Hz, 1H), 8.24 (s, 0.59 HCOOH), 7.85 - 7.78 (m, 2H), 7.71 -7.68 (m, 1H), 7.03 - 7.01 (m, 2H), 6.81 (s, 1H), 5.07 - 5.02 (mz, 2H), 4.23 (d, *J =* 14.4 Hz, 1H), 3.78 (d, *J =* 14.4 Hz, 1H), 2.93 - 2.89 (m, 2H), 2.85 - 2.81 (m, 1H), 2.70 - 2.67 (m, 1H), 2.63 - 2.58 (m, 1H), 2.55 - 2.49 (m, 3H), 2.38 - 2.33 (m, 1H), 2.19 (s, 2H), 2.01 (s, 1H), 1.95 - 1.85 (m, 1H), 0.96 - 0.91 (m, 9H).

### Example 9 Preparation of Compound 9

Replacing Compound 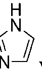 with Compound 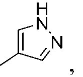 referring to the synthesis method of Compound 5, (S)-3-((4-(5-isobutyl-3-((4-methyl-1H-pyrazol-1-yl)methyl)-2-(2H-tetrazol-5-yl)phenyl)-2-methylpiperazin-1-yl)methyl)pyridazine (Compound 9, 22.24 mg, yield 22.53%) was obtained. LCMS: (ESI, m/z): 487.5 [M+H]⁺.

¹H NMR (400 MHz, MeOD) δ 9.11 (d, *J* = 4.4 Hz, 1H), 7.81 (d, *J =* 8.4 Hz, 1H), 7.73 - 7.70 (m, 1H), 7.20 (s, 1H), 6.99 (d, *J* = 5.6 Hz, 2H), 6.73 (s, 1H), 5.08 (s, 2H), 4.33 (d, *J* = 14.4 Hz, 1H), 3.85 (d, *J* = 14.4 Hz, 1H), 2.90 - 2.84 (m, 3H), 2.79 - 2.73 (m, 1H), 2.66 - 2.58 (mz, 2H), 2.48 (d, J = 7.2 Hz, 2H), 2.44 - 2.39 (m, 1H), 1.99 (s, 3H), 1.89 - 1.82 (m, 1H), 1.00 (d, *J =* 5.2 Hz, 3H), 0.89 (d, *J* = 6.4 Hz, 6H).

### Example 10 Preparation of Compound 10

Replacing Compound 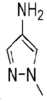 with Compound 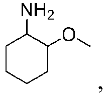 referring to the preparation method of Compound 3, 5-isobutyl-N-(2-methoxycyclohexyl)-3-((S)-3-methyl-4-(pyridazin-3-ylmethyl)piperazin-1-yl)-2-(2H-tetrazol-5-yl)aniline (Compound **10,** 25.88 mg, 8%) was obtained. LCMS: (ESI, m/z): 520 [M+H]⁺.

¹H NMR (400 MHz, DMSO-d₆) δ 9.121 - 9.105 (m, 1H), 7.694 - 7.633 (m, 2H), 6.449 - 6.356 (m, 2H), 6.258 (d, *J* = 12.4 Hz, 1H), 4.168 - 4.133 (m, 1H), 3.640 - 3.604 (m, 1H), 3.205 - 3.139 (m, 3H), 2.742 - 2.6148 (m, 3H), 2.556 - 2.526 (m, 1H), 2.450 - 2.328 (m, 5H), 1.946 - 1.844 (m, 3H), 1.588 - 1.158 (m, 8H), 0.957 - 0.922(m, 1H), 0.889 (d, *J =* 6.4 Hz, 6H).

### Example 11 Preparation of Compound 11

Replacing Compound 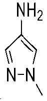 with Compound 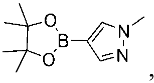 referring to the synthesis method of Compound 3, (S)-3-((4-(5-isobutyl-3-(1-methyl-1H-pyrazol-4-yl)-2-(2H-tetrazol-5-yl)phenyl)-2-methylpiperazin-1-yl)methyl)pyridazine (Compound 11, 4.69 mg, 9%) was obtained. LCMS: (ESI, m/z): 473.2 [M+H]⁺.

¹H NMR (400 MHz, DMSO-d₆) δ 9.11 - 9.09 (m, 1H), 7.67 - 7.62 (m, 2H), 7.27 (s, 1H), 7.10 (s, 1H), 6.95 (s, 1H), 6.88 (s, 1H), 4.00 (d, *J =* 14.0 Hz, 1H), 3.70 (s, 3H), 3.58 (d, *J* = 14.0 Hz, 1H), 2.87 - 2.68 (m, 4H), 2.58 - 2.54 (m, 2H), 2.45 - 2.41 (m, 1H), 2.30 - 2.22 (m, 1H), 2.00 - 1.88 (m, 2H), 0.92 (d, *J =* 6.4 Hz, 6H), 0.83 (d, *J=* 6.0 Hz, 3H).

### Example 12 Preparation of Compound 12

Replacing Compound 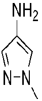 with Compound 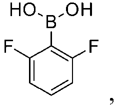 referring to the synthesis method of Compound **3,** (S)-3-((4-(2',6'-difluoro-5-isobutyl-2-(2H-tetrazol-5-yl)-[1,1'-biphenyl]-3-yl)-2-methylpiperazin-1-yl)methyl)pyridazine (Compound **12,** 10.07 mg, 4.8%) was obtained. LCMS: (ESI, m/z): 505.2 [M+H]⁺.

¹H NMR (400 MHz, DMSO-d₆) δ 9.11 - 9.10 (M, 1H), 8.18 (s, 1H), 7.68 - 7.62 (m, 2H), 7.33 - 7.26 (m, 1H), 7.09 - 7.04 (m, 1H), 6.97 - 6.86 (m, 3H), 4.05 (d, *J* = 14.0 Hz, 1H), 3.58 (d, *J* = 14.4 Hz, 1H), 2.79 - 2.67 (m, 3H), 2.54 (s, 3H), 2.46 (s, 1H), 2.35 - 2.30 (m, 1H), 2.12 - 2.07 (m, 1H), 1.94 - 1.86 (m, 1H), 0.90 (d, *J =* 6.4 Hz, 6H), 0.86 (d, *J =* 6.0 Hz, 3H).

### Example 13 Preparation of Compound 13

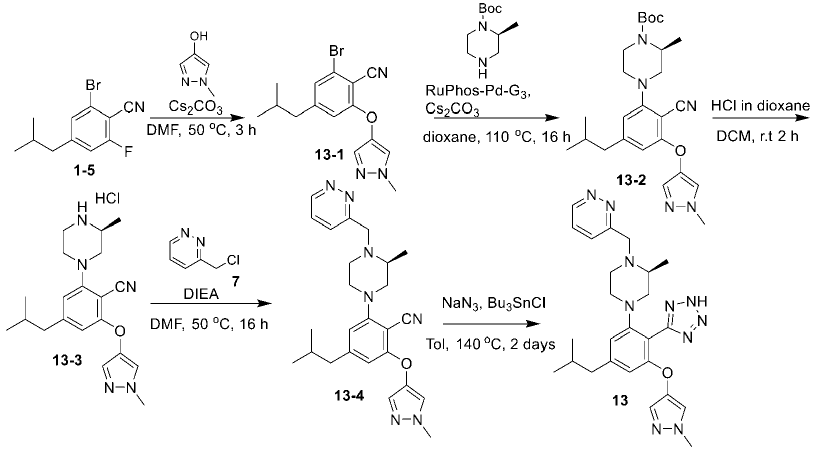

### Step 1: Preparation of Compound 13-1

Under a nitrogen atmosphere, cesium carbonate (687 mg, 2.11 mmol, 3.0 equiv) and 1-methyl-1H-pyrazol-4-ol (82.7 mg, 0.843 mmol, 1.2 equiv) were added to a solution of 2-bromo-6-fluoro-4-isobutylbenzonitrile (180.0 mg, 0.702 mmol, 1.0 equiv) in N,N-dimethylformamide (5 mL) at room temperature. The reaction mixture was heated to 50 °C and stirred for 3 hours. After the reaction was completed, it was extracted with ethyl acetate (20 mL x 3). The combined organic layers were washed with saturated brine (20 mL), dried over anhydrous sodium sulfate and concentrated. The resulting residue was purified by silica gel column chromatography with ethyl acetate/petroleum ether (0 - 20%) to give the product as a yellow oil (13-1, 210.0 mg, yield 89.40%). LCMS: (ESI, m/z): 334.0 [M+H]⁺.

### Step 2: Preparation of Compound 13-2

At room temperature, tert-butyl (S)-2-methylpiperazine-1-carboxylate (151.0 mg, 0.754 mmol, 1.2 equiv), (2-dicyclohexylphosphino-2',6'-diisopropoxy-1,1'-biphenyl)(2-amino-1,1'-biphenyl-2-yl)palladium(II) methanesulfonate (52.6 mg, 0.063 mmol, 0.1 equiv), and cesium carbonate (614.2 mg, 1.89 mmol, 3.0 equiv) were added to a solution of Compound **13-1** in 1,4-dioxane (5 mL). The reaction mixture was heated to 110 °C under a nitrogen atmosphere and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give the product as a yellow solid (13-2, 150.0 mg, yield 52.63%). LCMS: (ESI, m/z): 454.3 [M+H]⁺.

### Step 3: Preparation of Compound 13-3

At room temperature, a solution of hydrogen chloride in 1,4-dioxane (4 M, 2 mL) was added to Compound **13-2** (150 mg, 0.33 mmol, 1.0 equiv) in dichloromethane (3 mL), and the reaction was carried out at room temperature for 2 hours. The desired product was detected by LCMS. Concentration under reduced pressure gave a white solid (13-3, 170.0 mg, crude). LCMS: (ESI, m/z): 354.5 [M+H]⁺

### Step 4: Preparation of Compound 13-4

At room temperature, 3-(chloromethyl)pyridazine (67.3 mg, 0.523 mmol, 1.2 equiv) and N,N-diisopropylethylamine (169.0 mg, 1.31 mmol, 3.0 equiv) were added to a solution of Compound **13-3** (170.0 mg, 0.436 mmol, 1.0 equiv) in N,N-dimethylformamide (3 mL). The reaction mixture was heated to 50 °C under a nitrogen atmosphere and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give the product as a yellow oil (13-4, 130.0 mg, yield 66.92%). LCMS: (ESI, m/z): 446.7 [M+H]⁺.

### Step 5: Preparation of Compound 13

Under a nitrogen atmosphere, sodium azide (94.8 mg, 1.46 mmol, 5.0 equiv) and tri-n-butyltin azide (474.8 mg, 1.46 mmol, 5.0 equiv) were added to a solution of Compound 13-4 (130.0 mg, 0.291 mmol, 1.0 equiv) in toluene (3 mL) in a microwave tube. The reaction mixture was heated to 140 °C and stirred for 2 days. After the reaction was completed, the reaction mixture was filtered through solid potassium fluoride, the solid was washed with ethyl acetate, and the filtrate was concentrated. The resulting residue was directly purified by column chromatography with methanol/dichloromethane (0 - 10%) to give a crude yellow solid. The obtained crude product was purified by preparative high-performance liquid chromatography. Purification conditions: (Column specifications: Sunfire C18 10 µm, 19 mm * 250 mm; Mobile phase A: water (0.1% formic acid), Mobile phase B: acetonitrile; flow rate: 20 mL/min; elution gradient: 16 - 26% B over 16 minutes; Detection wavelength: UV 254 nm/214 nm; Retention time (minutes): 9.2 - 10.6) to give the white Compound (13, 31.87 mg, yield 22.36%). LCMS: (ESI, m/z): 489.4 [M+H]⁺.

¹H NMR (400 MHz, CD₃OD) δ 9.11 - 9.09 (m, 1H), 8.14 (s, 0.12HCOOH), 7.84 - 7.81 (m, 1H), 7.73 - 7.70 (m, 1H), 7.45 (s, 1H), 7.23 (s, 1H), 6.74 (s, 1H), 6.57 (s, 1H), 4.30 (d, *J= 14.4* Hz, 1H), 3.85 - 3.81 (m, 4H), 2.94 - 2.84 (m, 3H), 2.78 - 2.72 (m, 1H), 2.69 - 2.58 (m, 2H), 2.46 - 2.40 (m, 3H), 1.87 - 1.77 (m, 1H), 1.02 (d, *J* = 6.0 Hz, 3H), 0.89 (d, *J* = 6.4 Hz, 6H).

### Example 14 Preparation of Compound 14

Replacing Compound 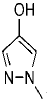 with Compound 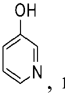 referring to the preparation method of Compound 13, (S)-3-((4-(5-isobutyl-3-(pyridin-3-yloxy)-2-(2H-tetrazol-5-yl)phenyl)-2-methylpiperazin-1-yl)methyl)pyridazine (Compound 14, 32.81 mg, yield 20.62%) was obtained. LCMS: (ESI, m/z): 486.2 [M+H]⁺.

¹H NMR (400 MHz, CD₃OD) δ 9.11 (d, *J* = 3.2 Hz, 1H), 8.21 (d, *J* = 3.2 Hz, 1H), 8.13 (d, *J =* 2.0 Hz, 1H), 7.83 (d, *J* = 8.4 Hz, 1H), 7.74 - 7.70 (m, 1H), 7.36 - 7.29 (m, 2H), 6.89 (s, 1H), 6.62 (s, 1H), 4.34 (d, *J* = 14.8 Hz, 1H), 3.87 (d, *J* = 14.0 Hz, 1H), 3.00 - 2.87 (m, 3H), 2.83 - 2.76 (m, 1H), 2.73 - 2.62 (m, 2H), 2.52 - 2.47 (m, 3H), 1.92 - 1.82 (m, 1H), 1.02 (d, *J* = 6.0 Hz, 3H), 0.92 (d, *J* = 6.4 Hz, 6H).

### Example 15 Preparation of Compound 15

Replacing Compound 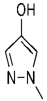 with Compound 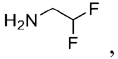 referring to the synthesis method of Compound 13, (S)-N-(2,2-difluoroethyl)-5-isobutyl-3-(3-methyl-4-(pyridazin-3-ylmethyl)piperazin-1-yl)-2-(2H-tetrazol-5-yl)aniline (Compound 15, 16.29 mg, yield 8.71%) was obtained. LCMS: (ESI, m/z): 472.4 [M+H]⁺, RT (min): 1.353.

¹H NMR (400 MHz, CD₃OD) δ 9.10 - 9.09 (m, 1H), 7.88 - 7.85 (m, 1H), 7.74 - 7.70 (m, 1H), 6.51 (d, *J* = 14.4 Hz, 2H), 6.08 - 5.78 (m, 1H), 4.34 (d, *J* = 14.4 Hz, 1H), 3.79 (d, *J* = 14.4 Hz, 1H), 3.65 - 3.57 (m, 2H), 2.94 - 2.91 (m, 3H), 2.81 - 2.66 (m, 3H), 2.60 - 2.53 (m, 1H), 2.47 (d, *J =* 7.2 Hz, 2H), 1.95 - 1.88 (m, 1H), 1.09 (d, *J =* 6.0 Hz, 3H), 0.94 (d, *J=* 6.4 Hz, 6H).

### Example 16 Preparation of Compound 16

Replacing Compound 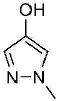 with Compound 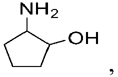 referring to the synthesis method of Compound 13, 2-((5-isobutyl-3-((S)-3-methyl-4-(pyridazin-3-ylmethyl)piperazin-1-yl)-2-(2H-tetrazol-5-yl)phenyl)amino)cyclopentan-1-ol (Compound 16, 36.65 mg, yield 25.74%) was obtained. LCMS: (ESI, m/z): 492.6 [M+H]⁺.

¹H NMR (400 MHz, CD₃OD) δ 9.18 (d, *J* = 3.6 Hz, 1H), 7.88 - 7.86 (m, 1H), 7.79 - 7.76 (m, 1H), 6.61 (s, 1H), 6.46 (s, 1H), 4.64 (d, *J* = 13.6 Hz, 1H), 4.16 (d, *J* = 14.8 Hz, 1H), 4.01 - 3.97 (m, 1H), 3.67 - 3.64 (m, 1H), 3.21 - 3.12 (m, 2H), 3.07 - 3.03 (m, 3H), 2.97 - 2.92 (m, 1H), 2.88 - 2.83 (m, 1H), 2.49 (d, *J= 7.2* Hz, 2H), 2.27 - 2.22 (m, 1H), 1.97 - 1.87 (m, 2H), 1.85 - 1.71 (m, 2H), 1.66 - 1.60 (m, 1H), 1.52 - 1.42 (m, 1H), 1.25 (d, *J* = 6.0 Hz, 3H), 0.95 (d, *J* = 6.4 Hz, 6H).

### Example 17 Preparation of Compound 17

Replacing Compound 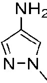 with Compound 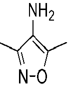 , referring to the synthesis method of Compound **3,** (S)-N-(5-isobutyl-3-(3-methyl-4-(pyridazin-3-ylmethyl)piperazin-1-yl)-2-(2H-tetrazol-5-yl)phenyl)-3,5-dimethylisoxazol-4-amine (Compound **17,** 28.66 mg, yield 23.89%) was obtained. LCMS: (ESI, m/z): 503.4 [M+H]⁺.

¹H NMR (400 MHz, CD₃OD) δ 9.26 (s, 1H), 7.83 (d, *J* = 2.8 Hz, 2H), 6.58 (s, 1H), 6.14 (s, 1H), 3.57 - 3.40 (m, 4H), 3.21 - 3.12 (m, 4H), 3.04 - 2.93 (m, 1H), 2.41 (d, *J = 7.2* Hz, 2H), 2.24 (s, 3H), 2.04 (s, 3H), 1.85 - 1.79 (m, 1H), 1.36 - 1.29 (m, 3H), 0.89 (d, *J* = 6.4 Hz, 6H).

### Example 18 Preparation of Compound 18

Replacing Compound 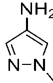 with Compound 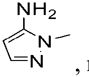 referring to the synthesis method of Compound 3, (S)-N-(5-isobutyl-3-(3-methyl-4-(pyridazin-3-ylmethyl)piperazin-1-yl)-2-(2H-tetrazol-5-yl)phenyl)-1-methyl-1H-pyrazol-5-amine (Compound **18,** 47.79 mg, yield 18.94%) was obtained. LCMS: (ESI, m/z): 488.4 [M+H]⁺.

¹H NMR (400 MHz, CD₃OD) δ 9.11 - 9.10 (m, 1H), 7.88 - 7.86 (m, 1H), 7.74 - 7.71 (m, 1H), 7.43 (d, *J* = 2.0 Hz, 1H), 6.63 (s, 1H), 6.36 (s, 1H), 6.04 (d, *J* = 2.0 Hz, 1H), 4.36 (d, *J* = 14.4 Hz, 1H), 3.83 (d, *J* = 14.4 Hz, 1H), 3.65 (s, 3H), 2.98 - 2.95 (m, 3H), 2.83 - 2.68 (m, 3H), 2.63 - 2.57 (m, 1H), 2.41 (d, *J =* 7.2 Hz, 2H), 1.86 - 1.79 (m, 1H), 1.11 (d, *J* = 6.0 Hz, 3H), 0.89 (d, *J* = 6.4 Hz, 6H).

### Example 19 Preparation of Compound 19

Replacing Compound 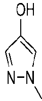 with Compound 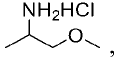 referring to the synthesis method of Compound 13, 5-isobutyl-N-(1-methoxypropan-2-yl)-3-((S)-3-methyl-4-(pyridazin-3-ylmethyl)piperazin-1-yl)-2-(2H-tetrazol-5-yl)aniline (Compound 19, 32.91 mg) was obtained. LCMS: (ESI, m/z): 480.5 [M+H]⁺.

¹H NMR (400 MHz, CD₃OD) δ 9.11 - 9.09 (m, 1H), 7.88 - 7.86 (m, 1H), 7.74 - 7.70 (m, 1H), 6.45 (d, *J* = 20.0 Hz, 2H), 4.34 (d, J = 14.4 Hz, 1H), 3.80 - 3.72 (m, 2H), 3.44 - 3.41 (m, 1H), 3.37 - 3.33 (m, 1H), 3.32 (s, 3H), 2.95 - 2.90 (m, 3H), 2.81 - 2.70 (m, 2H), 2.67 - 2.55 (m, 2H), 2.45 (d, *J* = 7.2 Hz, 2H), 1.96 - 1.85 (m, 1H), 1.20 (d, *J* = 6.4 Hz, 3H), 1.09 (d, *J* = 6.0 Hz, 3H), 0.94 (d, *J=* 6.4 Hz, 6H).

### Example 20 Preparation of Compound 20

Replacing Compound 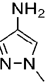 with Compound 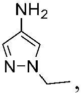 referring to the synthesis method of Compound **3,** (S)-1-ethyl-N-(5-isobutyl-3-(3-methyl-4-(pyridazin-3-ylmethyl)piperazin-1-yl)-2-(2H-tetrazol-5-yl)phenyl)-1H-pyrazol-4-amine (Compound **20,** 42.48 mg, yield 29.86%) was obtained. LCMS: (ESI, m/z): 502.4 [M+H]⁺.

¹H NMR (400 MHz, CD₃OD) δ 9.11 - 9.09 (m, 1H), 7.89 - 7.86 (m, 1H), 7.74 - 7.71 (m, 1H), 7.59 (s, 1H), 7.39 (s, 1H), 6.52 (d, *J* = 10.8 Hz, 2H), 4.35 (d, *J* = 14.4 Hz, 1H), 4.18 - 4.13 (m, 2H), 3.80 (d, *J =* 14.4 Hz, 1H), 2.97 - 2.95 (m, 3H), 2.82 - 2.68 (m, 3H), 2.63 - 2.58 (m, 1H), 2.38 (d, *J* = 7.2 Hz, 2H), 1.87 - 1.77 (m, 1H), 1.45 (t, *J* = 7.2 Hz, 3H), 1.11 (d, *J =* 6.0 Hz, 3H), 0.89 (d, *J* = 6.4 Hz, 6H).

### Example 21 Preparation of Compound 21

Replacing Compound 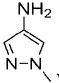 with Compound 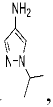 referring to the synthesis method of Compound **3,** (S)-N-(5-isobutyl-3-(3-methyl-4-(pyridazin-3-ylmethyl)piperazin-1-yl)-2-(2H-tetrazol-5-yl)phenyl)-1-isopropyl-1H-pyrazol-4-amine (Compound **21,** 37.96 mg, yield 29.02%) was obtained. LCMS: (ESI, m/z): 516.5 [M+H]⁺.

¹H NMR (400 MHz, CD₃OD) δ 9.11 - 9.09 (m, 1H), 7.89 - 7.86 (m, 1H), 7.74 - 7.71 (m, 1H), 7.61 (s, 1H), 7.39 (s, 1H), 6.52 (d, *J* = 9.6 Hz, 2H), 4.52 - 4.45 (m, 1H), 4.35 (d, *J* = 14.4 Hz, 1H), 3.81 (d, *J* = 14.4 Hz, 1H), 2.97 - 2.95 (m, 3H), 2.82 - 2.69 (m, 3H), 2.64 - 2.59 (m, 1H), 2.38 (d, *J* = 7.2 Hz, 2H), 1.85 - 1.79 (m, 1H), 1.49 (d, *J =* 6.8 Hz, 6H), 1.11 (d, *J* = 6.0 Hz, 3H), 0.89 (d, *J* = 6.4 Hz, 6H).

### Example 22 Preparation of Compound 22

Replacing Compound 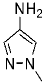 with Compound 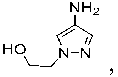 referring to the synthesis method of Compound 3, (S)-N-(5-isobutyl-3-(3-methyl-4-(pyridazin-3-ylmethyl)piperazin-1-yl)-2-(2H-tetrazol-5-yl)phenyl)-1-isopropyl-1H-pyrazol-4-amine (Compound 22, 78.21 mg, yield 38.99%) was obtained. LCMS: (ESI, m/z): 518.6 [M+H]⁺.

¹H NMR (400 MHz, CD₃OD) δ 9.13 - 9.11 (m, 1H), 7.88 - 7.86 (m, 1H), 7.76 - 7.72 (m, 1H), 7.61 (s, 1H), 7.41 (s, 1H), 6.60 (s, 1H), 6.52 (s, 1H), 4.43 (d, *J* = 14.4 Hz, 1H), 4.20 (t, *J =* 5.6 Hz, 2H), 3.92 - 3.87 (m, 3H), 3.01 - 2.98 (m, 3H), 2.91 - 2.88 (m, 2H), 2.78 - 2.67 (m, 2H), 2.39 (d, *J* = 7.2 Hz, 2H), 1.88 - 1.80 (m, 1H), 1.15 (d, *J* = 6.0 Hz, 3H), 0.90 (d, *J* = 6.4 Hz, 6H).

### Example 23 Preparation of Compound 23

Replacing Compound 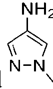 with Compound 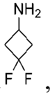 referring to the synthesis method of Compound **3,** (S)-N-(3,3-difluorocyclobutyl)-5-isobutyl-3-(3-methyl-4-(pyridazin-3-ylmethyl)piperazin-1-yl)-2-(2H-tetrazol-5-yl)aniline (Compound **23,** 54.54 mg, yield 50.80%) was obtained. LCMS: (ESI, m/z): 498.4 [M+H]⁺.

¹H NMR (400 MHz, CD₃OD) δ 9.10 - 9.09 (m, 1H), 7.88 - 7.86 (m, 1H), 7.74 - 7.70 (m, 1H), 6.51 (s, 1H), 6.28 (s, 1H), 4.33 (d, *J* = 14.4 Hz, 1H), 3.94 - 3.88 (m, 1H), 3.79 (d, *J* = 14.4 Hz, 1H), 3.10 - 3.00 (m, 2H), 2.95 - 2.92 (m, 3H), 2.80 - 2.67 (m, 3H), 2.61 - 2.55 (m, 1H), 2.50 - 2.37 (m, 4H), 1.95 - 1.85 (m, 1H), 1.10 (d, *J* = 6.0 Hz, 3H), 0.94 (d, *J* = 6.4 Hz, 6H).

### Example 24 Preparation of Compound 24

Replacing Compound 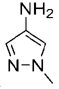 with Compound 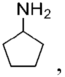 referring to the synthesis method of Compound **3,** (S)-N-cyclopentyl-5-isobutyl-3-(3-methyl-4-(pyridazin-3-ylmethyl)piperazin-1-yl)-2-(2H-tetrazol-5-yl)aniline (Compound **24,** 15.80 mg, yield 22.2%) was obtained. LCMS: (ESI, m/z): 476.3 [M+H]⁺.

¹H NMR (400 MHz, CD₃OD) δ 9.13 - 9.02 (m, 1H), 7.89 - 7.86 (m, 1H), 7.74 - 7.70 (m, 1H), 6.44 (d, *J* = 8.4 Hz, 2H), 4.32 (d, *J* = 14.4 Hz, 1H), 3.91 - 3.86 (m, 1H), 3.77 (d, *J* = 14.4 Hz, 1H), 2.97 - 2.94 (m, 3H), 2.80 - 2.69 (m, 3H), 2.62 - 2.56 (m, 1H), 2.46 (d, *J =* 7.2 Hz, 2H), 2.04 - 1.97 (m, 2H), 1.94 - 1.87 (m, 1H), 1.74 - 1.60 (m, 4H), 1.54 - 1.47 (m, 2H), 1.11 (d, *J=* 6.0 Hz, 3H), 0.94 (d, *J =* 6.4 Hz, 6H).

### Example 25 Preparation of Compound 25

Replacing Compound 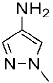 with Compound 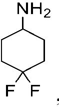 referring to the synthesis method of Compound **3,** (S)-N-(4,4-difluorocyclohexyl)-5-isobutyl-3-(3-methyl-4-(pyridazin-3-ylmethyl)piperazin-1-yl)-2-(2H-tetrazol-5-yl)aniline (Compound **25,** 38.70 mg, yield 34.77%) was obtained. LCMS: (ESI, m/z): 526.5 [M+H]⁺.

¹H NMR (400 MHz, CD₃OD) δ 9.10 - 9.09 (m, 1H), 7.89 - 7.86 (m, 1H), 7.74 - 7.70 (m, 1H), 6.48 (d, *J* = 15.6 Hz, 2H), 4.33 (d, *J =* 14.4 Hz, 1H), 3.78 (d, *J* = 14.4 Hz, 1H), 3.68 - 3.63 (m, 1H), 2.98 - 2.95 (m, 3H), 2.81 - 2.71 (m, 3H), 2.63 - 2.57 (m, 1H), 2.46 (d, *J* = 7.2 Hz, 2H), 2.04 - 1.88 (m, 7H), 1.66 - 1.59 (m, 2H), 1.11 (d, *J* = 6.0 Hz, 3H), 0.94 (d, *J =* 6.4 Hz, 6H).

### Example 26 Preparation of Compound 26

Replacing Compound 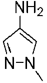 with Compound 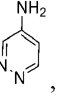 referring to the synthesis method of Compound **3,** (S)-N-(5-isobutyl-3-(3-methyl-4-(pyridazin-3-ylmethyl)piperazin-1-yl)-2-(2H-tetrazol-5-yl)phenyl)pyridazin-4-amine (Compound **26,** 52.95 mg, yield 30.16%) was obtained. LCMS: (ESI, m/z): 486.1 [M+H]⁺.

¹H NMR (400 MHz, CD₃OD) δ 9.10 - 9.08 (m, 1H), 8.52 (d, *J =* 6.8 Hz, 1H), 8.46 (d, *J* = 3.2 Hz, 1H), 8.19 (s, 0.26 HCOOH), 7.83 - 7.81 (m, 1H), 7.72 - 7.69 (m, 1H), 7.00 - 6.97 (m, 2H), 6.85 - 6.83 (m, 1H), 4.29 (d, *J* = 14.4 Hz, 1H), 3.81 (d, *J* = 14.4 Hz, 1H), 2.96 - 2.85 (m, 3H), 2.75 - 2.72 (m, 1H), 2.66 - 2.56 (m, 4H), 2.46 - 2.41 (m, 1H), 1.99 - 1.89 (m, 1H), 1.00-0.95 (m, 9H).

### Example 27 Preparation of Compound 27

Replacing Compound 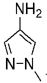 with Compound 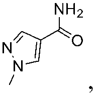 referring to the synthesis method of Compound **3,** (S)-N-(5-isobutyl-3-(3-methyl-4-(pyridazin-3-ylmethyl)piperazin-1-yl)-2-(2H-tetrazol-5-yl)phenyl)-1-methyl-1H-pyrazole-4-carboxamide (Compound 27, 33.21 mg, yield 35.81%) was obtained. LCMS: (ESI, m/z): 516.5 [M+H]⁺.

¹H NMR (400 MHz, CD₃OD) δ 9.12 (d, *J* = 5.2 Hz, 1H), 8.10 (s, 1H), 7.92 - 7.86 (m, 3H), 7.75 - 7.72 (m, 1H), 6.95 (s, 1H), 4.40 (d, *J* = 14.4 Hz, 1H), 3.94 - 3.86 (m, 4H), 2.99 - 2.97 (m, 3H), 2.88 - 2.83 (m, 2H), 2.78 - 2.73 (m, 1H), 2.68 - 2.63 (m, 1H), 2.56 (d, J = 7.2 Hz, 2H), 1.99 - 1.93 (m, 1H), 1.13 (d, *J =* 6.0 Hz, 3H), 0.97 (d, *J* = 6.4 Hz, 6H).

### Example 28 Preparation of Compound 28

Replacing Compound 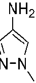 with Compound 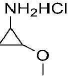 referring to the synthesis method of Compound **3,** the white solid 5-isobutyl-N-(2-methoxycyclopropyl)-3-((S)-3-methyl-4-(pyridazin-3-ylmethyl)piperazin-1-yl)-2-(2H-tetrazol-5-yl)aniline (Compound **28,** 1.13 mg, yield 1.58%) was obtained. LCMS: (ESI, m/z): 478.5 [M+H]⁺.

¹H NMR (400 MHz, CD3OD) δ 9.11 - 9.10 (m, 1H), 7.88 - 7.86 (m, 1H), 7.74 - 7.71 (m, 1H), 6.79 (d, J = 34.4 Hz, 1H), 6.50 (d, J = 8.8 Hz, 1H), 4.37 (d, J = 14.8 Hz, 1H), 3.82 (d, J = 14.4 Hz, 1H), 3.41 (s, 3H), 3.19 - 3.17 (m, 1H), 2.95 - 2.93 (m, 3H), 2.84 - 2.79 (m, 2H), 2.74 - 2.69 (m, 1H), 2.65 - 2.60 (m, 1H), 2.57 - 2.54 (m, 1H), 2.50 - 2.47 (m, 2H), 1.97 - 1.90 (m, 1H), 1.11 (d, J = 6.0 Hz, 3H), 1.08 - 1.02 (m, 1H), 0.95 (d, J = 6.4 Hz, 6H), 0.77 - 0.73 (m, 1H).

### Example 29 Preparation of Compound 29

Replacing Compound 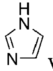 with Compound 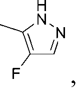 referring to the synthesis method of Compound 5, the white solid (S)-3-((4-(3-((4-fluoro-3-methyl-1H-pyrazol-1-yl)methyl)-5-isobutyl-2-(2H-tetrazol-5-yl)phenyl)-2-methylpiperazin-1-yl)methyl)pyridazine (Compound 29, 8.33 mg, yield 11.72%) was obtained. LCMS: (ESI, m/z): 505.8 [M+H]⁺.

¹H NMR (400 MHz, CD₃OD) δ 9.12 (d, *J =* 4.8 Hz, 1H), 7.82 (d, *J =* 8.4 Hz, 1H), 7.74 - 7.71 (m, 1H), 7.29 -7.12 (m, 1H), 7.03 - 7.01 (m, 1H), 6.82 - 6.42 (m, 1H), 5.05 - 5.00 (m, 2H), 4.42 - 4.37 (m, 1H), 3.97 - 3.88 (m, 1H), 2.90 - 2.81 (m, 4H), 2.71 - 2.64 (m, 2H), 2.53 - 2.46 (m, 3H), 2.09 (s, 2H), 1.97 (s, 1H), 1.91 - 1.78 (m, 1H), 1.04 - 1.02 (m, 3H), 0.92 - 0.87 (m, 6H).

### Example 30 Preparation of Compound 30

Replacing Compound 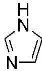 with Compound 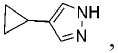 referring to the synthesis method of Compound **5,** the white solid (S)-3-((4-(3-((4-cyclopropyl-1H-pyrazol-1-yl)methyl)-5-isobutyl-2-(2H-tetrazol-5-yl)phenyl)-2-methylpiperazin-1-yl)methyl)pyridazine (Compound **30,** 17.57 mg, yield 32.07%) was obtained. LCMS: (ESI, m/z): 513.8 [M+H]⁺.

¹H NMR (400 MHz, CD₃OD) δ 9.14 (d, *J* = 4.8 Hz, 1H), 7.82 - 7.80 (m, 1H), 7.75 - 7.72 (m, 1H), 7.20 (s, 1H), 7.03 (s, 1H), 6.90 (s, 1H), 6.79 (s, 1H), 5.10 (s, 2H), 4.43 (d, *J* = 15.2 Hz, 1H), 3.98 (d, *J* = 11.6 Hz, 1H), 2.91 - 2.87 (m, 4H), 2.76 - 2.67 (m, 2H), 2.57 - 2.50 (m, 3H), 1.90 - 1.84 (m, 1H), 1.64 - 1.58 (m, 1H), 1.05 (d, *J =* 6.0 Hz, 3H), 0.90 (d, *J =* 6.8 Hz, 6H), 0.81 - 0.76 (m, 2H), 0.45 - 0.41 (m, 2H).

### Example 31 Preparation of Compound 31

Replacing Compound 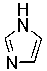 with Compound 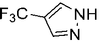 , referring to the synthesis method of Compound **5,** the white solid (S)-3-((4-(5-isobutyl-2-(2H-tetrazol-5-yl)-3-((4-(trifluoromethyl)-1H-pyrazol-1-yl)methyl)phenyl)-2-methylpiperazin-1-yl)methyl)pyridazine (Compound **31,** 33.97 mg, yield 51.99%) was obtained. LCMS: (ESI, m/z): 541.5 [M+H]⁺.

¹H NMR (400 MHz, CD₃OD) δ 9.10 (d, *J =* 3.2 Hz, 1H), 7.83 - 7.80 (m, 1H), 7.73 - 7.70 (m, 1H), 7.65 (d, *J =* 3.6 Hz, 2H), 7.01 (s, 1H), 6.85 (s, 1H), 5.19 (s, 2H), 4.32 (d, *J =* 14.0 Hz, 1H), 3.85 (d, *J =* 16.4 Hz, 1H), 2.90 - 2.86 (m, 3H), 2.78 - 2.74 (m, 1H), 2.66 - 2.62 (m, 2H), 2.52 - 2.41 (m, 3H), 1.90 - 1.83 (m, 1H), 1.00 (d, *J* = 5.6 Hz, 3H), 0.90 (d, *J* = 6.8 Hz, 6H).

### Example 32 Preparation of Compound 32

Replacing Compound 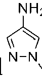 with Compound 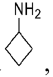 referring to the synthesis method of Compound **3,** the white solid (S)-N-cyclobutyl-5-isobutyl-3-(3-methyl-4-(pyridazin-3-ylmethyl)piperazin-1-yl)-2-(2H-tetrazol-5-yl)aniline (Compound **32,** 28.66 mg, yield 12.10%) was obtained. LCMS: (ESI, m/z): 462.7 [M+H]⁺.

¹H NMR (400 MHz, CD₃OD) δ 9.11 (d, *J* = 4.8 Hz, 1H), 7.89 - 7.86 (m, 1H), 7.74 - 7.71 (m, 1H), 6.44 (s, 1H), 6.31 (s, 1H), 4.40 - 4.35 (m, 1H), 4.01 - 3.94 (m, 1H), 3.87 - 3.80 (m, 1H), 2.97 - 2.95 (m, 3H), 2.86 - 2.81 (m, 2H), 2.76 - 2.71 (m, 1H), 2.67 - 2.62 (m, 1H), 2.47 - 2.41 (m, 4H), 1.93 - 1.82 (m, 5H), 1.13 (d, *J =* 6.0 Hz, 3H), 0.93 (d, *J =* 6.4 Hz, 6H).

### Example 33 Preparation of Compound 33

Replacing Compound 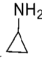 with Compound 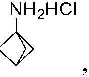 referring to the synthesis method of Compound **4,** the white solid (S)-N-(5-isobutyl-3-(3-methyl-4-(pyridazin-3-ylmethyl)piperazin-1-yl)-2-(2H-tetrazol-5-yl)phenyl)bicyclo[1.1.1]pentan-1-amine (Compound **33,** 7.60 mg, yield 6.95%) was obtained. LCMS: (ESI, m/z): 474.5 [M+H]⁺.

¹H NMR (400 MHz, CD₃OD) δ 9.27 (s, 1H), 7.83 (d, *J* = 3.2 Hz, 2H), 6.80 (s, 1H), 6.49 (s, 1H), 5.00 - 4.91 (m, 1H), 4.62 - 4.53 (m, 1H), 3.60 - 3.53 (m, 1H), 3.50 - 3.45 (m, 1H), 3.15 - 3.06 (m, 3H), 2.98 - 2.93 (m, 1H), 2.50 (s, 1H), 2.48 (s, 2H), 2.10 (s, 6H), 1.97 - 1.88 (m, 1H), 1.39 - 1.29 (m, 4H), 0.96 (d, *J* = 6.8 Hz, 6H).

### Example 34 Preparation of Compound 34

Referring to the preparation method of Compound 3, using 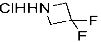 to replace 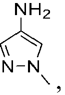 Compound 34 (26.47 mg, yield 21.92%) was obtained by column chromatography after a 2-step reaction.

LCMS: (ESI, m/z): 484.4 [M+H]⁺.

¹H NMR (400 MHz, DMSO-d₆) δ 9.11 - 9.10 (m, 1H), 7.64 (d, *J* = 3.2 Hz, 2H), 6.53 (s, 1H), 6.24 (s, 1H), 4.01 (d, *J =* 14.0 Hz, 1H), 3.71 - 3.65 (m, 4H), 3.56 (d, *J =* 14.0 Hz, 1H), 2.78 - 2.71 (m, 2H), 2.66 - 2.62 (m, 1H), 2.43 (d, *J* = 6.8 Hz, 4H), 2.24 (s, 1H), 2.01 - 1.96 (m, 1H), 1.88 - 1.85 (m, 1H), 0.90 (d, *J* = 6.8 Hz, 6H), 0.83 (d, *J* = 6.4 Hz, 3H).

### Example 35 Preparation of Compound 35

Referring to the preparation method of Compound 3, using 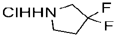 to replace 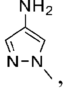 Compound **35** (48.68 mg, yield 37.10%) was obtained by column chromatography after a 2-step reaction.

LCMS: (ESI, m/z): 498.3 [M+H]⁺.

¹H NMR (400 MHz, DMSO-d₆) δ 16.16 (s, 1H), 9.10 (s, 1H), 7.65 (d, *J* = 2.8 Hz, 2H), 6.62 (s, 1H), 6.55 (s, 1H), 4.02 (d, *J* = 14.4 Hz, 1H), 3.56 (d, *J* = 14.0 Hz, 1H), 3.10 - 2.98 (m, 4H), 2.80 (d, *J* = 10.0 Hz, 1H), 2.72 - 2.67 (m, 2H), 2.45 (d, *J* = 6.8 Hz, 4H), 2.33 - 2.21 (m, 3H), 1.95 - 1.85 (m, 2H), 0.90 (d, *J =* 6.4 Hz, 6H), 0.85 (d, *J =* 5.6 Hz, 3H).

### Example 36 Preparation of Compound 36

Referring to the preparation method of Compound **2,** using 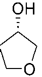 to replace 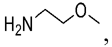 Compound **36** (50.30 mg, yield 32.68%) was obtained by column chromatography after a 3-step reaction. LCMS: (ESI, m/z): 479.4 [M+H]⁺.

¹H NMR (400 MHz, CD₃OD) δ 9.08 - 9.07 (m, 1H), 7.82 (d, *J* = 8.4 Hz, 1H), 7.71 - 7.68 (m, 1H), 6.65 (d, *J* = 8.4 Hz, 2H), 5.01 (s, 1H), 4.23 (d, *J* = 14.0 Hz, 1H), 3.90 - 3.87 (m, 1H), 3.78 - 3.70 (m, 3H), 3.68 - 3.62 (m, 1H), 2.88 - 2.80 (m, 3H), 2.69 - 2.61 (m, 2H), 2.53 (d, *J =* 7.2 Hz, 3H), 2.34 - 2.30 (m, 1H), 2.17 - 2.08 (m, 1H), 1.94 - 1.89 (m, 2H), 0.99 - 0.93 (m, 9H).

### Example 37 Preparation of Compound 37

Referring to the preparation method of Compound **2,** using 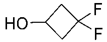 to replace 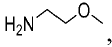 Compound **37** (22.74 mg, yield 56%) was obtained by column chromatography after a 3-step reaction. LCMS: (ESI, m/z): 499.4 [M+H]⁺.

¹H NMR (400 MHz, MeOD) δ 9.08 (dd, *J* = 4.9, 1.6 Hz, 1H), 7.82 (dd, *J* = 8.5, 1.6 Hz, 1H), 7.70 (dd, *J* = 8.5, 4.9 Hz, 1H), 6.66 (s, 1H), 6.51 (s, 1H), 4.69 (s, 1H), 4.21 (d, *J* = 14.4 Hz, 1H), 3.73 (d, *J* = 14.3 Hz, 1H), 3.02 (td, *J* = 13.0, 6.8 Hz, 2H), 2.83 (dd, *J* = 27.0, 9.8 Hz, 3H), 2.65 (d, *J* = 11.7 Hz, 1H), 2.61 - 2.43 (m, 6H), 2.31 (s, 1H), 1.91 (dt, *J =* 13.6, 6.9 Hz, 1H), 0.97 (d, *J* = 6.2 Hz, 3H), 0.93 (d, *J* = 6.6 Hz, 6H).

### Example 38 Preparation of Compound 104

Referring to the preparation method of Compound **3,** using 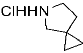 to replace 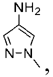 Compound **104** (142.88 mg, yield 31.79%) was obtained by column chromatography after a 2-step reaction.

LCMS: (ESI, m/z): 488.4 [M+H]⁺.

¹H NMR (400 MHz, CD₃OD) δ 9.10 (d, *J* = 4.4 Hz, 1H), 7.82 (d, *J* = 8.4 Hz, 1H), 7.73 - 7.70 (m, 1H), 6.49 (d, *J =* 10.0 Hz, 2H), 4.27 (d, *J =* 14.4 Hz, 1H), 3.78 (d, *J* = 14.4 Hz, 1H), 3.03 - 2.99 (m, 2H), 2.96 - 2.93 (m, 1H), 2.86 - 2.84 (m, 2H), 2.70 - 2.65 (m, 3H), 2.62 - 2.57 (m, 1H), 2.47 - 2.45 (m, 3H), 2.27 (m, 1H), 1.93 - 1.86 (m, 1H), 1.76 - 1.64 (m, 2H), 1.01 (d, *J =* 6.0 Hz, 3H), 0.93 (d, *J =* 8.4 Hz, 6H), 0.51 - 0.43 (m, 4H).

### Example 39 Preparation of Compound 106

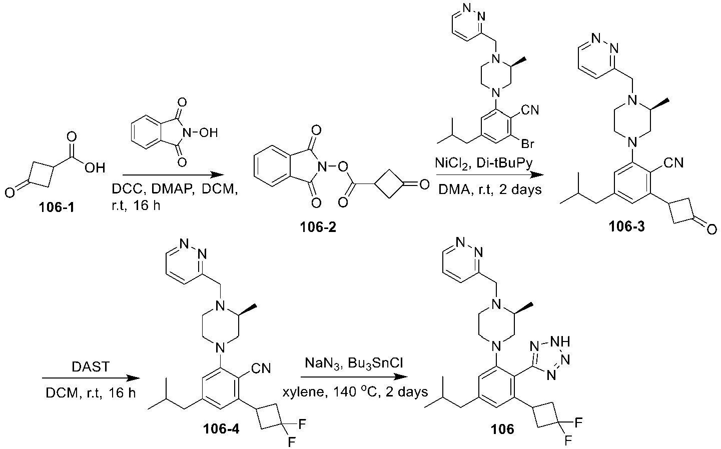

### Step 1: Synthesis of Compound 106-2

At room temperature, 2-hydroxyisoindoline-1,3-dione (2.86 g, 17.53 mmol, 1.0 equiv), 4-dimethylaminopyridine (2.14 g, 17.53 mmol, 1.0 equiv), and N,N'-dicyclohexylcarbodiimide (5.43 g, 26.29 mmol, 1.5 equiv) were added to a solution of 3-oxocyclobutane-1-carboxylic acid (2 g, 17.53 mmol, 1.0 equiv) in dichloromethane (30 mL). The reaction mixture was stirred at room temperature for 16 hours. After the reaction was completed, the reaction mixture was quenched by adding ice water (50 mL) and extracted with ethyl acetate (50 mL x 3). The combined organic layers were washed with saturated brine (50 mL), dried over anhydrous sodium sulfate and concentrated. The resulting residue was purified by silica gel column chromatography with ethyl acetate/petroleum ether (0 - 50%) to give Compound 106-2 as a yellow solid (3.6 g, yield 79.23%).

¹H NMR (400 MHz, CDCl₃) δ 7.92 - 7.90 (m, 2H), 7.83 - 7.81 (m, 2H), 3.69 - 3.50 (m, 5H).

### Step 2: Synthesis of Compound 106-3

(S)-2-bromo-4-isobutyl-6-(3-methyl-4-(pyridazin-3-ylmethyl)piperazin-1-yl)benzonitrile (250 mg, 0.578 mmol, 1.0 equiv), 6,6'-di-tert-butyl-2,2'-bipyridine (31 mg, 0.115 mmol, 0.2 equiv), and nickel chloride (15 mg, 0.115 mmol, 0.2 equiv) were added to a solution of Compound 106-2 (150 mg, 0.578 mmol, 1.0 equiv) in N,N-dimethylacetamide (2 mL) in a microwave tube. The reaction mixture was stirred at room temperature for 2 days. After the reaction was completed, the reaction mixture was quenched by adding ice water (10 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound 106-3 as a yellow solid (19 mg, yield 7.86%).

LCMS: (ESI, m/z): 418.3 [M+H]⁺.

### Step 3: Synthesis of Compound 106-4

At room temperature, diethylaminosulfur trifluoride (14.6 mg, 0.091 mmol, 2.0 equiv) was added to a solution of Compound **106-3** (19.0 mg, 0.045 mmol, 1.0 equiv) in dichloromethane (2 mL). The reaction mixture was stirred at room temperature for 16 hours. After the reaction was completed, the reaction mixture was quenched by adding ice water (5 mL) and extracted with ethyl acetate (5 mL x 3). The combined organic layers were washed with saturated brine (5 mL), dried over anhydrous sodium sulfate and concentrated. The resulting residue was purified by silica gel column chromatography with methanol/dichloromethane (0 - 10%) to give Compound **106-4** as a yellow solid (9 mg, yield 45.0%).

LCMS: (ESI, m/z): 440.5 [M+H]⁺.

### Step 4: Synthesis of Compound 106

Referring to the preparation method of tetrazole in Compound 2, Compound 106 (1.54 mg, yield 15.59%) was obtained.

LCMS: (ESI, m/z): 483.3 [M+H]⁺.

¹H NMR (400 MHz, CD₃OD) δ 9.09 (s, 1H), 8.36 (s, 0.65HCOOH), 7.82 (d, *J* = 7.6 Hz, 1H), 7.72 - 7.69 (m, 1H), 6.94 (d, *J* = 16.8 Hz, 2H), 4.26 (d, *J* = 14.4 Hz, 1H), 3.80 (d, *J* = 15.2 Hz, 1H), 3.20 - 3.13 (m, 1H), 2.92 - 2.81 (m, 3H), 2.73 - 2.69 (m, 1H), 2.65 - 2.62 (m, 1H), 2.56 - 2.52 (m, 3H), 2.50 - 2.41 (m, 4H), 2.38 - 2.32 (m, 1H), 1.94 - 1.88 (m, 1H), 0.97 (d, *J* = 6.0 Hz, 3H), 0.94 (d, *J* = 6.4 Hz, 6H).

### Example 40 Preparation of Compound 121

Referring to the preparation method of Compound **3,** using 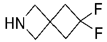 to replace 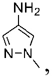 Compound **121** (18.33 mg, yield 21%) was obtained by column chromatography after a 2-step reaction.

LCMS: (ESI, m/z): 524.4 [M+H]⁺.

¹H NMR (400 MHz, CD₃OD) δ 9.10 (d, *J* = 4.8 Hz, 1H), 7.81 (d, *J* = 8.8 Hz, 1H), 7.73 - 7.69 (m, 1H), 6.48 (s, 1H), 6.17 (s, 1H), 4.28 (d, *J* = 14.4 Hz, 1H), 3.81 (d, *J* = 14.0 Hz, 1H), 3.42 (d, *J* = 4.0 Hz, 4H), 2.91 - 2.85 (m, 3H), 2.72 - 2.69 (m, 1H), 2.65 - 2.59 (m, 5H), 2.52 - 2.44 (m, 3H), 2.33 (s, 1H), 1.90 - 1.85 (m, 1H), 0.99 (d, *J* = 6.4 Hz, 3H), 0.92 (d, *J* = 6.8 Hz, 6H).

### Example 41 Preparation of Compound 129

Referring to the preparation method of Compound **3,** using 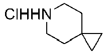 to replace 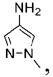 Compound **129** (45.14 mg, yield 48.4%) was obtained by column chromatography after a 2-step reaction.

LCMS: (ESI, m/z): 502.3 [M+H]⁺.

¹H NMR (400 MHz, CD₃OD) δ 9.07 (d, *J* = 4.8 Hz, 1H), 7.83 (d, *J* = 8.4 Hz, 1H), 7.72 - 7.68 (m, 1H), 6.77 (d, *J* = 17.6 Hz, 2H), 4.20 (d, *J* = 14.0 Hz, 1H), 3.70 (d, *J* = 14.4 Hz, 1H), 2.97 - 2.93 (m, 1H), 2.91 - 2.88 (m, 1H), 2.86 - 2.83 (m, 5H), 2.65 - 2.59 (m, 2H), 2.51 (d, *J* = 7.2 Hz, 2H), 2.44 (s, 1H), 2.25 - 2.22 (m, 1H), 1.93 - 1.86 (m, 1H), 1.17 (s, 4H), 0.98 (d, *J =* 6.4 Hz, 3H), 0.93 (d, *J =* 6.4 Hz, 6H), 0.24 (s, 4H).

### Example 42 Preparation of Compound 131

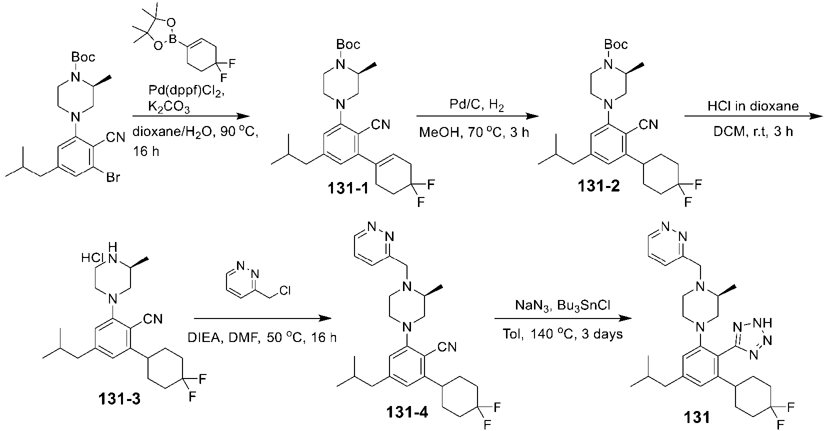

### Step 1: Synthesis of Compound 131-1

2-(4,4-Difluorocyclohex-1-en-1-yl)-4,4,5,5-tetramethyl-1,3,2-dioxaborolane (101.0 mg, 0.412 mmol, 1.2 equiv), [1,1'-bis(diphenylphosphino)ferrocene]dichloropalladium(II) (25 mg, 0.0346 mmol, 0.1 equiv), and potassium carbonate (143 mg, 1.0 mmol, 3.0 equiv) were added to a solution of tert-butyl (S)-4-(3-bromo-2-cyano-5-isobutylphenyl)-2-methylpiperazine-1-carboxylate (150.0 mg, 0.343 mmol, 1.0 equiv) in 1,4-dioxane (5 mL) and water (1 mL) in a microwave tube. The reaction mixture was heated to 90 °C under a nitrogen atmosphere and stirred for 16 hours After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with ethyl acetate/petroleum ether (0 - 50%) to give Compound **131-1** as a yellow solid (123.0 mg, yield 75.56%).

LCMS: (ESI, m/z): 474.2 [M+H]⁺.

### Step 2: Synthesis of Compound 131-2

At room temperature, Pd/C (20 mg) was added to a solution of Compound **131-1** (123.0 mg, 0.26 mmol, 1.0 equiv) in methanol (3 mL), and the atmosphere was replaced with hydrogen. The reaction mixture was heated to 70 °C and stirred for 3 hours. After the reaction was completed, the reaction mixture was filtered, and the filtrate was concentrated. The resulting residue was purified by silica gel column chromatography with ethyl acetate/petroleum ether (0 - 50%) to give Compound **131-2** as a yellow solid (100.0 mg, yield 80.96%).

LCMS: (ESI, m/z): 476.1 [M+H]⁺.

### Step 3: Synthesis of Compound 131-3

At room temperature, a solution of hydrogen chloride in 1,4-dioxane (2 ml) was added to a solution of Compound **131-2** (100.0 mg, 0.21 mmol, 1.0 equiv) in dichloromethane (5 mL). The reaction mixture was stirred at room temperature for 3 hours. After the reaction was completed, the reaction mixture was concentrated to give Compound **131-3** hydrochloride as a yellow solid (110.0 mg, crude).

LCMS: (ESI, m/z): 376.4 [M+H]⁺.

### Step 4: Synthesis of Compound 131-4

At room temperature, N,N-diisopropylethylamine (104.0 mg, 0.8 mmol, 3.0 equiv) and 3-(chloromethyl)pyridazine (41 mg, 0.32 mmol, 1.2 equiv) were added to a solution of Compound **131-3** hydrochloride (110.0 mg, 0.26 mmol, 1.0 equiv) in N,N-dimethylformamide (3 mL). The reaction mixture was heated to 50 °C and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **131-4** as a yellow solid (76.0 mg, yield 60.87%).

LCMS: (ESI, m/z): 468.6 [M+H]⁺.

### Step 5: Synthesis of Compound 131

Referring to the preparation method of tetrazole in Compound **2,** Compound **131** (34.88 mg, yield 42.03%) was obtained.

LCMS: (ESI, m/z): 511.3 [M+H]⁺.

¹H NMR (400 MHz, CD₃OD) δ 9.11 (d, *J* = 3.6 Hz, 1H), 8.18 (s, 0.13 HCOOH), 7.81 (d, *J* = 7.2 Hz, 1H), 7.74 - 7.70 (m, 1H), 6.94 (d, *J* = 17.6 Hz, 2H), 4.32 (d, *J* = 14.4 Hz, 1H), 3.86 (d, *J* = 14.4 Hz, 1H), 2.93 - 2.84 (m, 3H), 2.78 - 2.74 (m, 1H), 2.68 - 2.63 (m, 1H), 2.60 - 2.52 (m, 3H), 2.41 - 2.36 (m, 1H), 2.28 - 2.21 (m, 1H), 2.06 - 1.99 (m, 2H), 1.92 - 1.85 (m, 1H), 1.80 - 1.71 (m, 4H), 1.68 - 1.52 (m, 2H), 1.00 (d, *J* = 6.0 Hz, 3H), 0.93 (d, *J* = 6.4 Hz, 6H).

### Example 43 Preparation of Compound 132

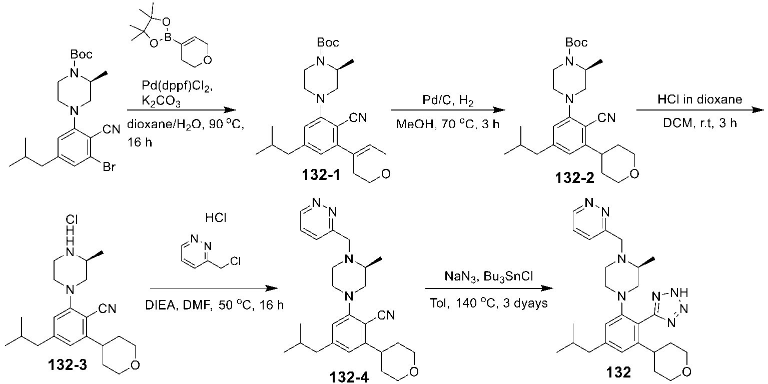

### Step 1: Synthesis of Compound 132-1

Under a nitrogen atmosphere, 2-(3,6-dihydro-2H-pyran-4-yl)-4,4,5,5-tetramethyl-1,3,2-dioxaborolane (145.0 mg, 0.690 mmol, 1.5 equiv), [1,1'-bis(diphenylphosphino)ferrocene]dichloropalladium(II) (38 mg, 0.046 mmol, 0.1 equiv), and cesium carbonate (450 mg, 1.379 mmol, 3.0 equiv) were added to a solution of tert-butyl (S)-4-(3-bromo-2-cyano-5-isobutylphenyl)-2-methylpiperazine-1-carboxylate (200.0 mg, 0.460 mmol, 1.0 equiv) in 1,4-dioxane/water (5.0/1.0 mL) in a microwave tube. The reaction mixture was heated to 90 °C and reacted for 16 hours. The desired product was detected by LCMS, the reaction mixture was cooled to room temperature and filtered, and the filtrate was extracted with ethyl acetate (100 mL). The organic layer was washed with water (2 x 50 mL) and brine (50 mL), dried over anhydrous Na₂SO₄, and concentrated under reduced pressure to give a residue. The resulting residue was purified by silica gel column chromatography with ethyl acetate/petroleum ether (100%) to give Compound **132-1** as a yellow oil (190 mg, 94.09%).

LCMS: (ESI, m/z): 440.1 [M+H]⁺.

### Step 2: Synthesis of Compound 132-2

Compound **132-1** (100.0 mg, 0.228 mmol, 1.0 equiv) was dissolved in methanol (10 mL), and Pd/C (20.0 mg, 10%) was added. The atmosphere was replaced with hydrogen, and the reaction was carried out at 70 °C for 3 hours. Then, the mixture was filtered through Celite, and the filtrate was concentrated under reduced pressure to give Compound **132-2** as a colorless oil (85 mg, 84.92%).

LCMS: (ESI, m/z): 442.4 [M+H]⁺.

### Step 3: Synthesis of Compound 132-3

Under a nitrogen atmosphere, a solution of hydrogen chloride in dioxane (4 mL, 4 mol/L) was added to a solution of Compound **132-2** (85.0 mg, 0.193 mmol, 1.0 equiv) in dichloromethane (4 mL) at room temperature, and the reaction was carried out at room temperature for 3 hours. The desired product was detected by LCMS. Concentration under reduced pressure gave Compound **132-3** hydrochloride as a white solid (92 mg, crude).

LCMS: (ESI, m/z): 342.1 [M+H]⁺.

### Step 4: Synthesis of Compound 132-4

Under a nitrogen atmosphere, 3-(chloromethyl)pyridazine hydrochloride (80.0 mg, 0.488 mmol, 2.0 equiv) and N,N-diisopropylethylamine (157.0 mg, 1.220 mmol, 5.0 equiv) were added to a solution of Compound **132-3** hydrochloride (92.0 mg, 0.244 mmol, 1.0 equiv) in N,N-dimethylformamide (5 mL). The reaction was carried out at room temperature for 16 hours. The desired product was detected by LCMS, and the reaction mixture was poured into water (20 mL). The mixture was extracted with ethyl acetate (3 x 20 mL). The combined organic phases were washed with saturated brine (30 mL), dried over anhydrous sodium sulfate, and concentrated to dryness. The resulting residue was directly purified by column chromatography with methanol/dichloromethane (10%) to give Compound **132-4** as a yellow solid (45 mg, 40.59%).

LCMS: (ESI, m/z): 434.4 [M+H]⁺.

### Step 5: Synthesis of Compound 132

Referring to the preparation method of tetrazole in Compound **2,** Compound **132** (11.83 mg, 23.90%) was obtained.

LCMS: (ESI, m/z): 477.3 [M+H]⁺.

¹H NMR (400 MHz, CD₃OD) δ 9.10 (d, *J =* 3.6 Hz, 1H), 7.82 (d, *J* = 7.2 Hz, 1H), 7.73 - 7.70 (m, 1H), 7.00 (s, 1H), 6.92 (s, 1H), 4.30 (d, *J =* 14.4 Hz, 1H), 3.93 - 3.82 (m, 3H), 3.31 - 3.22 (m, 2H), 2.93 - 2.84 (m, 3H), 2.76 - 2.71 (m, 1H), 2.67 - 2.62 (m, 1H), 2.54 (d, *J* = 7.2 Hz, 3H), 2.42 - 2.36 (m, 2H), 1.93 - 1.86 (m, 1H), 1.81 - 1.71 (m, 2H), 1.62 - 1.56 (m, 2H), 0.99 (d, *J* = 6.0 Hz, 3H), 0.93 (d, *J* = 6.4 Hz, 6H).

### Example 44 Preparation of Compound 133

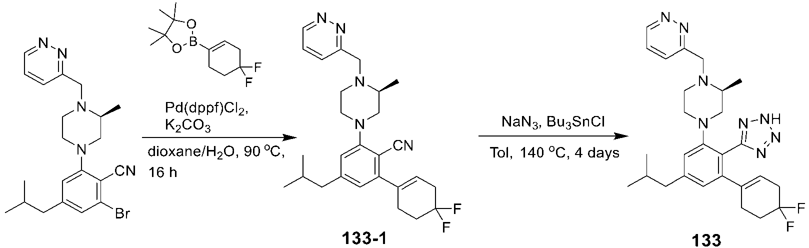

### Step 1: Synthesis of Compound 133-1

2-(4,4-Difluorocyclohex-1-en-1-yl)-4,4,5,5-tetramethyl-1,3,2-dioxaborolane (114.0 mg, 0.466 mmol, 1.0 equiv), [1,1'-bis(diphenylphosphino)ferrocene]dichloropalladium(II) (35 mg, 0.046 mmol, 0.1 equiv), and potassium carbonate (194 mg, 1.4 mmol, 3.0 equiv) were added to a solution of Compound 3-1 (200.0 mg, 0.466 mmol, 1.0 equiv) in 1,4-dioxane (5 mL) and water (1 mL) in a microwave tube. The reaction mixture was heated to 90 °C under a nitrogen atmosphere and stirred for 16 hours After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **133-1** as a yellow solid (220.0 mg, crude).

LCMS: (ESI, m/z): 466.3 [M+H]⁺.

### Step 2: Synthesis of Compound 133

Referring to the preparation method of tetrazole in Compound **2,** Compound **133** (51.63 mg, yield 21.48%) was obtained.

LCMS: (ESI, m/z): 509.2 [M+H]⁺.

¹H NMR (400 MHz, CD₃OD) δ 9.10 (d, *J =* 5.2 Hz, 1H), 8.14 (s, 0.16 HCOOH), 7.82 (d, *J* = 8.4 Hz, 1H), 7.73 - 7.70 (m, 1H), 6.99 (s, 1H), 6.87 (s, 1H), 5.25 (s, 1H), 4.29 (d, *J* = 14.4 Hz, 1H), 3.82 (d, *J* = 14.4 Hz, 1H), 2.93 - 2.87 (m, 3H), 2.75 - 2.71 (m, 1H), 2.68 - 2.61 (m, 1H), 2.59 - 2.50 (m, 3H), 2.46 - 2.31 (m, 3H), 2.26 - 2.22 (m, 2H), 1.94 - 1.86 (m, 3H), 1.00 (d, *J* = 6.0 Hz, 3H), 0.93 (d, *J* = 6.4 Hz, 6H).

### Example 45 Preparation of Compound 135

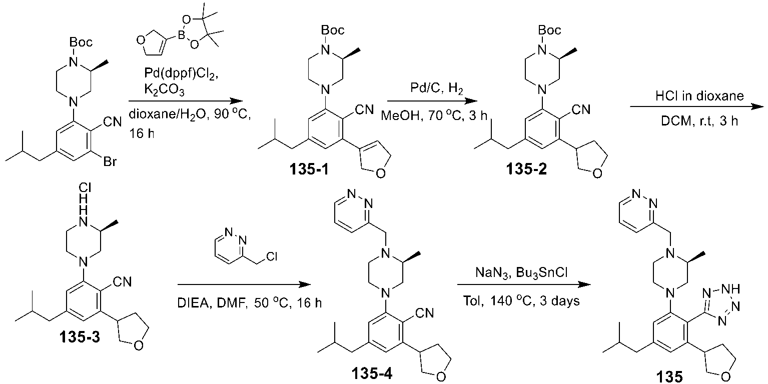

### Step 1: Synthesis of Compound 135-1

2-(2,5-Dihydrofuran-3-yl)-4,4,5,5-tetramethyl-1,3,2-dioxaborolane (81.0 mg, 0.412 mmol, 1.2 equiv), [1,1'-bis(diphenylphosphino)ferrocene]dichloropalladium(II) (25 mg, 0.0346 mmol, 0.1 equiv), and potassium carbonate (143 mg, 1.0 mmol, 3.0 equiv) were added to a solution of tert-butyl (S)-4-(3-bromo-2-cyano-5-isobutylphenyl)-2-methylpiperazine-1-carboxylate (150.0 mg, 0.343 mmol, 1.0 equiv) in 1,4-dioxane (5 mL) and water (1 mL) in a microwave tube. The reaction mixture was heated to 90 °C under a nitrogen atmosphere and stirred for 16 hours After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with ethyl acetate/petroleum ether (0 - 50%) to give Compound **135-1** (105.0 mg, yield 71.78%).

LCMS: (ESI, m/z): 426.2 [M+H]⁺.

### Step 2: Synthesis of Compound 135-2

At room temperature, Pd/C (20 mg) was added to a solution of Compound **135-1** (105.0 mg, 0.246 mmol, 1.0 equiv) in methanol (3 mL), and the atmosphere was replaced with hydrogen. The reaction mixture was heated to 70 °C and stirred for 3 hours. After the reaction was completed, the reaction mixture was filtered, and the filtrate was concentrated. The resulting residue was purified by silica gel column chromatography with ethyl acetate/petroleum ether (0 - 50%) to give Compound **135-2** (92.0 mg, yield 87.21%).

LCMS: (ESI, m/z): 428.3 [M+H]⁺.

### Step 3: Synthesis of Compound 135-3

At room temperature, a solution of hydrogen chloride in 1,4-dioxane (2 ml) was added to a solution of Compound **135-2** (92.0 mg, 0.21 mmol, 1.0 equiv) in dichloromethane (5 mL). The reaction mixture was stirred at room temperature for 3 hours. After the reaction was completed, the reaction mixture was concentrated to give Compound **135-3** (105.0 mg, crude).

LCMS: (ESI, m/z): 328.3 [M+H]⁺.

### Step 4: Synthesis of Compound 135-4

At room temperature, N,N-diisopropylethylamine (112.0 mg, 0.86 mmol, 3.0 equiv) and 3-(chloromethyl)pyridazine (37 mg, 0.288 mmol, 1.0 equiv) were added to a solution of Compound **135-3** (105.0 mg, 0.288 mmol, 1.0 equiv) in N,N-dimethylformamide (3 mL). The reaction mixture was heated to 50 °C and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **135-4** (62.0 mg, yield 51.22%).

LCMS: (ESI, m/z): 420.3 [M+H]⁺.

### Step 5: Synthesis of Compound 135

Referring to the preparation method of tetrazole in Compound **2,** Compound **135** (20.02 mg, yield 29.29%) was obtained.

LCMS: (ESI, m/z): 463.3 [M+H]⁺.

¹H NMR (400 MHz, CD₃OD) δ 9.11 (d, *J* = 3.6 Hz, 1H), 8.13 (s, 0.29 HCOOH), 7.81 (d, *J* = 8.4 Hz, 1H), 7.74 - 7.71 (m, 1H), 7.06 (s, 1H), 6.94 (s, 1H), 4.34 (d, *J* = 14.4 Hz, 1H), 4.00 - 3.96 (m, 1H), 3.91 - 3.81 (m, 2H), 3.76 - 3.70 (m, 1H), 3.68 - 3.65 (m, 1H), 3.04 - 2.97 (m, 1H), 2.94 - 2.85 (m, 3H), 2.80 - 2.76 (m, 1H), 2.69 - 2.59 (m, 2H), 2.54 (d, *J =* 7.2 Hz, 2H), 2.45 - 2.40 (m, 1H), 2.21 - 2.11 (m, 1H), 2.00 - 1.87 (m, 2H), 1.01 (d, *J =* 6.0 Hz, 3H), 0.93 (d, *J* = 6.4 Hz, 6H).

### Example 46 Preparation of Compound 136

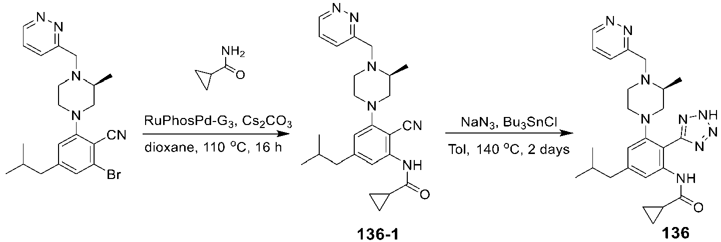

Referring to the preparation method of Compound **137,** using 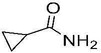 to replace 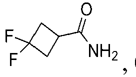 Compound **136** (9.15 mg, yield 7.93%) was obtained by column chromatography after a 2-step reaction.

LCMS: (ESI, m/z): 476.1 [M+H]⁺.

¹H NMR (400 MHz, CD₃OD) δ 9.11 -9.09 (m, 1H), 7.86 - 7.83 (m, 1H), 7.73 - 7.70 (m, 1H), 7.58 (s, 1H), 6.88 (s, 1H), 4.33 (d, *J =* 14.4 Hz, 1H), 3.81 (d, *J =* 14.8 Hz, 1H), 2.91 - 2.86 (m, 3H), 2.77 (d, *J =* 11.6 Hz, 1H), 2.66 - 2.61 (m, 2H), 2.53 - 2.46 (m, 3H), 1.95 - 1.88 (m, 1H), 1.62 - 1.56 (m, 1H), 1.05 (d, *J =* 6.0 Hz, 3H), 0.94 (d, *J =* 6.4 Hz, 6H), 0.84 - 0.78 (m, 4H).

### Example 47 Preparation of Compound 137

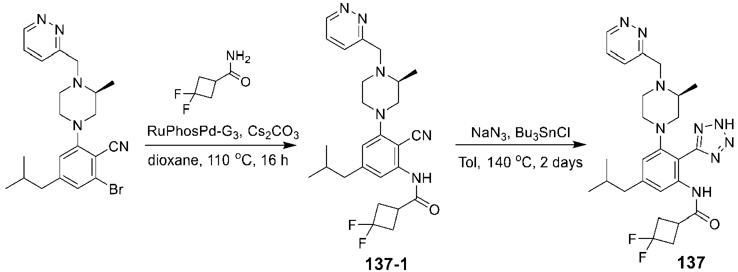

### Step 1: Synthesis of Compound 137-1

3,3-difluorocyclobutane-1-carboxamide (47.0 mg, 0.351 mmol, 1.5 equiv), (2-dicyclohexylphosphino-2',6'-diisopropoxy-1,1'-biphenyl)(2-amino-1,1'-biphenyl-2-yl)palladium(II) methanesulfonate (20 mg, 0.023 mmol, 0.1 equiv), and cesium carbonate (229 mg, 0.703 mmol, 3.0 equiv) were added to a solution of Compound **3-1** (100.0 mg, 0.234 mmol, 1.0 equiv) in 1,4-dioxane (5 mL) in a microwave tube. The reaction mixture was heated to 110 °C under a nitrogen atmosphere and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **137-1** (102.0 mg, 90.44%).

LCMS: (ESI, m/z): 483.3 [M+H]⁺.

### Step 2: Synthesis of Compound 137

Referring to the preparation method of tetrazole in Compound **2,** Compound **137** (6.08 mg, yield 5.46%) was obtained.

LCMS: (ESI, m/z): 526.2 [M+H]⁺.

¹H NMR (400 MHz, CD₃OD) δ 9.11 - 9.09 (m, 1H), 7.85 - 7.83 (m, 1H), 7.74 - 7.70 (m, 1H), 7.47 (s, 1H), 6.92 (s, 1H), 4.34 (d, *J* = 14.4 Hz, 1H), 3.82 (d, *J* = 14.8 Hz, 1H), 2.97 - 2.93 (m, 1H), 2.89 - 2.85 (m, 3H), 2.80 - 2.75 (m, 2H), 2.71 -2.63 (m, 5H), 2.55 - 2.53 (m, 3H), 1.96 - 1.89 (m, 1H), 1.05 (d, *J =* 5.6 Hz, 3H), 0.94 (d, *J =* 6.8 Hz, 6H).

### Example 48 Preparation of Compound 165

Referring to the preparation method of Compound **3,** using 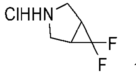 to replace 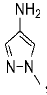 Compound **165** (23.05 mg, yield 27.78%) was obtained by column chromatography after a 2-step reaction.

LCMS: (ESI, m/z): 510.3 [M+H]⁺.

¹H NMR (400 MHz, CD₃OD) δ 9.09 (d, *J* = 4.8 Hz, 1H), 7.81 (d, *J* = 8.4 Hz, 1H), 7.72 - 7.69 (m, 1H), 6.55 (d, *J =* 19.6 Hz, 2H), 4.25 (d, *J* = 14.4 Hz, 1H), 3.77 (d, *J* = 15.6 Hz, 1H), 3.21 (d, *J* = 9.2 Hz, 2H), 3.13 - 3.06 (m, 2H), 2.99 - 2.93 (m, 1H), 2.86 - 2.84 (m, 2H), 2.68 - 2.58 (m, 2H), 2.48 - 2.41 (m, 3H), 2.28 - 2.22 (m, 3H), 1.92 - 1.86 (m, 1H), 1.00 (d, *J* = 6.4 Hz, 3H), 0.93 (d, *J =* 6.4 Hz, 6H).

### Example 49 Preparation of Compound 173

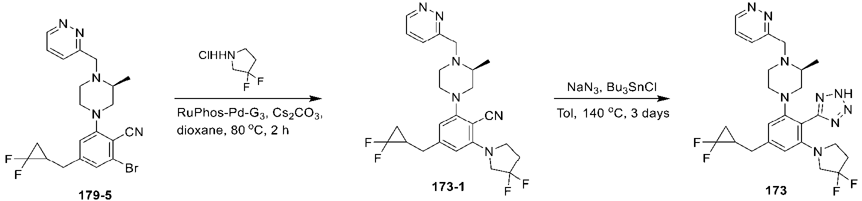

Referring to the preparation method of Compound **3,** using **179-5** to replace **3-1** and using 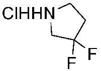 to replace 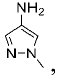 Compound **173** (4.62 mg, yield 4.25%) was obtained by column chromatography after a 2-step reaction.

LCMS: (ESI, m/z): 532.2 [M+H]⁺.

¹H NMR (400 MHz, CD₃OD) δ 9.09 (d, *J* = 3.2 Hz, 1H), 7.83 - 7.80 (m, 1H), 7.72 - 7.69 (m, 1H), 6.68 (d, *J* = 33.6 Hz, 2H), 4.26 (d, *J =* 13.6 Hz, 1H), 3.79 (d, *J* = 13.6 Hz, 1H), 3.16 - 3.12 (m, 2H), 2.98 (d, *J* = 3.2 Hz, 1H), 2.96 - 2.87 (m, 4H), 2.81 - 2.77 (m, 2H), 2.70 - 2.59 (m, 2H), 2.51 - 2.46 (m, 1H), 2.41 - 2.15 (m, 3H), 1.92 - 1.85 (m, 1H), 1.56 - 1.48 (m, 1H), 1.20 - 1.13 (m, 1H), 1.00 (d, *J=* 6.4 Hz, 3H).

### Example 50 Preparation of Compound 179

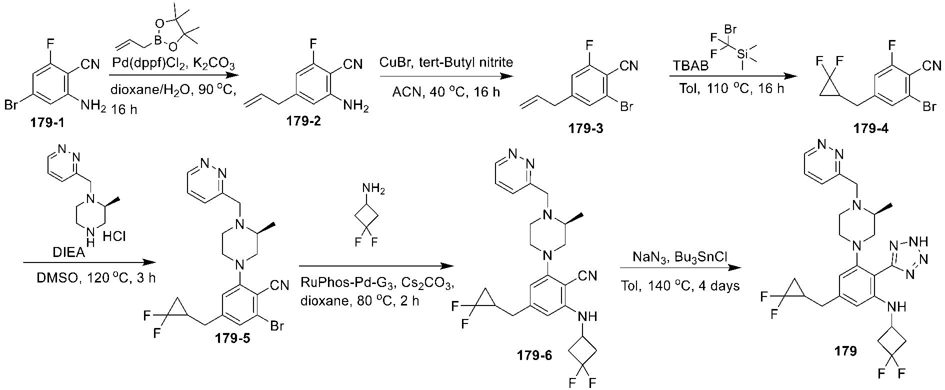

### Step 1: Synthesis of Compound 179-2

At room temperature, 2-allyl-4,4,5,5-tetramethyl-1,3,2-dioxaborolane (4.69 g, 27.90 mmol, 1.2 equiv), [1,1'-bis(diphenylphosphino)ferrocene]dichloropalladium(II) (1.7 g, 2.33 mmol, 0.1 equiv), and potassium carbonate (9.64 g, 69.76 mmol, 3.0 equiv) were added to a solution of 2-amino-4-bromo-6-fluorobenzonitrile (5 g, 23.25 mmol, 1.0 equiv) in 1,4-dioxane (30 mL) and water (6 mL). The reaction mixture was heated to 90 °C under a nitrogen atmosphere and stirred for 16 hours. After the reaction was completed, the reaction mixture was quenched by adding ice water (50 mL) and extracted with ethyl acetate (50 mL x 3). The combined organic layers were washed with saturated brine (50 mL), dried over anhydrous sodium sulfate and concentrated. The resulting residue was purified by silica gel column chromatography with ethyl acetate/petroleum ether (0 - 50%) to give Compound **179-2** as a yellow oil (6 g, crude).

LCMS: (ESI, m/z): 177.1 [M+H]⁺.

### Step 2: Synthesis of Compound 179-3

At room temperature, copper(I) bromide (9.77 g, 68.11 mmol, 2.0 equiv) and tert-butyl nitrite (10.53 g, 102.16 mmol, 3.0 equiv) were added to a solution of 4-allyl-2-amino-6-fluorobenzonitrile (6 g, 34.05 mmol, 1.0 equiv) in acetonitrile (50 mL). The reaction mixture was heated to 40 °C under a nitrogen atmosphere and stirred for 16 hours. After the reaction was completed, the reaction mixture was quenched by adding ice water (50 mL) and extracted with ethyl acetate (50 mL x 3). The combined organic layers were washed with saturated brine (50 mL), dried over anhydrous sodium sulfate and concentrated. The resulting residue was purified by silica gel column chromatography with ethyl acetate/petroleum ether (0 - 10%) to give Compound **179-3** as a yellow oil (1.99 g, yield: 24.34%).

¹H NMR (400 MHz, CDCl₃) δ 7.34 (s, 1H), 7.02 (d, *J* = 9.2 Hz, 1H), 5.91 - 5.84 (mz, 1H), 5.23 - 5.14 (m, 2H), 3.42 (d, *J =* 6.8 Hz, 2H).

### Step 3: Synthesis of Compound 179-4

At room temperature, tetrabutylammonium bromide (267.2 mg, 0.829 mmol, 0.1 equiv) and (bromodifluoromethyl)trimethylsilane (8.42 g, 41.45 mmol, 5.0 equiv) were added to a solution of Compound **179-3** (1.99 g, 8.29 mmol, 1.0 equiv) in toluene (10 mL) in a microwave tube. The reaction mixture was heated to 110 °C under a nitrogen atmosphere and stirred for 16 hours. After the reaction was completed, the reaction mixture was concentrated. The resulting residue was purified by silica gel column chromatography with ethyl acetate/petroleum ether (0 - 10%) to give Compound **179-4** as a yellow oil (1.2 g, yield: 49.91%).

¹H NMR (400 MHz, CDCl₃) δ 7.38 (s, 1H), 7.08 (d, *J* = 9.2 Hz, 1H), 2.90 - 2.79 (m, 2H), 1.82 - 1.71 (m, 1H), 1.64 - 1.57 (m, 1H), 1.17 - 1.09 (m, 1H).

### Step 5: Synthesis of Compound 179-4

At room temperature, N,N-diisopropylethylamine (468 mg, 3.63 mmol, 3.0 equiv) and (S)-3-((2-methylpiperazin-1-yl)methyl)pyridazine hydrochloride (331 mg, 1.45 mmol, 1.2 equiv) were added to a solution of Compound **179-4** (350 mg, 1.21 mmol, 1.0 equiv) in dimethyl sulfoxide (5 mL). The reaction mixture was heated to 120 °C under a nitrogen atmosphere and stirred for 3 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound 179-5 as a yellow solid (280 mg, crude).

LCMS: (ESI, m/z): 461.9 [M+H]⁺.

### Step 5: Synthesis of Compound 179-6

At room temperature, 3,3-difluorocyclobutan-1-amine (67 mg, 0.62 mmol, 1.2 equiv), (2-dicyclohexylphosphino-2',6'-diisopropoxy-1,1'-biphenyl)(2-amino-1,1'-biphenyl-2-yl)palladium(II) methanesulfonate (44 mg, 0.0519 mmol, 0.1 equiv), and cesium carbonate (508 mg, 1.56 mmol, 3.0 equiv) were added to a solution of Compound 179-5 (240 mg, 0.519 mmol, 1.0 equiv) in 1,4-dioxane (5 mL). The reaction mixture was heated to 80 °C under a nitrogen atmosphere and stirred for 2 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **179-6** as a yellow solid (70 mg, yield: 27.60%).

LCMS: (ESI, m/z): 489.5 [M+H]⁺.

### Step 6: Synthesis of Compound 179

Referring to the preparation method of tetrazole in Compound **2,** Compound **179** (28.03 mg, yield 36.80%) was obtained.

LCMS: (ESI, m/z): 532.3 [M+H]⁺.

¹H NMR (400 MHz, CD₃OD) δ 9.10 - 9.09 (m, 1H), 7.87 - 7.85 (m, 1H), 7.74 - 7.70 (m, 1H), 6.58 (s, 1H), 6.38 (s, 1H), 4.33 (d, *J=* 14.4 Hz, 1H), 3.92 - 3.87 (m, 1H), 3.78 (d, *J=* 14.4 Hz, 1H), 3.09 - 3.02 (m, 2H), 2.92 - 2.90 (m, 3H), 2.79 - 2.64 (m, 5H), 2.58 - 2.55 (m, 1H), 2.49 - 2.39 (m, 2H), 1.93 - 1.80 (m, 1H), 1.55 - 1.52 (m, 1H), 1.20 - 1.16 (m, 1H), 1.09 (d, *J =* 6.0 Hz, 3H).

### Example 51 Preparation of Compound 184-A

Referring to the preparation method of Compound **3,** using 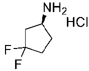 to replace 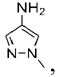 Compound **184-A** (35.47 mg, yield 36.10%) was obtained by column chromatography after a 2-step reaction.

LCMS: (ESI, m/z): 512.4 [M+H]⁺.

¹H NMR (400 MHz, CD₃OD) δ 9.09 (d, *J* = 4.0 Hz, 1H), 8.35 (s, 0.24 HCOOH), 7.87 (d, *J* = 7.6 Hz, 1H), 7.73 - 7.70 (m, 1H), 6.44 (d, *J =* 24.0 Hz, 2H), 4.32 (d, *J* = 14.4 Hz, 1H), 4.11 - 4.06 (m, 1H), 3.77 (d, *J* = 14.4 Hz, 1H), 2.95 - 2.93 (m, 3H), 2.79 - 2.67 (m, 3H), 2.61 - 2.53 (m, 2H), 2.47 (d, *J =* 7.2 Hz, 2H), 2.31 - 2.07 (m, 3H), 1.98 - 1.88 (m, 2H), 1.78 - 1.72 (m, 1H), 1.09 (d, *J =* 5.6 Hz, 3H), 0.94 (d, *J* = 6.4 Hz, 6H).

### Example 52 Preparation of Compound 184-B

Referring to the preparation method of Compound 3, using 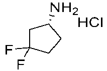 to replace 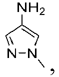 Compound **184-B** (43.25 mg, yield 44.01%) was obtained by column chromatography after a 2-step reaction.

LCMS: (ESI, m/z): 512.3 [M+H]⁺.

¹H NMR (400 MHz, CD₃OD) δ 9.09 (d, *J* = 4.8 Hz, 1H), 7.87 (d, *J* = 8.2 Hz, 1H), 7.73 - 7.70 (m, 1H), 6.44 (d, *J* = 23.6 Hz, 2H), 4.32 (d, *J* = 14.4 Hz, 1H), 4.11 - 4.08 (m, 1H), 3.77 (d, *J* = 14.4 Hz, 1H), 2.95 - 2.92 (m, 3H), 2.79 - 2.66 (m, 3H), 2.62 - 2.52 (m, 2H), 2.47 (d, *J = 7.2* Hz, 2H), 2.31 - 2.06 (m, 3H), 1.96 - 1.88 (m, 2H), 1.79 - 1.72 (m, 1H), 1.09 (d, *J* = 6.0 Hz, 3H), 0.94 (d, *J* = 6.4 Hz, 6H).

### Example 53 Preparation of Compound 185

Referring to the preparation method of Compound **3,** using 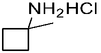 to replace 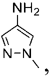 Compound **185** (32.09 mg, yield 30.72%) was obtained by column chromatography after a 2-step reaction.

LCMS: (ESI, m/z): 476.3 [M+H]⁺.

¹H NMR (400 MHz, CD₃OD) δ 9.09 (s, 1H), 7.87 (d, *J* = 7.6 Hz, 1H), 7.73 - 7.70 (m, 1H), 6.32 (d, *J =* 66.0 Hz, 2H), 4.33 (d, *J =* 14.4 Hz, 1H), 3.77 (d, *J =* 14.4 Hz, 1H), 2.98 - 2.91 (m, 3H), 2.80 - 2.68 (m, 3H), 2.61 - 2.56 (m, 1H), 2.43 - 2.40 (m, 2H), 2.23 - 2.19 (m, 2H), 2.15 - 2.09 (m, 2H), 1.97 - 1.84 (m, 3H), 1.48 (s, 3H), 1.10 (d, *J* = 4.8 Hz, 3H), 0.93 (d, *J =* 5.6 Hz, 6H).

### Example 54 Preparation of Compound 196

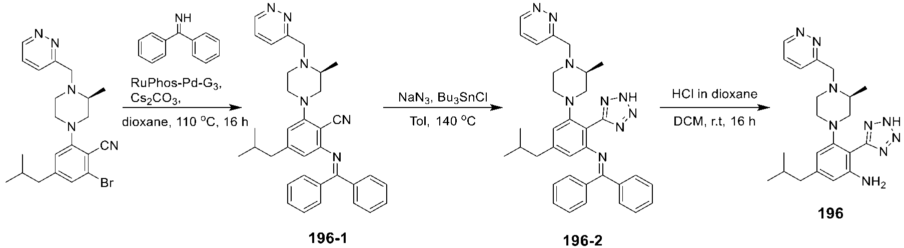

### Step 1: Synthesis of Compound 196-1

Benzophenone imine (212 mg, 1.17 mmol, 1.0 equiv), (2-dicyclohexylphosphino-2',6'-diisopropoxy-1,1'-biphenyl)(2-amino-1,1'-biphenyl-2-yl)palladium(II) methanesulfonate (98 mg, 0.117 mmol, 0.1 equiv), and cesium carbonate (1.14 g, 3.5 mmol, 3.0 equiv) were added to a solution of Compound 3-1 (500.0 mg, 1.17 mmol, 1.0 equiv) in 1,4-dioxane (10 mL) in a microwave tube. The reaction mixture was heated to 110 °C under a nitrogen atmosphere and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **196-1** (600.0 mg, 97.23%).

LCMS: (ESI, m/z): 529.4 [M+H]⁺.

### Step 2: Synthesis of Compound 196-2

Under a nitrogen atmosphere, sodium azide (369.0 mg, 5.67 mmol, 5 equiv) and tri-n-butyltin azide (1.85 g, 5.67 mmol, 5 equiv) were added to a solution of Compound **196-1** (600.0 mg, 1.13 mmol, 1.0 equiv) in toluene (6 mL) in a microwave tube. The reaction mixture was heated to 140 °C and stirred for 3 days. After the reaction was completed, the reaction mixture was filtered through solid potassium fluoride, and the solid was washed with ethyl acetate. The filtrate was concentrated, and the resulting residue was directly purified by column chromatography with methanol/dichloromethane (0 - 10%) to give Compound 196-2 (292 mg, yield 45.0%).

LCMS: (ESI, m/z): 572.2 [M+H]⁺.

### Step 3: Synthesis of Compound 196

Referring to **the** preparation **method of tetrazole** in Compound **2,** Compound **196** (78.57 mg, yield 37.75%) was obtained.

LCMS: (ESI, m/z): 408.2 [M+H]⁺.

¹H NMR (400 MHz, CD₃OD) δ 9.10 - 9.08 (m, 1H), 7.88 - 7.86 (m, 1H), 7.73 - 7.70 (m, 1H), 6.48 (s, 1H), 6.40 (s, 1H), 4.32 (d, *J* = 14.4 Hz, 1H), 3.76 (d, *J* = 14.4 Hz, 1H), 2.91 - 2.85 (m, 3H), 2.77 - 2.71 (m, 2H), 2.68 - 2.61 (m, 1H), 2.60 - 2.51 (m, 1H), 2.40 (d, *J=* 7.2 Hz, 2H), 1.92 - 1.85 (m, 1H), 1.09 (d, *J=* 6.0 Hz, 3H), 0.92 (d, *J* = 6.4 Hz, 6H).

### Example 55 Preparation of Compound 197

Referring to the preparation method of Compound 3, using 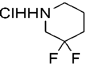 to replace 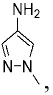 Compound **197** (46.29 mg, yield 47.18%) was obtained by column chromatography after a 2-step reaction.

LCMS: (ESI, m/z): 512.7 [M+H]⁺.

¹H NMR (400 MHz, CD₃OD) δ 9.09 - 9.07 (m, 1H), 7.84 - 7.81 (m, 1H), 7.72 - 7.68 (m, 1H), 6.81 (d, *J* = 3.2 Hz, 2H), 4.20 (d, *J* = 14.4 Hz, 1H), 3.72 (d, *J* = 14.0 Hz, 1H), 3.04 - 2.99 (m, 3H), 2.94 - 2.83 (m, 4H), 2.66 - 2.61 (m, 2H), 2.52 (d, *J =* 7.2 Hz, 2H), 2.46 - 2.42 (m, 1H), 2.26 - 2.20 (m, 1H), 1.94 - 1.76 (m, 3H), 1.50 - 1.44 (m, 2H), 0.98 (d, *J =* 6.4 Hz, 3H), 0.93 (d, *J* = 6.8 Hz, 6H).

### Example 56 Preparation of Compound 198

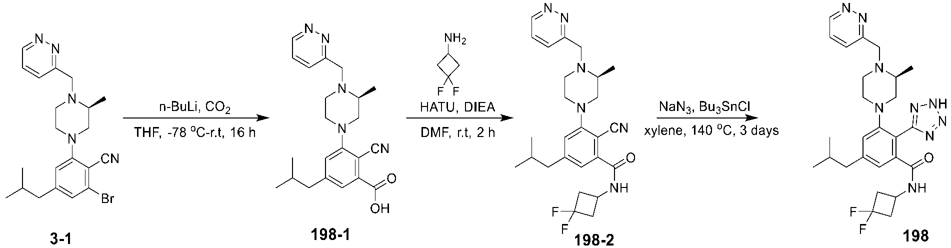

### Step 1: Synthesis of Compound 198-1

n-Butyllithium (0.9 mL, 1.6 M, 2.0 equiv) was added to a solution of Compound **3-1** (300.0 mg, 0.7 mmol, 1.0 equiv) in tetrahydrofuran (5 mL) in a three-necked flask at -78 °C. The mixture was stirred under a nitrogen atmosphere for 30 minutes, then carbon dioxide gas was introduced, and the reaction mixture was stirred at room temperature for 16 hours. After the reaction was completed, the reaction mixture was added to an ice-cold dilute hydrochloric acid solution and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **198-1** as a yellow solid (60.0 mg, 21.77%).

LCMS: (ESI, m/z): 394.1 [M+H]⁺.

### Step 2: Synthesis of Compound 198-2

N,N,N',N'-tetramethyl-O-(7-azabenzotriazol-1-yl)uronium hexafluorophosphate (87 mg, 0.228 mmol, 1.5 equiv), N,N-diisopropylethylamine (60 mg, 0.457 mmol, 3.0 equiv), and 3,3-difluorocyclobutan-1-amine (17 mg, 0.152 mmol, 0.1 equiv) were added to a solution of Compound **198-1** (60.0 mg, 0.152 mmol, 1.0 equiv) in N,N-dimethylformamide (2 mL) in a microwave tube. The reaction mixture was stirred at room temperature for 2 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **198-2** as a yellow solid (40.0 mg, 54.36%).

LCMS: (ESI, m/z): 483.3 [M+H]⁺.

### Step 3: Synthesis of Compound 198

Referring to the preparation method of tetrazole in Compound 2, Compound 198 (10.74 mg, yield 24.65%) was obtained.

LCMS: (ESI, m/z): 526.2 [M+H]⁺.

¹H NMR (400 MHz, CD₃OD) δ 9.10 - 9.08 (m, 1H), 7.84 - 7.81 (m, 1H), 7.72 - 7.69 (m, 1H), 7.15 (d, *J =* 6.4 Hz, 2H), 4.27 (d, *J =* 14.4 Hz, 1H), 4.08 - 4.03 (m, 1H), 3.78 (d, *J =* 14.4 Hz, 1H), 2.88 - 2.79 (m, 5H), 2.72 - 2.70 (m, 1H), 2.63 - 2.45 (m, 6H), 2.39 - 2.34 (m, 1H), 1.98 - 1.92 (m, 1H), 0.99 (d, *J=* 6.0 Hz, 3H), 0.95 (d, *J* = 6.4 Hz, 6H).

### Example 57 Preparation of Compound 199

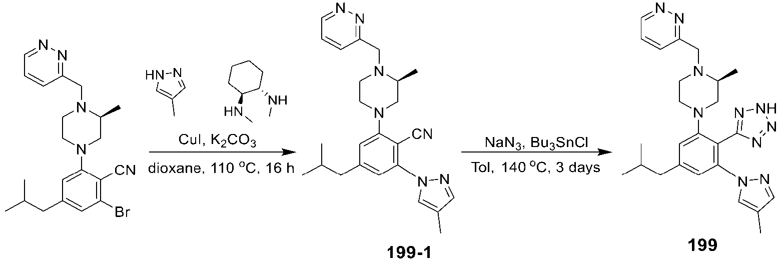

### Step 1: Synthesis of Compound 199-1

4-methylpyrazole (77.0 mg, 0.936 mmol, 2.0 equiv), (1R,2R)-(-)-N,N'-dimethyl-1,2-cyclohexanediamine (13 mg, 0.094 mmol, 0.2 equiv), copper(I) iodide (9.0 mg, 0.047 mmol, 0.1 equiv), and potassium carbonate (194 mg, 1.405 mmol, 3.0 equiv) were added to a solution of Compound **3-1** (200.0 mg, 0.468 mmol, 1.0 equiv) in 1,4-dioxane (5 mL) in a microwave tube. The reaction mixture was heated to 110 °C under a nitrogen atmosphere and stirred for 16 hours. After the reaction was completed, the reaction mixture was quenched by adding ice water (20 mL) and extracted with ethyl acetate (20 mL x 3). The combined organic layers were washed with saturated brine (20 mL), dried over anhydrous sodium sulfate and concentrated. The resulting residue was purified by silica gel column chromatography with methanol/dichloromethane (0 - 10%) to give Compound **199-1** as a yellow solid (72.0 mg, 35.86%).

LCMS: (ESI, m/z): 430.3 [M+H]⁺.

### Step 2: Synthesis of Compound 199

Referring to the preparation method of tetrazole in Compound **2,** Compound **199** (26.47 mg, yield 19.85%) was obtained.

LCMS: (ESI, m/z): 473.3 [M+H]⁺.

¹H NMR (400 MHz, DMSO-*d*₆) δ 9.11 - 9.10 (m, 1H), 7.68 - 7.62 (m, 2H), 7.42 (s, 1H), 7.22 (s, 1H), 7.12 (d, *J =* 1.2 Hz, 1H), 7.05 (s, 1H), 4.02 (d, *J =* 14.4 Hz, 1H), 3.59 (d, *J =* 14.0 Hz, 1H), 2.86 - 2.79 (m, 2H), 2.73 - 2.69 (m, 1H), 2.55 (d, *J =* 7.2 Hz, 3H), 2.47 - 2.44 (m, 1H), 2.29 (s, 1H), 2.05 - 2.00 (m, 1H), 1.96 - 1.89 (m, 4H), 0.91 (d, *J =* 6.4 Hz, 6H), 0.85 (d, *J =* 6.0 Hz, 3H).

The following compounds were synthesized referring to the general preparation methods of the present invention and the aforementioned examples:

| Compound | LCMS (ESI, m/z) | Compound | LCMS (ESI, m/z) |
|---|---|---|---|
| 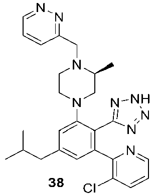 | 504.2 [M+H]⁺ | 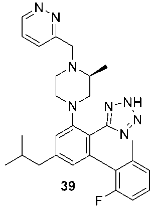 | 501.3 [M+H]⁺ |
| 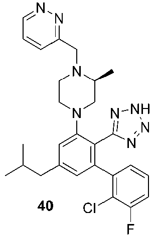 | 521.2 [M+H]⁺ | 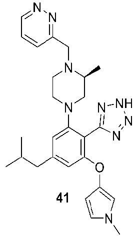 | 488.3 [M+H]⁺ |
| 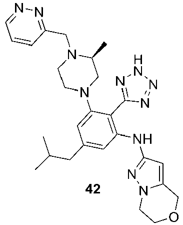 | 530.3 [M+H]⁺ | 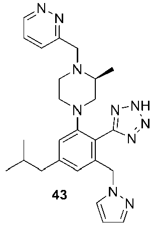 | 473.3 [M+H]⁺ |
| 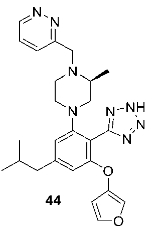 | 475.3 [M+H]⁺ | 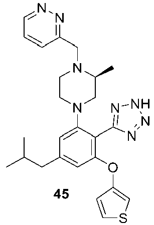 | 491.2 [M+H]⁺ |
| 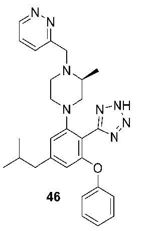 | 485.3 [M+H]⁺ | 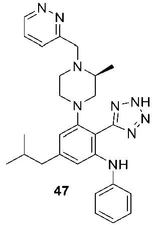 | 484.3 [M+H]⁺ |
| 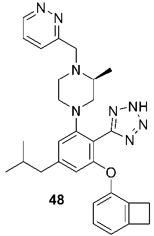 | 511.3 [M+H]⁺ | 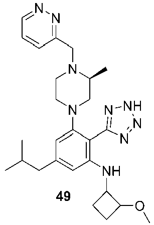 | 492.3 [M+H]⁺ |
| 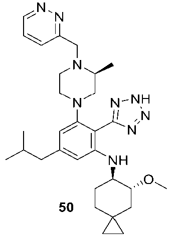 | 546.4 [M+H]⁺ | 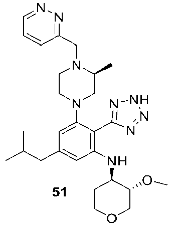 | 522.3 [M+H]⁺ |
| 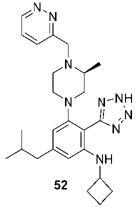 | 462.3 [M+H]⁺ | 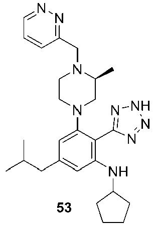 | 476.3 [M+H]⁺ |
| 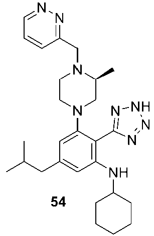 | 490.3 [M+H]⁺ | 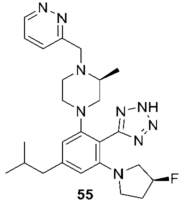 | 480.3 [M+H]⁺ |
| 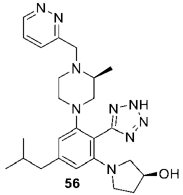 | 478.3 [M+H]⁺ | 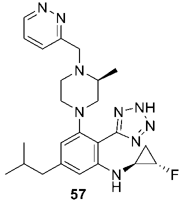 | 466.3 [M+H]⁺ |
| 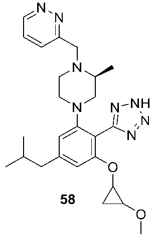 | 479.3 [M+H]⁺ | 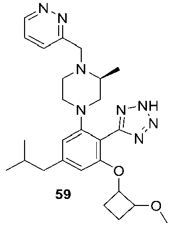 | 493.3 [M+H]⁺ |
| 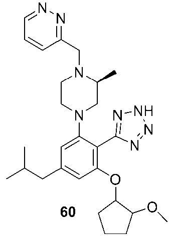 | 507.3 [M+H]⁺ | 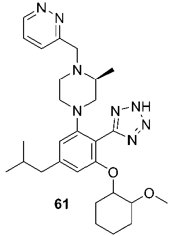 | 521.3 [M+H]⁺ |
| 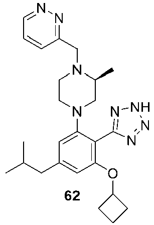 | 463.3 [M+H]⁺ | 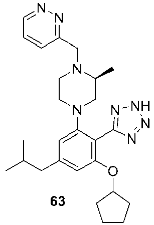 | 477.3 [M+H]⁺ |
| 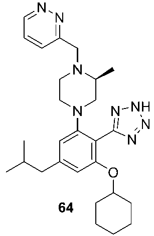 | 491.3 [M+H]⁺ | 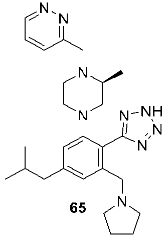 | 476.3 [M+H]⁺ |
| 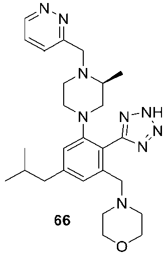 | 492.3 [M+H]⁺ | 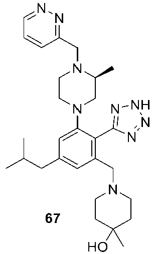 | 520.3 [M+H]⁺ |
| 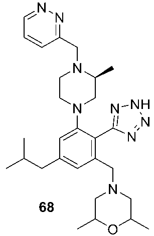 | 520.3 [M+H]⁺ | 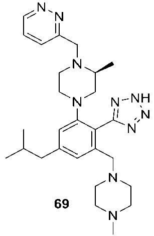 | 505.3 [M+H]⁺ |
| 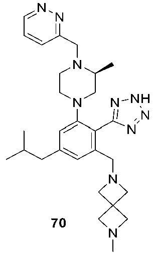 | 517.3 [M+H]⁺ | 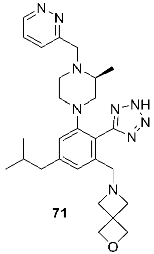 | 504.3 [M+H]⁺ |
| 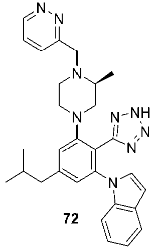 | 508.3 [M+H]⁺ | 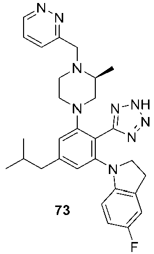 | 528.3 [M+H]⁺ |
| 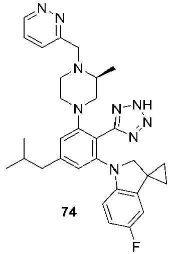 | 554.3 [M+H]⁺ | 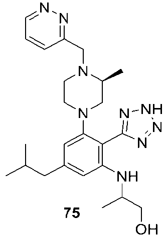 | 466.3 [M+H]⁺ |
| 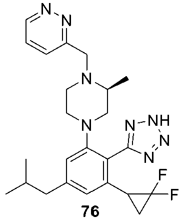 | 469.3 [M+H]⁺ | 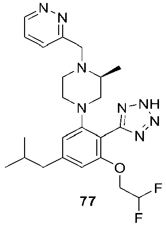 | 473.3 [M+H]⁺ |
| 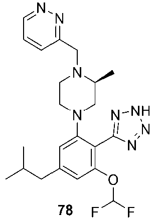 | 459.2 [M+H]⁺ | 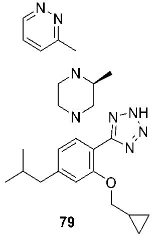 | 463.3 [M+H]⁺ |
| 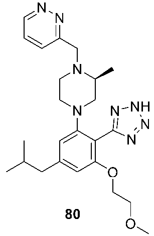 | 467.3 [M+H]⁺ | 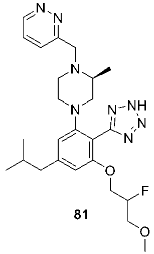 | 499.3 [M+H]⁺ |
| 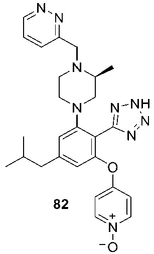 | 502.3 [M+H]⁺ | 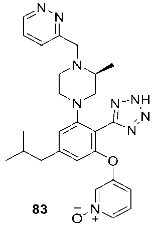 | 502.3 [M+H]⁺ |
| 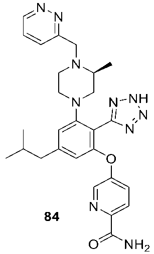 | 529.3 [M+H]⁺ | 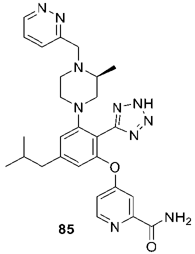 | 529.3 [M+H]⁺ |
| 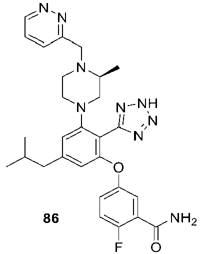 | 546.3 [M+H]⁺ | 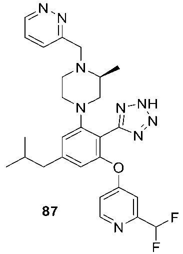 | 536.3 [M+H]⁺ |
| 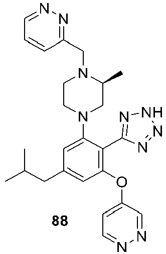 | 487.3 [M+H]⁺ | 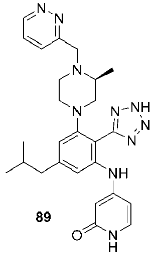 | 501.3 [M+H]⁺ |
| 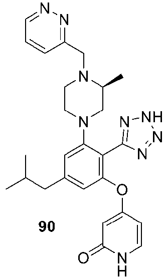 | 502.3 [M+H]⁺ | 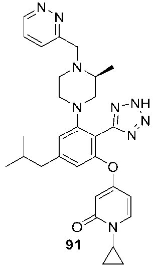 | 542.3 [M+H]⁺ |
| 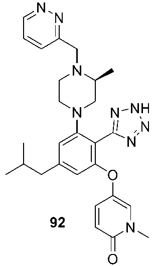 | 516.3 [M+H]⁺ | 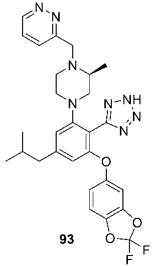 | 565.2 [M+H]⁺ |
| 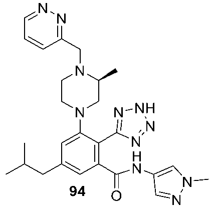 | 516.3 [M+H]⁺ | 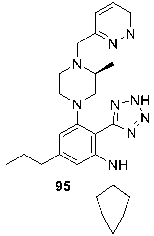 | 488.3 [M+H]⁺ |
| 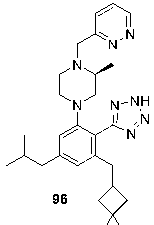 F F | 497.3 [M+H]⁺ | 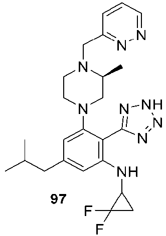 | 484.3 [M+H]⁺ |
| 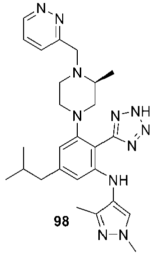 | 502.3 [M+H]⁺ | 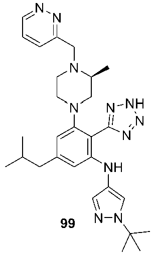 | 530.3 [M+H]⁺ |
| 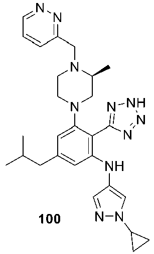 | 513.3 [M+H]⁺ | 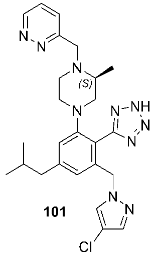 | 507.3 [M+H]⁺ |
| 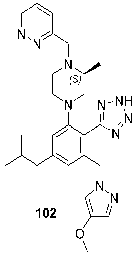 | 503.3 [M+H]⁺ | 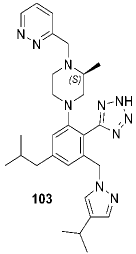 | 515.3 [M+H]⁺ |
| 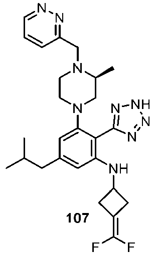 | 510.3 [M+H]⁺ | 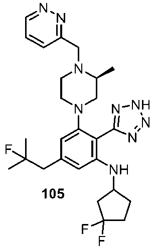 | 530.3 [M+H]⁺ |
| 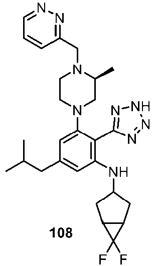 | 524.3 [M+H]⁺ | 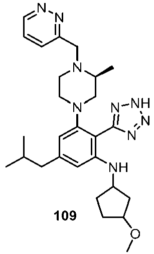 | 506.3 [M+H]⁺ |
| 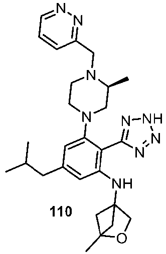 | 504.3 [M+H]⁺ | 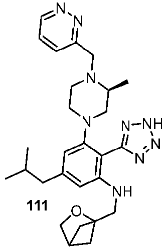 | 504.3 [M+H]⁺ |
| 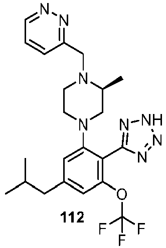 | 477.3 [M+H]⁺ | 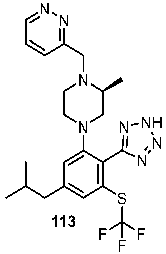 | 493.3 [M+H]⁺ |
| 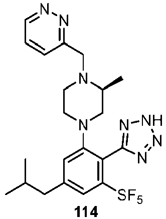 | 512.2 [M+H]⁺ | 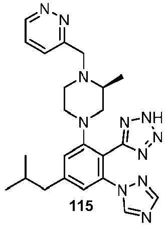 | 460.3 [M+H]⁺ |
| 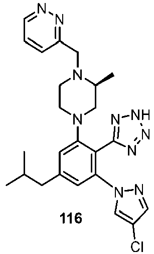 | 492.3 [M+H]⁺ | 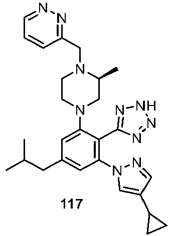 | 499.3 [M+H]⁺ |
| 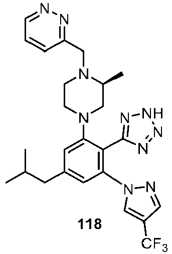 | 527.3 [M+H]⁺ | 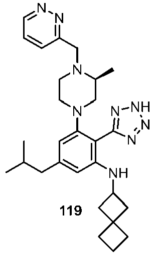 | 502.3 [M+H]⁺ |
| 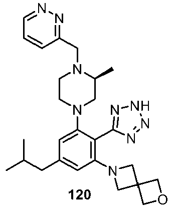 | 490.3 [M+H]⁺ | 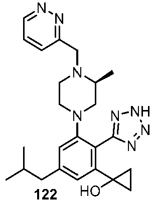 | 449.3 [M+H]⁺ |
| 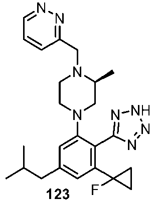 | 451.3 [M+H]⁺ | 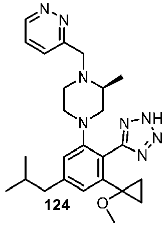 | 462.3 [M+H]⁺ |
| 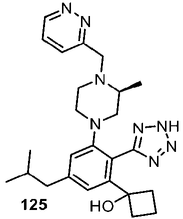 | 463.3 [M+H]⁺ | 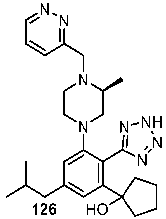 | 477.3 [M+H]⁺ |
| 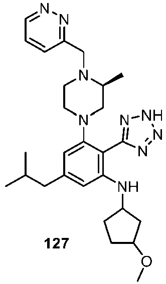 | 506.3 [M+H]⁺ | 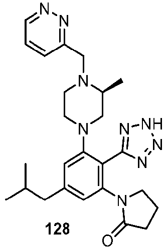 | 476.3 [M+H]⁺ |
| 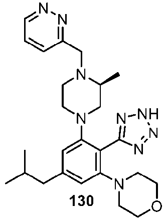 | 478.3 [M+H]⁺ | 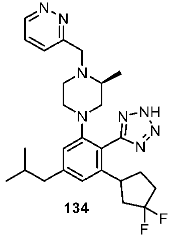 | 497.3 [M+H]⁺ |
| 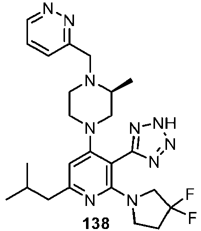 | 499.3 [M+H]⁺ | 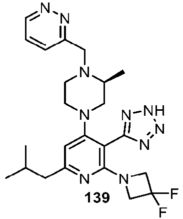 | 485.3 [M+H]⁺ |
| 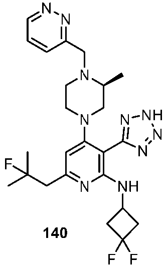 | 517.3 [M+H]⁺ | 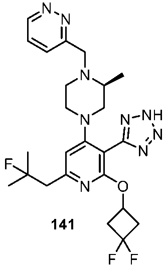 | 518.3 [M+H]⁺ |
| 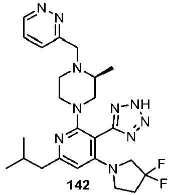 | 499.3 [M+H]⁺ | 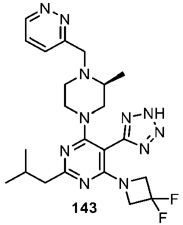 | 486.3 [M+H]⁺ |
| 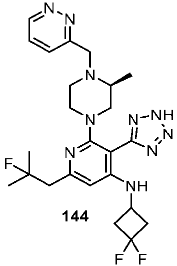 | 517.3 [M+H]⁺ | 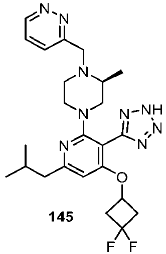 | 500.3 [M+H]⁺ |
| 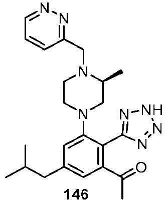 | 435.3 [M+H]⁺ | 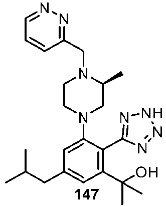 | 451.3 [M+H]⁺ |
| 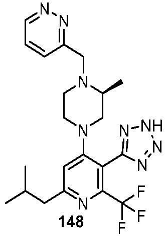 | 462.3 [M+H]⁺ | 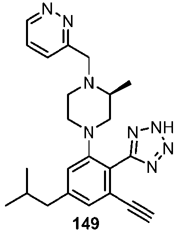 | 417.3 [M+H]⁺ |
| 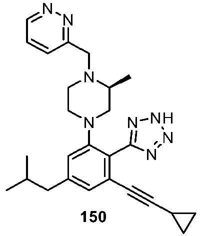 | 457.3 [M+H]⁺ | 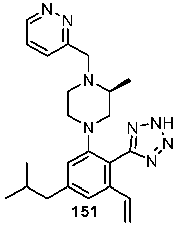 | 419.3 [M+H]⁺ |
| 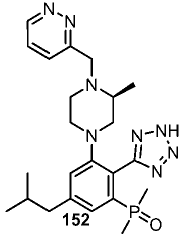 | 469.3 [M+H]⁺ | 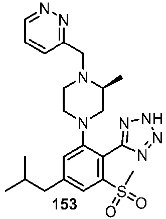 | 471.3 [M+H]⁺ |
| 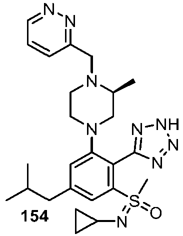 | 510.3 [M+H]⁺ | 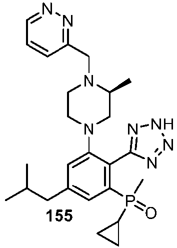 | 495.3 [M+H]⁺ |
| 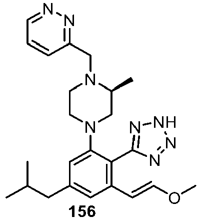 | 449.3 [M+H]⁺ | 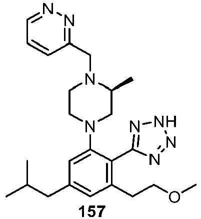 | 451.3 [M+H]⁺ |
| 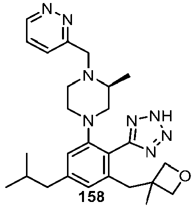 | 477.3 [M+H]⁺ | 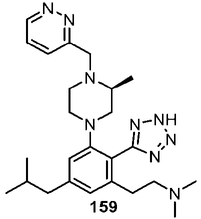 | 464.3 [M+H]⁺ |
| 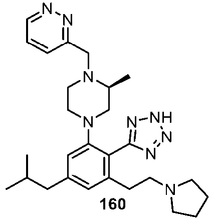 | 490.3 [M+H]⁺ | 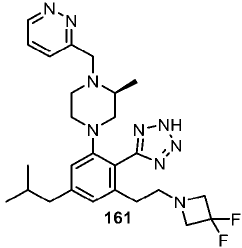 | 512.3 [M+H]⁺ |
| 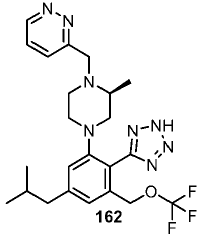 | 491.3 [M+H]⁺ | 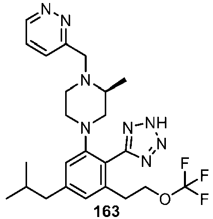 | 505.3 [M+H]⁺ |
| 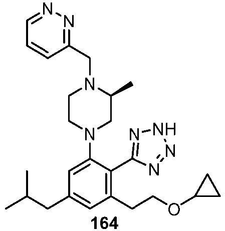 | 477.3 [M+H]⁺ | 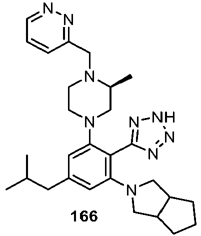 | 502.3 [M+H]⁺ |
| 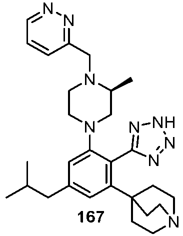 | 502.3 [M+H]⁺ | 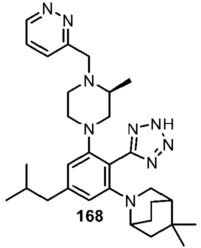 | 530.3 [M+H]⁺ |
| 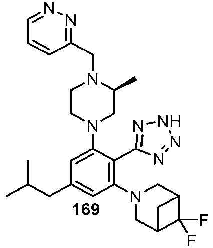 | 524.3 [M+H]⁺ | 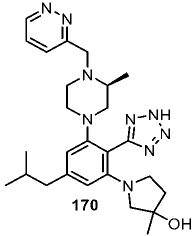 | 492.3 [M+H]⁺ |
| 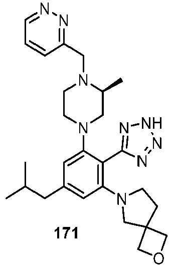 | 504.3 [M+H]⁺ | 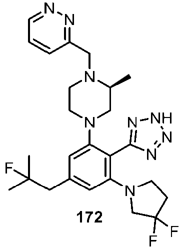 | 516.3 [M+H]⁺ |
| 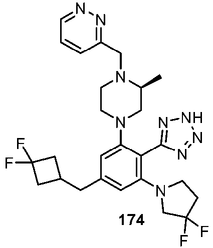 | 546.3 [M+H]⁺ | 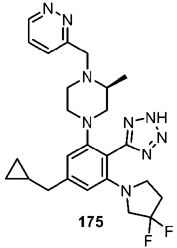 | 496.3 [M+H]⁺ |
| 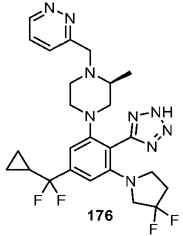 | 532.3 [M+H]⁺ | 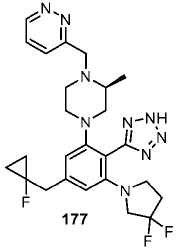 | 514.3 [M+H]⁺ |
| 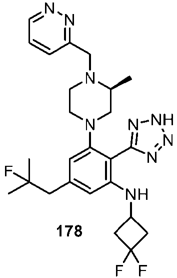 | 516.3 [M+H]⁺ | 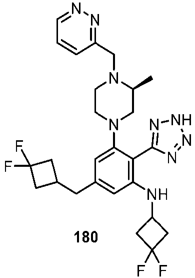 | 546.3 [M+H]⁺ |
| 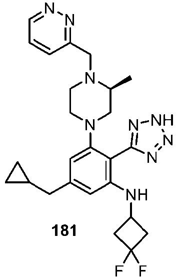 | 496.3 [M+H]⁺ | 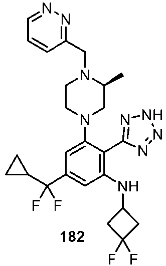 | 532.3 [M+H]⁺ |
| 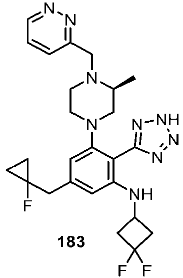 | 514.3 [M+H]⁺ | 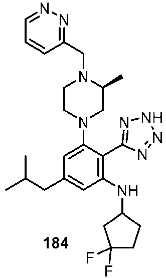 | 512.3 [M+H]⁺ |
| 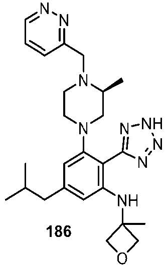 | 478.3 [M+H]⁺ | 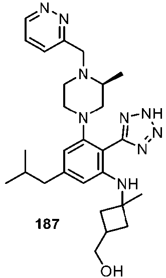 | 506.3 [M+H]⁺ |
| 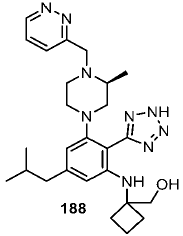 | 492.3 [M+H]⁺ | 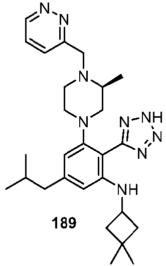 | 490.3 [M+H]⁺ |
| 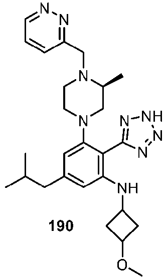 | 492.3 [M+H]⁺ | 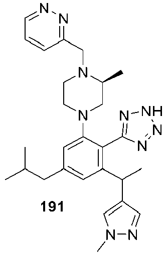 | 501.3 [M+H]⁺ |
| 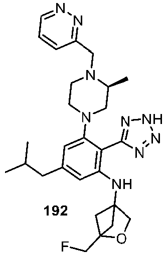 | 522.3 [M+H]⁺ | 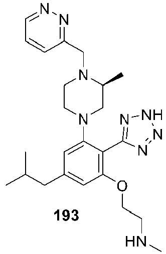 | 466.3 [M+H]⁺ |
| 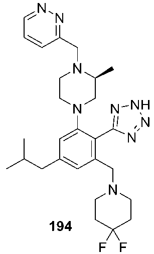 | 526.3 [M+H]⁺ | 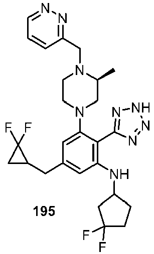 | 546.3 [M+H]⁺ |
| 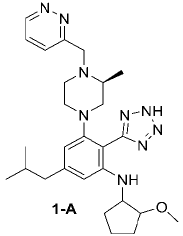 | 506.3 [M+H]⁺ | 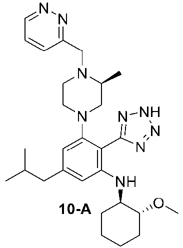 | 520.3 [M+H]⁺ |

### Test Example 1 Determination of the affinity of the test compounds for AT2 receptor by AT2R Binding assay

### Experimental procedure:

1. Reagent preparation
1.1 Cell line: Tag-lite Angiotensin AT2 labeled Cells;
1.2 Assay buffer: 1X TLB (5 mL 5X TLB + 20 mL H₂O);

2. Activity assay of the test compounds
2.1 1X TLB assay buffer, 4X working solutions of the test compounds, and 4X Tag-lite angiotensin receptor red agonist (Cisbio, L0007RED) were prepared;
2.2 One vial of labeled-AT2R cells was thawed in a water bath at 37 °C until the ice completely melted (1-2 min). The thawed cells were quickly transferred to 5 mL of 1X TLB, mixed gently, and centrifuged at 200 g for 5 min;
2.3 The supernatant was discarded, and the cells were resuspended and mixed with 1 mL of 1X TLB. Then, 1.7 mL of 1X TLB was added and mixed, and the mixture was kept at room temperature for later use;
2.4 10 µL of cells were added to a 384-well plate (Greiner, 784075) and centrifuged at 200 g at RT for 3 s; 5 µL of the 4X compound was added to the 384-well plate; 5 µL of the 4X Tag-lite angiotensin receptor red agonist was added to all test wells;
2.5 The reaction plate was left to stand at room temperature of 25 °C for 1 hour, and then centrifuged at 200 g, RT for 60 s. Data was collected using an Envision HTRF detector.

3. Data analysis
3.1 Calculation of % inhibition: 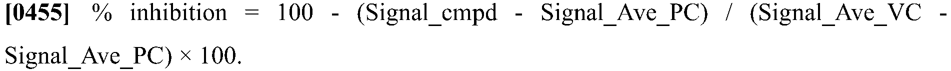 % inhibition = 100 - (Signal_cmpd - Signal_Ave_PC) / (Signal_Ave_VC - Signal_Ave_PC) × 100.
3.2 Calculation of compound IC₅₀ using GraphPad non-linear fitting formula:$Y = Bottom + \left(Top-Bottom\right) / \left(1+{10}^{∧}\left(\left({LogIC}_{50}-X\right)*HillSlope\right)\right)$
X: log value of compound concentration; Y: % inhibition

4. Experimental results

| Compound No. | AT2R IC₅₀ (nM) | Compound No. | AT2R IC₅₀ (nM) |
|---|---|---|---|
| 1 | 12.8 | 16 | 11.03 |
| 2 | 18.2 | 20 | 2.96 |
| 3 | 4.3 | 21 | 1.899 |
| 4 | 6.6 | 23 | 1.90 |
| 6 | 6.75 | 24 | 3.80 |
| 7 | 3.80 | 25 | 3.35 |
| 9 | 1.78 | 28 | 7.18 |
| 10 | 17.7 | 31 | 1.07 |
| 13 | 7.41 | 32 | 2.58 |
| 15 | 2.96 | 33 | 8.47 |
| 34 | 2.36 | 35 | 3.08 |
| 36 | 5.05 | 37 | 4.11 |
| 30 | 0.891 | 104 | 5.08 |
| 106 | 4.77 | 121 | 6.30 |
| 136 | 11.89 | 137 | 3.31 |
| 165 | 4.11 | 173 | 7.0 |
| 179 | 3.45 | 184-A | 3.31 |
| 184-B | 3.01 | 185 | 10.98 |
| 196 | 6.53 | 199 | 25.5 |

The experimental results show that the compounds of the present invention have a good binding effect on AT2R.

### Test Example 2 Determination of the affinity of the test compounds for AT1 receptor by AT1R Binding assay

### Experimental procedure:

1 Reagent preparation
1) Cell line: Tag-lite Angiotensin AT1 labeled Cells;
2) Assay buffer: 1X TLB (5 mL 5X TLB + 20 mL H₂O);

2 Inhibitory activity assay of the test compounds
1) 1X TLB assay buffer, 4X working solutions of the test compounds, and 4X Tag-lite angiotensin receptor red agonist (Cisbio, L0007RED) were prepared;
2) One vial of labeled-AT1R cells was thawed in a water bath at 37 °C until the ice completely melted (1-2 min). The thawed cells were quickly transferred to 5 mL of 1X TLB, mixed gently, and centrifuged at 200 g for 5 min;
3) The supernatant was discarded, and the cells were resuspended and mixed with 1 mL of 1X TLB. Then, 1.7 mL of 1X TLB was added and mixed, and the mixture was kept at room temperature for later use;
4) 10 µL of cells were added to a 384-well plate (Greiner, 784075) and centrifuged at 200 g at RT for 3 s; 5 µL of the 4X compound was added to the 384-well plate; 5 µL of the 4X Tag-lite angiotensin receptor red agonist was added to all test wells;
5) The reaction plate was left to stand at room temperature of 25 °C for 1 hour, and then centrifuged at 200 g, RT for 60 s. Data was collected using an Envision HTRF detector.

3 Data analysis
1) $Z ′ factor = 1 - 3 * \left(SDMax+SDMin\right) / \left(MeanMax-MeanMin\right) ;$
2) $CVMax = \left(SDMax/MeanMax\right) * 100 % ;$
3) $CVMin = \left(SDMin/MeanMin\right) * 100 % ;$
4) S/B = Singal/Background;
5) Calculation Equation for EC₅₀/IC₅₀ Value:
   $Y = Bottom + \left(Top-Bottom\right) / \left(1+{10}^{∧}\left(\left({LogEC}_{50}-X\right)*HillSlope\right)\right)$

4. Experimental results

| Compound No. | AT1R IC₅₀ (nM) |
|---|---|
| 1 | NA |
| 2 | NA |
| 3 | >1000 |
| 4 | NA |

The experimental results show that the compounds of the present invention have no binding effect on AT1R. (Note: NA: Not tested)

### Test Example 3 Study on the pharmacokinetic behavior of the compounds of the present invention in mice

### Experimental protocol:

Healthy ICR mice (SPF grade, source: Beijing Vital River Laboratory Animal Technology Co., Ltd.), 3 animals, male, weighing 18-25 g, were intravenously injected with 1 mg/kg of the compound at a dosing volume of 5 mL/kg, formulated with 5% DMSO + 10% Solutol + 85% Saline (w/v). Animals were fasted prior to the test.

Healthy ICR mice (SPF grade, source: Beijing Vital River Laboratory Animal Technology Co., Ltd.), 3 animals, male, weighing 18-25 g, were intragastrically administered with 5 mg/kg of the compound at a dosing volume of 10 mL/kg, formulated with 5% DMSO + 10% Solutol + 85% Saline (w/v). Animals were fasted prior to the test.

Blood samples (approx. 0.05 mL each) were collected via the cheek at 5 min, 15 min, 30 min, 1 h, 2 h, 4 h, 6 h, 8 h, and 24 h after intravenous administration, and at 5 min, 15 min, 30 min, 1 h, 2 h, 4 h, 6 h, 8 h, and 24 h after intragastric administration, respectively, using sodium heparin as an anticoagulant. After collection, the blood samples were placed on ice, and the plasma was separated by centrifugation within 1 hour (centrifugation conditions: 6000 g, 3 minutes, 2-8 °C). The concentration of the compound in plasma was determined by liquid chromatography-tandem mass spectrometry. Plasma samples were stored in a -80 °C freezer before analysis. Pharmacokinetic parameters were calculated using Phoenix WinNonlin 8.2.0 based on plasma concentration data at different time points. The experimental results are as follows:

| **Compound** | Intravenous injection (1 mg/kg) | | | | Intragastric administration (5 mg/kg) | | | |
|---|---|---|---|---|---|---|---|---|
| | Cₘₐₓ (ng/mL) | AUClast (h*ng/mL) | CL (mL/kg/min) | Vss (L/kg) | Cₘₐₓ (ng/mL) | AUClast (h*ng/mL) | T_{1/2} (h) | F% |
| **A174** | 4817 | 3043 | 5.48 | 0.212 | 4373 | 4812 | 3.3 | 31.7 |
| **3** | 1470 | 1006 | 17.3 | 0.873 | 4870 | 6812 | 1.90 | 70 |
| **4** | 4599 | *5551* | 3.03 | 0.372 | 11236 | 26737 | 2.41 | 96 |
| **23** | 4001 | 2832 | 5.82 | 0.767 | 10922 | 12988 | 1.3 | 91.7 |
| **179** | 6257 | 7828 | 2.02 | 0.306 | 15358 | 28784 | 1.6 | 73.5 |

Reference compound **A174:** 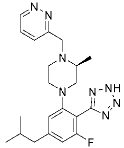 synthesized referring to the preparation method in WO2023006893A1.

The experimental results show: The compounds of the present invention exhibit low clearance, high plasma exposure, and good oral bioavailability in rodent ICR mice, demonstrating good pharmacokinetic properties, which is favorable for drug development.

### Test Example 4 Study on the pharmacokinetic behavior of the compounds of the present invention in rats

Experimental drugs: Compounds of the present invention, self-prepared.

### Experimental protocol:

Healthy SD rats (SPF grade, source: Beijing Vital River Laboratory Animal Technology Co., Ltd.), 3 animals, male, weighing 180-250 g, were intravenously injected with 1 mg/kg of the compound at a dosing volume of 2 mL/kg. The compound was formulated with 5% DMSO + 10% Solutol + 85% Saline (w/v), and the animals were not fasted prior to the test.

Healthy SD rats (SPF grade, source: Beijing Vital River Laboratory Animal Technology Co., Ltd.), 3 animals, male, weighing 180-250 g, were intragastrically administered with 5 mg/kg of the compound at a dosing volume of 10 mL/kg. The compound was formulated with 5% DMSO + 10% Solutol + 85% Saline (w/v), and the animals were not fasted prior to the test.

Blood samples (approx. 0.25 mL each) were collected via the vein at 5 min, 15 min, 30 min, 1 h, 2 h, 4 h, 6 h, 8 h, and 24 h after intravenous administration, and at 5 min, 15 min, 30 min, 1 h, 2 h, 4 h, 6 h, 8 h, and 24 h after intragastric administration, respectively, using sodium heparin as an anticoagulant. After collection, the blood samples were placed on ice, and the plasma was separated by centrifugation within 1 hour (centrifugation conditions: 6000 g, 3 minutes, 2-8 °C). The concentration of the compound in plasma was determined by liquid chromatography-tandem mass spectrometry. Plasma samples were stored in a -80 °C freezer before analysis. Pharmacokinetic parameters were calculated using Phoenix WinNonlin 8.2.0 based on plasma concentration data at different time points. The experimental results are as follows:

| Compound | Intravenous injection (1 mg/kg) | | | | Intragastric administration (5 mg/kg) | | | |
|---|---|---|---|---|---|---|---|---|
| | Cₘₐₓ (ng/mL) | AUClast (h*ng/mL) | CL (mL/kg/min) | Vss (L/kg) | Cₘₐₓ (ng/mL) | AUClast (h*ng/mL) | T_{1/2} (h) | F(%) |
| **A174** | 5321 | 2936 | 5.69 | 0.263 | 3303 | 5617 | 1.3 | 38.3 |
| **3** | 2074 | 2533 | 0.395 | 1.01 | 1637 | 6269 | 4.06 | 49.5 |
| **4** | 5510 | 14313 | 1.17 | 0.243 | 7089 | 72093 | 4.08 | 100 |
| **23** | 2700 | 3939 | 4.46 | 0.5 | 3729 | 19592 | 3.6 | 99 |

The experimental results show: The compounds of the present invention exhibit high plasma exposure in rodent SD rats, demonstrating good pharmacokinetic properties.

### Test Example 5 Study on the analgesic effect of the compounds of the present invention on inhibiting pain in a mouse selective nerve injury model

### 1. Test drugs

Examples 6, 16, 23, 31 and reference compound A174.

A 5% DMSO + 10% Solutol + 85% Saline solution was used.

### 2. Experimental methods and materials

### 2.1 Experimental animals and housing conditions

Experimental animals: C57BL/6 mice, weighing 18-22 g, male, purchased from Beijing Vital River Laboratory Animal Technology Co., Ltd.

Housing conditions: 4 animals/cage, regulated 12/12-hour light/dark cycle, temperature 22-25 °C, humidity 40-70%, with free access to food and water.

### 2.2 Experimental methods:

### 1.1 Acclimatization

After the experimental animals arrived, they were acclimatized for 3-7 days.

### 1.2 Model establishment

(1) The surgical procedure was performed under aseptic conditions.
(2) Surgical instruments (scissors, forceps, scalpels, surgical cotton, and sutures) were sterilized before surgery.
(3) The animals were anesthetized with Zoletil 50 (50 mg/kg) + xylazine hydrochloride injection (8 mg/kg) (intraperitoneal injection, 2.5 mL/kg). The toes of the animals were pinched to confirm complete anesthesia before surgery; an ophthalmic ointment was applied to the eyes of the animals to prevent corneal drying.
(4) The hair in the surgical area of the animals was shaved, and the skin was disinfected three times with iodophor and 70% ethanol; the surgery was started after the skin dried.
(5) The sciatic nerve and its three distal branches (the tibial, common peroneal, and sural nerves) were exposed. The tibial and common peroneal nerves were transected using ophthalmic scissors, and the sural nerve was spared.
(6) The wound was sutured.
(7) The surgical instruments were cleaned and sterilized using a hot bead sterilizer.
(8) After surgery, the animals were placed on a heating pad, and 1 mL of normal saline was injected subcutaneously to prevent dehydration. After the animals were fully awake (able to move freely), they were returned to their cages.

### 1.3 Grouping

On days 4-6 post-modeling, the animals were placed in the testing environment for habituation for 30 minutes/day for 3 consecutive days. On day 7 post-modeling, the baseline measurement of mechanical allodynia was performed on the modeled animals. Animals that did not exhibit mechanical allodynia (paw withdrawal threshold greater than 0.6 g) were excluded, and 72 successfully modeled animals (paw withdrawal threshold less than 0.6 g) were selected and divided into 9 groups, with 8 animals in each group.

| Group No. | Grouping | Number of animals | Dose (mg/kg) | Route of administration (10 mL/kg) | Mechanical allodynia time |
|---|---|---|---|---|---|
| 1 | Vehicle control group | 8 | NA | p.o. | 1, 2, 4 hours |
| 2 | A174 | 7 | 10 | p.o. | 1, 2, 4 hours |
| 3 | Compound 23 | 8 | 10 | p.o. | 1, 2, 4 hours |
| 4 | Compound 31 | 7 | 10 | p.o. | 1, 2, 4 hours |
| 5 | Compound 6 | 7 | 10 | p.o. | 1, 2, 4 hours |
| 6 | Compound 16 | 7 | 10 | p.o. | 1, 2, 4 hours |

### 1.4 Administration

The mice in each group were given a single dose of the corresponding drug via intraperitoneal or oral administration at 10 mL/kg according to the experimental protocol.

### 1.5 Mechanical allodynia test

On day 8 post-modeling, the mechanical allodynia test was performed on the left hind paw of the mice at 1, 2, and 4 hours after administration.

The mice were placed individually in a Plexiglas box with a wire mesh floor to ensure that the paws of the mice could be tested. The mice were allowed to habituate for 15 minutes before the test.

After habituation, the test was performed using testing filaments on the central plantar surface of the left hind paw of the mice. The testing filaments included 8 testing strengths: 2.36 (0.02 g), 2.44 (0.04 g), 2.83 (0.07 g), 3.22 (0.16 g), 3.61 (0.4 g), 3.84 (0.6 g), 4.08 (1 g), and 4.17 (1.4 g). During testing, the test filament was pressed vertically against the skin with enough force to bend the filament for 6-8 seconds, with an interval of 5 seconds between each test. During testing, a rapid paw withdrawal by the animal was recorded as a pain response. Paw withdrawal when the test filament was removed from the animal's skin was also recorded as a pain response. If the animal moved or walked around, it was not recorded as a pain response, and the test should be repeated.

During the test, the 3.22 (0.16 g) filament was used first. If the animal showed a pain response, the testing filament of the next lower strength was used for the next test; if the animal showed no pain response, the testing filament of the next higher strength was used for the next test (Chaplan et al. 1994). The maximum strength of the testing filaments was 4.17 (1.4 g).

The test results were recorded in the table below, where a pain response was recorded as "x" and no pain response was recorded as "o".

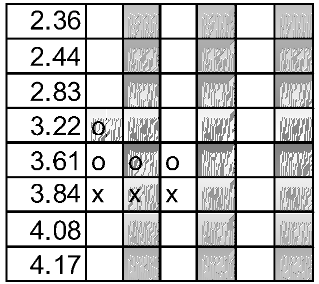

Mechanical allodynia is expressed as the paw withdrawal threshold (PWT) in the mouse behavioral test, which is calculated according to the following formula:
50% response threshold (g)= (10^{(*X*f+kδ})/10,000
Xf=value of the final testing filament used in the test
k = tabular value (Chaplan et al. 1994, page 62)
δ = mean difference

### 1.6 Key instruments

| Instrument name / Model | Location |
|---|---|
| Body weight balance / Model YH20002 | NT-1#1229 |
| Electronic balance / MS2205DM | NT-1#1225 |
| Touch-Test Sensory Evaluator / 58011 | NT-1#1229 |

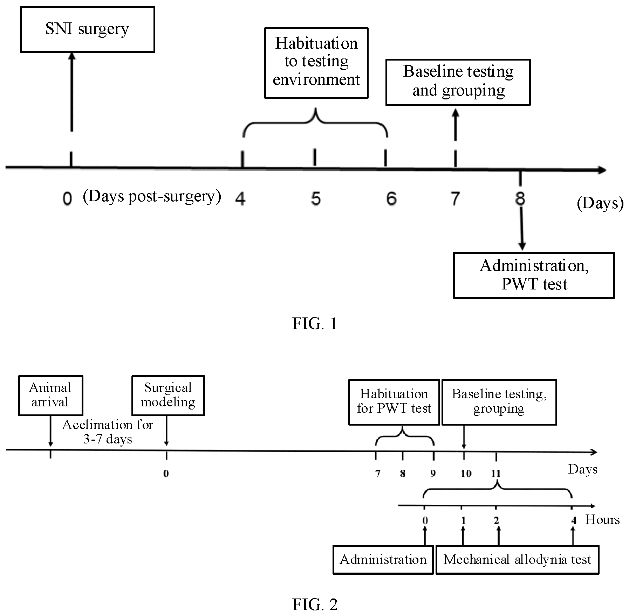

### 1.7 The timeline is shown in FIG. 1

### 1.8 Experimental testing endpoints

| |
|---|
| In vivo testing endpoints |
| Mechanical allodynia paw withdrawal threshold (PWT (g)) |

### 2.3 Statistical analysis

Data was collected using Excel software.

Data were analyzed using one-way or two-way analysis of variance (ANOVA) followed by Dunnett's multiple comparisons test in Prism software (GraphPad Software, Inc.). The area under the curve (AUC) of the analgesic efficacy was calculated using Prism software (GraphPad Software, Inc.) by first selecting the XY format, entering the data, clicking analyze, and selecting Area under curve (AUC) under XY analyses.

### 3. Results

In the mouse selective nerve injury model, the area under the curve (AUC₀₋₄ₕ) corresponding to the analgesic effect of each compound on inhibiting pain in mice at 1, 2, and 4 hours after administration was calculated. A larger AUC value indicates a better analgesic effect.

| Group | Vehicle control group | Compound **A174** | Compound **23** | Compound **31** | Compound **6** | Compound **16** |
|---|---|---|---|---|---|---|
| Mean AUC value | 1.192 | 1.826 | 3.027 | 2.856 | 2.280 | 2.480 |
| P value | / | ns | *** | *** | ** | *** |

| | | | | | | |
|---|---|---|---|---|---|---|
| Note: P value (compared with the vehicle control group), ***: P < 0.001; **: P < 0.01; ns: no statistically significant difference. | | | | | | |

### 4. Conclusion

The compounds of the present invention exhibit a good analgesic effect on inhibiting pain in the mouse selective nerve injury model.

### Test Example 6 Study on the analgesic effect of the compounds of the present invention on inhibiting pain in a rat chronic constriction injury of the sciatic nerve model

### 1. Test drugs

Example 35 and reference compound A174.

A 5% DMSO + 10% Solutol + 85% Saline solution was used.

### 2. Experimental materials and methods

### 2.1 Experimental materials

### 2.1.1 Experimental animals and housing conditions

Experimental animals: Sprague-Dawley rats, weighing 80-100 g, male, purchased from Beijing Vital River Laboratory Animal Technology Co., Ltd.

Housing conditions: 4 animals/cage, regulated 12/12-hour light/dark cycle, temperature 22-25 °C, humidity 40-70%, with free access to food and water.

### 2.1.2 Key experimental equipment

| Instrument name | Brand | Model |
|---|---|---|
| Body weight balance | Changzhou Keyuan | YH20002 |
| Electronic balance | METTLER TOLEDO | MS205DU |
| Touch-Test Sensory Evaluator | North Coast | 58011 |

### 2.2 Experimental methods:

### 2.2.1 Acclimatization

After the experimental animals arrived, they were acclimatized for 3-7 days.

### 2.2.2 Model establishment

1) The surgical procedure was performed under aseptic conditions.
2) Surgical instruments (scissors, forceps, scalpels, surgical cotton, and sutures) were sterilized before surgery.
3) The animals were anesthetized with Zoletil 50 + xylazine hydrochloride injection (20 mg/kg + 8 mg/kg, intraperitoneal injection). The toes of the animals were pinched to confirm complete anesthesia before surgery. An ophthalmic ointment was applied to the eyes of the animals to prevent corneal drying.
4) The skin in the surgical area of the left hind plantar surface was disinfected three times with iodophor and 70% ethanol. The surgery was started after the skin dried.
5) The left sciatic nerve was isolated. Four loose ligatures were tied around the sciatic nerve using 4-0 chromic gut sutures at approximately 7 mm proximal to the bifurcation, with a spacing of about 1 mm between the ligatures.
6) The wound was sutured.
7) The surgical instruments were cleaned and sterilized using a hot bead sterilizer.
8) After surgery, the animals were placed on a heating pad, and 5 mL of normal saline was injected subcutaneously to prevent dehydration. After the animals were fully awake (able to move freely), they were returned to their cages.

### 2.2.3 Baseline testing and grouping:

1) On days 7 to 9 post-surgery, the animals were placed in the testing environment to habituate for at least 30 minutes per day.
2) On day 10 post-surgery, the baseline measurement of mechanical allodynia (PWT) was performed on all animals. The modeled animals that did not exhibit mechanical allodynia (PWT greater than 5 g) were excluded, and 24 animals were selected and randomly divided into 3 groups, with 8 animals in each group.

| Group No. | Grouping | Number of animals | Dose (mg/kg) | Route of administration (10 mL/kg) | Mechanical allodynia time |
|---|---|---|---|---|---|
| G1 | Vehicle control group | 8 | NA | p.o. | 1, 2, 4 hours |
| G2 | Compound A-174 | 8 | 10 | p.o. | 1, 2, 4 hours |
| G3 | Compound 35 | 8 | 10 | p.o. | 1, 2, 4 hours |

### 2.2.4 Weighing and administration

On day 11 post-surgery, the animals in all groups were weighed and administered according to the grouping information.

### 2.2.5 Efficacy testing

The mechanical allodynia test was performed on each animal using testing filaments at 1 h and 2 h after a single dose administration.

### 2.2.6 Mechanical allodynia testing method:

1) The rats were placed individually in a Plexiglas box with a wire mesh floor to ensure that the paws of the rats could be tested. The rats were allowed to habituate for 15 minutes before the test.
2) After habituation, the test was performed using testing filaments on the central plantar surface of the left hind paw of the rats. The testing filaments included 8 testing strengths: 3.61 (0.4 g), 3.84 (0.6 g), 4.08 (1 g), 4.31 (2 g), 4.56 (4 g), 4.74 (6 g), 4.93 (8 g), and 5.18 (15 g). During testing, the test filament was pressed vertically against the skin with enough force to bend the filament for 6-8 seconds, with an interval of 5 seconds between each test. During testing, a rapid paw withdrawal by the animal was recorded as a pain response. Paw withdrawal when the test filament was removed from the animal's skin was also recorded as a pain response. If the animal moved or walked around, it was not recorded as a pain response, and the test should be repeated.
3) During the test, the 4.31 (2 g) filament was used first. If the animal showed a pain response, the testing filament of the next lower strength was used for the next test; if the animal showed no pain response, the testing filament of the next higher strength was used for the next test. The maximum strength of the testing filaments was 5.18 (15 g).
4) The test results were recorded in the table below, where a pain response was recorded as "x" and no pain response was recorded as "o".

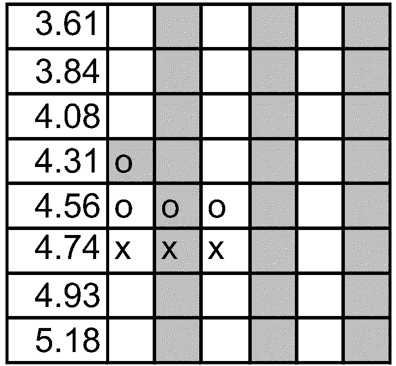

Mechanical allodynia is expressed as the paw withdrawal threshold (PWT) in the rat behavioral test, which is calculated according to the following formula:
$50 % response threshold \left(g\right) = \left({10}^{\left(Xf+kδ\right)}\right) / 10 , 000$
*Xf =* value of the final testing filament used in the test
k = tabular value (Chaplan et al. 1994, page 62)
δ = mean difference (0.224)

### 2.2.7 The timeline is shown in FIG. 2

### 2.2.8 Experimental testing endpoints:

| |
|---|
| In vivo testing endpoints |
| Rat paw withdrawal threshold (PWT) |

### 2.2.9 Data collection and analysis:

Data was collected using Excel software.

Data were analyzed using Prism software (GraphPad Software, Inc.).

### 3. Experimental results

| Testing time point | G1 Vehicle PO QD*3 / PWT (g) | G2 Compound A-174 10 mg/kg PO QD*3 /PWT(g) | G3 Compound 35 10 mg/kg PO QD*3 /PWT(g) |
|---|---|---|---|
| 0 h | 3.01 | 2.86 | 2.82 |
| 1 h | 2.74 | 4.76 | 5.88 ** |
| 2 h | 3.08 | 6.62 *** | 7.02 *** |

| | | | |
|---|---|---|---|
| Note: P value (compared with the vehicle control group), ***: P < 0.001; **: P < 0.01. | | | |

### 4. Conclusion

The compounds of the present invention exhibit a good analgesic effect on inhibiting pain in the rat chronic constriction injury of the sciatic nerve model.

## Claims

1. A compound represented by Formula (I) or a pharmaceutically acceptable salt thereof, 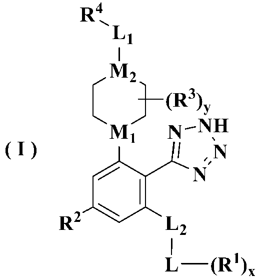
wherein: M₁ and M₂ are each independently selected from C or N;
L₁ is a bond or -CH₂-;
L2 is a bond, -CH₂-, -CF₂-, -C(O)-, -C(O)NH-, -NHC(O) -, -NH-, -O-, or -S-, wherein the -CH₂- and -NH- are optionally substituted with one or two R^{L2}, and R^{L2} are each independently selected from halogen, cyano, C₁₋₃ alkyl, C₁₋₃ alkoxy, Cₗ₋₃ haloalkyl, C₁₋₃ haloalkoxy, C₃₋₆ cycloalkyl, or 3- to 6-membered heterocyclyl;
L is selected from L₃ or Ring A;
L₃ is selected from C₁₋₆ alkyl, C₁₋₆ alkoxy, C₁₋₆ haloalkyl, C₁₋₆ haloalkoxy, or C₁₋₆ hydroxyalkyl;
Ring A is monocyclic or bicyclic, specifically selected from C₃₋₁₀ cycloalkyl, 3- to 10-membered heterocyclyl, C₆₋₁₄ aryl, 5- to 10-membered heteroaryl, 5- to 6-membered heterocyclyl fused to 5- to 6-membered heteroaryl, or 5- to 6-membered heterocyclyl fused to aryl;
R¹ are each independently selected from halogen, hydroxy, amino, amido, C₁₋₃ alkylamino, di(C₁₋₃ alkyl)amino, cyano, C₁₋₆ alkyl, C₁₋₆ alkoxy, C₁₋₆ haloalkyl, C₁₋₆ haloalkoxy, C₁₋₆ hydroxyalkyl, C₃₋₆ cycloalkyl, or 3- to 6-membered heterocyclyl;
or, any two R¹ are joined to form a 3- to 8-membered heterocyclyl or C₃₋₈ cycloalkyl;
R² is selected from amino, C₁₋₆ alkyl, C₁₋₆ haloalkyl, C₁₋₆ haloalkoxy, C₃₋₆ cycloalkyl, 3-to 6-membered heterocyclyl, C₆₋₁₀ aryl, 5- to 6-membered heteroaryl, -OR^{2.1} or -NHR^{2.1}; the C₁₋₆ alkyl, C₁₋₆ haloalkyl, C₁₋₆ haloalkoxy, C₃₋₆ cycloalkyl, 3- to 6-membered heterocyclyl, C₆₋₁₀ aryl, and 5- to 6-membered heteroaryl are optionally further substituted with one or more R^{2.1};
R^{2.1} is selected from C₁₋₆ alkyl, C₁₋₆ haloalkyl, C₁₋₆ haloalkoxy, C₃₋₆ cycloalkyl, or 3- to 6-membered heterocyclyl, wherein the C₁₋₆ alkyl, C₁₋₆ haloalkyl, C₁₋₆ haloalkoxy, C₃₋₆ cycloalkyl, and 3- to 6-membered heterocyclyl are optionally substituted with one or more R^{2.1.1};
R^{2.1.1} is selected from halogen, amino, cyano, hydroxy, C₁₋₆ alkoxy, C₃₋₆ cycloalkyl, or 3-to 6-membered heterocyclyl;
R³ are each independently selected from hydrogen, C₁₋₆ alkyl, C₁₋₆ alkoxy, C₁₋₆ haloalkyl, or C₁₋₆ haloalkoxy;
R⁴ is selected from 5- to 6-membered heteroaryl, 5- to 6-membered heterocyclyl fused to 5- to 6-membered heteroaryl, 5- to 6-membered heteroaryl fused to 5- to 6-membered heteroaryl, 5- to 6-membered heteroaryl fused to phenyl, 5- to 6-membered heterocyclyl fused to phenyl, or 5- to 6-membered heterocyclyl, wherein the 5- to 6-membered heteroaryl, 5- to 6-membered heterocyclyl fused to 5- to 6-membered heteroaryl, 5- to 6-membered heteroaryl fused to 5- to 6-membered heteroaryl, 5- to 6-membered heteroaryl fused to phenyl, 5- to 6-membered heterocyclyl fused to phenyl, and 5- to 6-membered heterocyclyl are optionally substituted with one or more R^{4.1};
R^{4.1} is selected from halogen, hydroxy, cyano, amino, C₁₋₆ alkyl, C₁₋₆ alkoxy, C₁₋₆ haloalkyl, C₁₋₆ hydroxyalkyl, C₁₋₆ haloalkoxy, C₃₋₆ cycloalkyl, or 3- to 6-membered heterocyclyl;
x and y are each independently selected from 0, 1, 2, 3, or 4.

2. The compound or a pharmaceutically acceptable salt thereof according to claim 1, wherein L₁ is selected from -CH₂-; or, R⁴ is selected from 5- to 6-membered heteroaryl, 5- to 6-membered heteroaryl fused to 5- to 6-membered heteroaryl, 5- to 6-membered heteroaryl fused to phenyl, 5- to 6-membered heterocyclyl fused to phenyl, 5- to 6-membered heterocyclyl fused to 5- to 6-membered heteroaryl, or 6-membered heterocyclyl; preferably, R⁴ is selected from thiazolyl, pyrazolyl, pyridazinyl, imidazolyl, pyridinyl, isoxazolyl, triazolyl, tetrazolyl, oxazolyl, pyrazinyl, pyrimidinyl, oxadiazolyl, pyridinonyl, pyridazinonyl, pyrimidinonyl, pyrazinonyl, imidazopyridinyl, benzimidazolyl, benzothiazolyl, benzoxazolyl, benzopyrimidinonyl, pyridopyrimidinonyl, benzopyrazolyl, benzopyridinonyl, benzopyrazinonyl, thiazolopyrimidinonyl, pyrazolopyrimidinonyl, or oxazolopyridinyl; more preferably, R⁴ is selected from thiazolyl, pyridazinyl, benzothiazolyl, or pyrazinyl.

3. The compound or a pharmaceutically acceptable salt thereof according to claim 1 or 2, wherein the compound of Formula (I) is further represented by Formula (II):

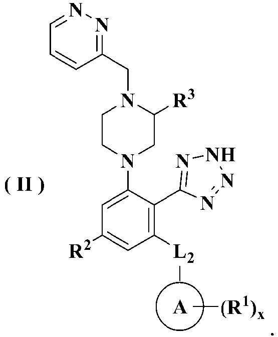

4. The compound or a pharmaceutically acceptable salt thereof according to any one of claims 1-3, wherein Ring A is selected from 5- to 6-membered heteroaryl, 5-membered heteroaryl fused to phenyl, 5-membered heterocyclyl fused to phenyl, 5-membered heterocyclyl fused to 6-membered heteroaryl, 6-membered heterocyclyl fused to 5-membered heteroaryl, C₆₋₁₀ aryl, C₃₋₈ cycloalkyl, or 3- to 8-membered heterocyclyl; preferably, Ring A is selected from pyridazinyl, pyrazolyl, pyridinyl, phenyl, pyrrolyl, imidazolyl, triazolyl, tetrazolyl, furyl, thienyl, cyclopropyl, cyclobutyl, cyclopentyl, cyclohexyl, tetrahydropyranyl, pyrrolidinyl, pyridinonyl, 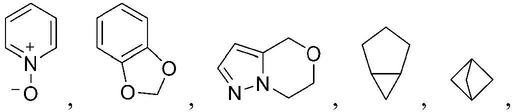 morpholinyl, piperidinyl, oxiranyl, aziridinyl, oxetanyl, azetidinyl, piperazinyl, pbenzopyrrolyl, benzopyrrolidinyl, diazaspiro[3.3]heptanyl, or oxaazaspiro[3.3]heptanyl.

5. The compound or a pharmaceutically acceptable salt thereof according to any one of claims 1-4, wherein R¹ are each independently selected from halogen, hydroxy, amino, amido, cyano, C₁₋₄ alkyl, C₁₋₄ alkoxy, C₁₋₄ haloalkyl, C₁₋₄ haloalkoxy, C₁₋₄ hydroxyalkyl, C₃₋₄ cycloalkyl, or 3- to 4-membered heterocyclyl; preferably, R¹ are each independently selected from fluoro, chloro, hydroxy, amino, amido, cyano, methyl, ethyl, propyl, methoxy, ethoxy, propoxy, trifluoromethyl, difluoromethyl, trifluoroethyl, hydroxymethyl, hydroxyethyl, cyclopropyl, cyclobutyl, oxiranyl, aziridinyl, oxetanyl, or azetidinyl.

6. The compound or a pharmaceutically acceptable salt thereof according to any one of claims 1-5, wherein L₂ is selected from -NH-, -CH₂-, -CF₂-, -C(O)NH-, -NHC(O)-, -O-, or -S-.

7. The compound or a pharmaceutically acceptable salt thereof according to any one of claims 1-6, wherein R² is selected from amino, C₁₋₄ alkyl, C₁₋₄ haloalkyl, C₁₋₄ haloalkoxy, C₃₋₆ cycloalkyl, 3- to 6-membered heterocyclyl, -OR^{2.1}, or -NHR^{2.1}; preferably, R² is selected from amino, methyl, ethyl, propyl, n-butyl, isobutyl, cyclopropyl, piperidinyl, morpholinyl, piperazinyl, -OR^{2.1}, or -NHR^{2.1};
R^{2.1} is selected from C₁₋₄ alkyl, C₁₋₄ haloalkyl, C₁₋₄ haloalkoxy, C₃₋₄ cycloalkyl, or 3- to 4-membered heterocyclyl, wherein the C₁₋₄ alkyl, C₁₋₄ haloalkyl, C₁₋₄ haloalkoxy, C₃₋₆ cycloalkyl, and 3- to 6-membered heterocyclyl are optionally substituted with one or more R^{2.1.1}; preferably, R^{2.1} is selected from methyl, ethyl, propyl, n-butyl, isobutyl, methoxy, ethoxy, propoxy, cyclopropyl, cyclobutyl, cyclopentyl, oxiranyl, aziridinyl, oxetanyl, azetidinyl, piperidinyl, piperazinyl, morpholinyl, trifluoromethyl, difluoromethyl, or difluoroethyl;
the R^{2.1.1} is selected from halogen, amino, cyano, hydroxy, C₁₋₄ alkoxy, C₃₋₆ cycloalkyl, or 3- to 6-membered heterocyclyl; preferably, the R^{2.1.1} is selected from fluoro, chloro, methyl, ethyl, propyl, n-butyl, isobutyl, methoxy, ethoxy, propoxy, cyclopropyl, cyclobutyl, cyclopentyl, oxiranyl, aziridinyl, oxetanyl, azetidinyl, piperidinyl, piperazinyl, morpholinyl, trifluoromethyl, difluoromethyl, or difluoroethyl;
or, R³ is selected from hydrogen, C₁₋₄ alkyl, C₁₋₄ alkoxy, C₁₋₄ haloalkyl, or C₁₋₄ haloalkoxy; preferably, R³ is selected from hydrogen, methyl, ethyl, propyl, methoxy, ethoxy, propoxy, trifluoromethyl, or difluoromethyl.

8. The compound or a pharmaceutically acceptable salt thereof according to any one of claims 1 to 7, **characterized in that** the compound is selected from the compounds shown in Table 1.

9. A pharmaceutical composition, comprising:
(1) the compound or a pharmaceutically acceptable salt thereof according to any one of claims 1 to 8, and
(2) a pharmaceutically acceptable excipient.

10. Use of the compound or a pharmaceutically acceptable salt thereof according to any one of claims 1 to 8, or the pharmaceutical composition according to claim 9, in the preparation of a medicament, wherein the medicament is useful for preventing and/or treating pain; preferably, the pain is neuropathic pain.
